# EUROPEAN PATENT APPLICATION

(11) **EP 3 078 384 A1**
(43) Date of publication of application: **12.10.2016**
(21) Application number: 16159631.7
(22) Date of filing: 21.02.2013
(51) Int. Cl.: A61K 38/07, A61P 27/02

(54) **METHODS AND COMPOSITIONS FOR PREVENTING OR TREATING OPHTHALMIC CONDITIONS**

(30) Priority: 22.02.2012 US 201261601878 P; 01.03.2012 US 201261605576 P
(62) Divisional of application: 13751091.3
(71) Applicant: Stealth Peptides International, Inc., 98000 Monaco (MC)
(72) Inventor: WILSON, Travis D, Newton, MA Massachusetts 02461 (US); MOONEY, George K, Lyme, CT Connecticut 06371 (US); OATES, Peter J, Gales Ferry, CT Connecticut 06335 (US)
(74) Representative: Atkinson, Jennifer

(57) **Abstract**

The disclosure provides methods of preventing or treating ophthalmic diseases or conditions in a mammalian subject. The methods comprise administering an effective amount of an aromatic-cationic peptide to subjects in need thereof. More specifically, the disclosure provides a composition for preventing, treating, or ameliorating the symptoms of diabetic macular edema in a mammalian subject in need thereof, comprising: a therapeutically effective amount of a peptide D-Arg- 2'6'-Dmt-Lys-Phe-NH2 or a pharmaceutically acceptable salt thereof.

## Description

### TECHNICAL FIELD

The present technology relates generally to compositions and methods of preventing or treating ophthalmic diseases or conditions such as diabetic macular edema. In particular, the present technology relates to administering aromatic-cationic peptides in effective amounts to prevent or treat ophthalmic diseases or conditions.

### BACKGROUND

The following description is provided to assist the understanding of the reader. None of the information provided or references cited is admitted to be prior art to the present invention.

Diseases and degenerative conditions of the optic nerve and retina are the leading causes of blindness in the world. A significant degenerative condition of the retina is age-related macular degeneration (ARMD). ARMD is the most common cause of blindness in people over 50 in the USA and its prevalence increases with age. ARMD is classified as either wet (neovascular) or dry (non-neovascular); the dry form of the disease is more common. Wet ARMD is considered an advanced stage of dry ARMD and is associated with angiogenesis. Macular degeneration occurs when the central retina has become distorted and thinned usually associated with age but also characterized by intra-ocular inflammation and angiogenesis (wet ARMD only) and/or intra-ocular infection. Inappropriate angiogenesis or neovascularization is thought to result from retinal ischemia, as the subsequent generation of free radicals, resulting in oxidative tissue damage, local inflammation and production of growth factors (such as VEGF and FGF) and inflammatory mediators.

The retina is the part of the eye that is sensitive to light. The macula is the central portion of the retina, a small area rich in cones, the specialized nerve endings that detect color and upon which daytime vision depends and is the part of the eye responsible for detailed central vision. Retinopathy is a leading cause of blindness in type I diabetes, and is also common in type II diabetes. The degree of retinopathy depends on the duration of diabetes, and generally begins to occur ten or more years after onset of diabetes. Diabetic retinopathy may be classified as non-proliferative, where the retinopathy is characterized by increased capillary permeability, edema and exudates, or proliferative, where the retinopathy is characterized by neovascularization extending from the retina to the vitreous, scarring, deposit of fibrous tissue and the potential for retinal detachment. Diabetic retinopathy is believed to be caused by the accumulation of glycosylated proteins due to high blood glucose that cause biochemical and hemodynamic abnormalities in the retina that in turn lead to chronic retinal hypoxia. Several other less common retinopathies include choroidal neovascular membrane (CNVM), cystoid macular edema (CME), epi-retinal membrane (ERM) and macular hole. Diseases of the macula, such as diabetic macular edema, account for a major proportion of legal blindness.

Glaucoma is made up of a collection of eye diseases that cause vision loss by damage to the optic nerve. Elevated intraocular pressure (IOP) due to inadequate ocular drainage is the primary cause of glaucoma. Glaucoma often develops as the eye ages, or it can occur as the result of an eye injury, inflammation, tumor or in advanced cases of cataract or diabetes. It can also be caused by the increase in IOP caused by treatment with steroids. Drug therapies that are proven to be effective in glaucoma reduce IOP either by decreasing vitreous humor production or by facilitating ocular draining. Such agents are often vasodilators and as such act on the sympathetic nervous system and include adrenergic antagonists.

### SUMMARY

The present technology relates generally to the treatment or prevention of ophthalmic diseases or conditions in mammals through administration of therapeutically effective amounts of aromatic-cationic peptides to subjects in need thereof.

In one aspect, the present disclosure provides a method for preventing, treating, or ameliorating the symptoms of diabetic macular edema in a mammalian subject in need thereof, the method comprising: administering to the subject a therapeutically effective amount of a peptide D-Arg-2'6'-Dmt-Lys-Phe-NH₂ or a pharmaceutically acceptable salt thereof, wherein the peptide is administered ocularly, orally, or parenterally.

In some embodiments, the therapeutically effective amount of the peptide is administered ocularly to one or more regions of the eye. In some embodiments, the one or more regions of the eye is selected from the group consisting of the posterior chamber, ora serrata, ciliary muscle, ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina. In some embodiments, the region of the eye is the macula. In some embodiments, the mammal is a human.

In some embodiments, the subject is at risk of having, suspected of having, or diagnosed as having one or more of macular degeneration, central retinal thickness, intraretinal water content, macular central fovea thickness, contrast sensitivity, or loss of visual acuity. In some embodiments, the peptide is administered in combination with at least one additional therapeutic agent. In some embodiments, the additional therapeutic agent is administered before peptide administration, after peptide administration, simultaneously with peptide administration, or a combination thereof.

In one aspect, the present disclosure provides a composition for preventing, treating, or ameliorating the symptoms of diabetic macular edema in a mammalian subject in need thereof, comprising: a therapeutically effective amount of a peptide D-Arg-2'6'-Dmt-Lys-Phe-NH₂ or a pharmaceutically acceptable salt thereof, wherein the composition is administered ocularly, orally, or parenterally.

In some embodiments, the composition is administered ocularly to one or more regions of the eye. In some embodiments, the one or more regions of the eye is selected from the group consisting of the posterior chamber, ora serrata, ciliary muscle, ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina.

In some embodiments, the region of the eye is the macula. In some embodiments, the mammal is a human. In some embodiments, the subject is at risk of having, suspected of having, or diagnosed as having one or more of macular edema, central retinal thickness, intraretinal water content, macular central fovea thickness, contrast sensitivity, or loss of visual acuity. In some embodiments, the composition is administered in combination with at least one additional therapeutic agent. In some embodiments, the additional therapeutic agent is administered before, after, or simultaneous to the composition, or a combination thereof.

In one aspect, the present disclosure provides a method for the treatment, prevention, or amelioration of microvascular damage caused by acute ocular ischemia in a mammalian subject in need thereof, the method comprising: administering to the subject a therapeutically effective amount of a peptide D-Arg-2'6'-Dmt-Lys-Phe-NH₂ or a pharmaceutically acceptable salt thereof, wherein the peptide is administered ocularly, orally, or parenterally.

In some embodiments, the method further comprises the step of performing a revascularization procedure on the subject. In some embodiments, the subject is administered the peptide prior to or following the formation of ocular ischemia. In some embodiments, the subject is administered the peptide before, during, or after the revascularization procedure, or continuously before, during, and after the revascularization procedure.

In one aspect, the disclosure provides a method of treating or preventing an ophthalmic condition comprising administering to a subject in need thereof an aromatic-cationic peptide. In some embodiments, the aromatic-cationic peptide is a peptide having:
at least one net positive charge;
a minimum of four amino acids;
a maximum of about twenty amino acids;
a relationship between the minimum number of net positive charges (pm) and the total number of amino acid residues (r) wherein 3pm is the largest number that is less than or equal to r + 1; and a relationship between the minimum number of aromatic groups (a) and the total number of net positive charges (pt) wherein 2a is the largest number that is less than or equal to pt + 1, except that when a is 1, pt may also be 1. In particular embodiments, the mammalian subject is a human.

In one embodiment, 2pm is the largest number that is less than or equal to r+1, and may be equal to pt. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide may have the formula Phe-D-Arg-Phe-Lys-NH₂ or 2',6'-Dmp-D-Arg-Phe-Lys-NH₂. In a particular embodiment, the aromatic-cationic peptide has the formula D-Arg-2',6'-Dmt-Lys-Phe-NH₂.

In one embodiment, the peptide is defined by formula I: 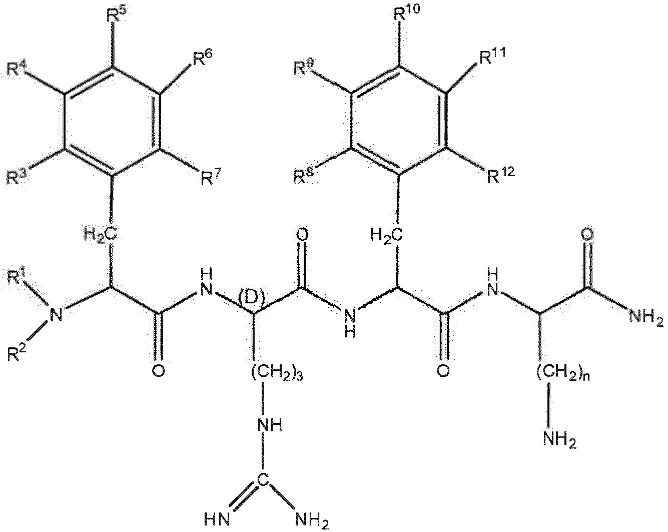
wherein R¹ and R² are each independently selected from
   (i) hydrogen;
   (ii) linear or branched C₁-C₆ alkyl;
   (iii) 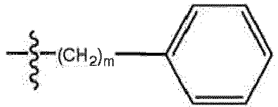 where m = 1-3;
   (iv) 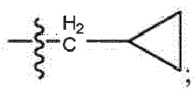
   (v) 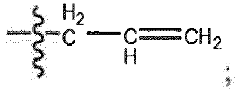
R³, R⁴, R⁵, R⁶, R⁷, R⁸, R⁹, R¹⁰, R¹¹ and R¹² are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and
n is an integer from 1 to 5.

In a particular embodiment, R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

In one embodiment, the peptide is defined by formula II: 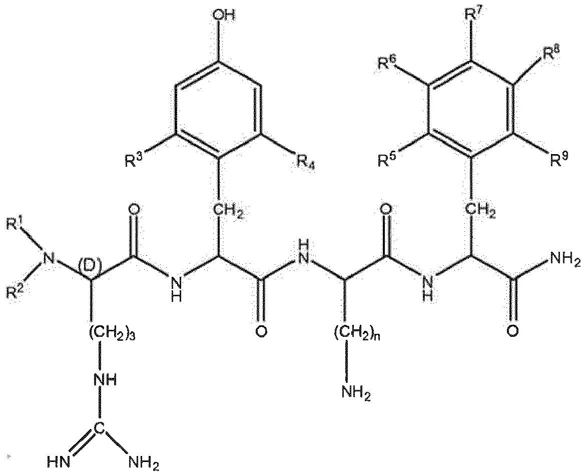
wherein R¹ and R² are each independently selected from
   (i) hydrogen;
   (ii) linear or branched C₁-C₆ alkyl;
   (iii) 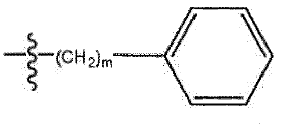 where m = 1-3;
   (iv) 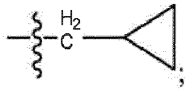
   (v) 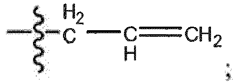
R³ and R⁴ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄dialkylamino;
   (vii) nitro;
   (viii) hydroxyl;
   (ix) halogen, where "halogen" encompasses chloro, fluoro, bromo, and iodo; and
n is an integer from 1 to 5.

The aromatic-cationic peptides may be administered in a variety of ways. In some embodiments, the peptides may be administered intraocularly, orally, topically, intranasally, ocularly, intraperitoneally, parenterally, intravenously, subcutaneously, or transdermally (*e.g.*, by iontophoresis).

In one aspect, the present disclosure provides a pharmaceutical composition comprising a therapeutically effective amount of the peptide D-Arg-2'6'-Dmt-Lys-Phe-NH₂ or Phe-D-Arg-Phe-Lys-NH₂ formulated for topical, iontophoretic, or intraocular administration.

In one aspect, the present disclosure provides an ophthalmic formulation comprising a therapeutically effective amount of the peptide D-Arg-2'6'-Dmt-Lys-Phe-NH₂ or a pharmaceutically acceptable salt thereof, such as an acetate salt and/or a tri-fluoro-acetate salt or Phe-D-Arg-Phe-Lys-NH₂ or a pharmaceutically acceptable salt thereof, such as an acetate salt and/or a tri-fluoro-acetate salt. In one embodiment, the formulation is soluble in the cornea, aqueous humor, and lens of the eye. In one embodiment, the formulation further comprises a preservative. In one embodiment, the preservative is present in a concentration of less than 1%.

In one embodiment, the formulation further comprises an active agent selected from the group consisting of: an antioxidant, a metal complexer, an anti-inflammatory drug, an antibiotic, and an antihistamine. In one embodiment, the antioxidant is vitamin A, vitamin C, vitamin E, lycopene, selenium, α-lipoic acid, coenzyme Q, glutathione, or a carotenoid. In one embodiment, the formulation further comprises an active agent selected from the group consisting of: aceclidine, acetazolamide, anecortave, apraclonidine, atropine, azapentacene, azelastine, bacitracin, befunolol, betamethasone, betaxolol, bimatoprost, brimonidine, brinzolamide, carbachol, carteolol, celecoxib, chloramphenicol, chlortetracycline, ciprofloxacin, cromoglycate, cromolyn, cyclopentolate, cyclosporin, dapiprazole, demecarium, dexamethasone, diclofenac, dichlorphenamide, dipivefrin, dorzolamide, echothiophate, emedastine, epinastine, epinephrine, erythromycin, ethoxzolamide, eucatropine, fludrocortisone, fluorometholone, flurbiprofen, fomivirsen, framycetin, ganciclovir, gatifloxacin, gentamycin, homatropine, hydrocortisone, idoxuridine, indomethacin, isoflurophate, ketorolac, ketotifen, latanoprost, levobetaxolol, levobunolol, levocabastine, levofloxacin, lodoxamide, loteprednol, medrysone, methazolamide, metipranolol, moxifloxacin, naphazoline, natamycin, nedocromil, neomycin, norfloxacin, ofloxacin, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol, tobramycin, travoprost, triamcinulone, trifluoromethazolamide, trifluridine, trimethoprim, tropicamide, unoprostone, vidarbine, xylometazoline, pharmaceutically acceptable salts thereof, and combinations thereof.

Aspects of the invention may also be provided by the following numbered paragraphs
1. A method for preventing, treating, or ameliorating the symptoms of diabetic macular edema in a mammalian subject in need thereof, the method comprising: administering to the subject a therapeutically effective amount of a peptide D-Arg-2'6'-Dmt-Lys-Phe-NH₂ or a pharmaceutically acceptable salt thereof, wherein the peptide is administered ocularly, orally, or parenterally.
2. The method of paragraph 1, wherein the therapeutically effective amount of the peptide is administered ocularly to one or more regions of the eye.
3. The method of paragraph 2, wherein the one or more regions of the eye is selected from the group consisting of the posterior chamber, ora serrata, ciliary muscle, ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina.
4. The method of paragraph 3, wherein the region of the eye is the macula.
5. The method of paragraph 1, wherein the mammal is a human.
6. The method of paragraph 1, wherein the subject is at risk of having, suspected of having, or diagnosed as having one or more of macular degeneration, central retinal thickness, intraretinal water content, macular central fovea thickness, contrast sensitivity, or loss of visual acuity.
7. The method of paragraph 1, wherein the peptide is administered in combination with at least one additional therapeutic agent.
8. The method of paragraph 7, wherein the additional therapeutic agent is administered before peptide administration, after peptide administration, simultaneously with peptide administration, or a combination thereof.
9. A composition for preventing, treating, or ameliorating the symptoms of diabetic macular edema in a mammalian subject in need thereof, comprising: a therapeutically effective amount of a peptide D-Arg-2'6'-Dmt-Lys-Phe-NH₂ or a pharmaceutically acceptable salt thereof, wherein the composition is administered ocularly, orally, or parenterally.
10. The composition of paragraph 9, wherein the composition is administered ocularly to one or more regions of the eye.
11. The composition of paragraph 10, wherein the one or more regions of the eye is selected from the group consisting of the posterior chamber, ora serrata, ciliary muscle, ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina.
12. The composition of paragraph 11, wherein the region of the eye is the macula.
13. The composition of paragraph 9, wherein the mammal is a human.
14. The composition of paragraph 9, wherein the subject is at risk of having, suspected of having, or diagnosed as having one or more of macular degeneration, central retinal thickness, intraretinal water content, macular central fovea thickness, contrast sensitivity, or loss of visual acuity.
15. The composition of paragraph 9, wherein the composition is administered in combination with at least one additional therapeutic agent.
16. The composition of paragraph 15, wherein the additional therapeutic agent is administered before, after, or simultaneous to the composition, or a combination thereof.
17. A method for the treatment, prevention, or amelioration of microvascular damage caused by acute ocular ischemia in a mammalian subject in need thereof, the method comprising: administering to the subject a therapeutically effective amount of a peptide D-Arg-2'6'-Dmt-Lys-Phe-NH₂ or a pharmaceutically acceptable salt thereof, wherein the peptide is administered ocularly, orally, or parenterally.
18. The method of paragraph 17, further comprising the step of performing a revascularization procedure on the subject.
19. The method of paragraph 17, wherein the subject is administered the peptide prior to or following the formation of ocular ischemia.
20. The method of paragraph 17, wherein the subject is administered the peptide before, during, or after the revascularization procedure, or continuously before, during, and after the revascularization procedure.

### BRIEF DESCRIPTION OF THE FIGURES

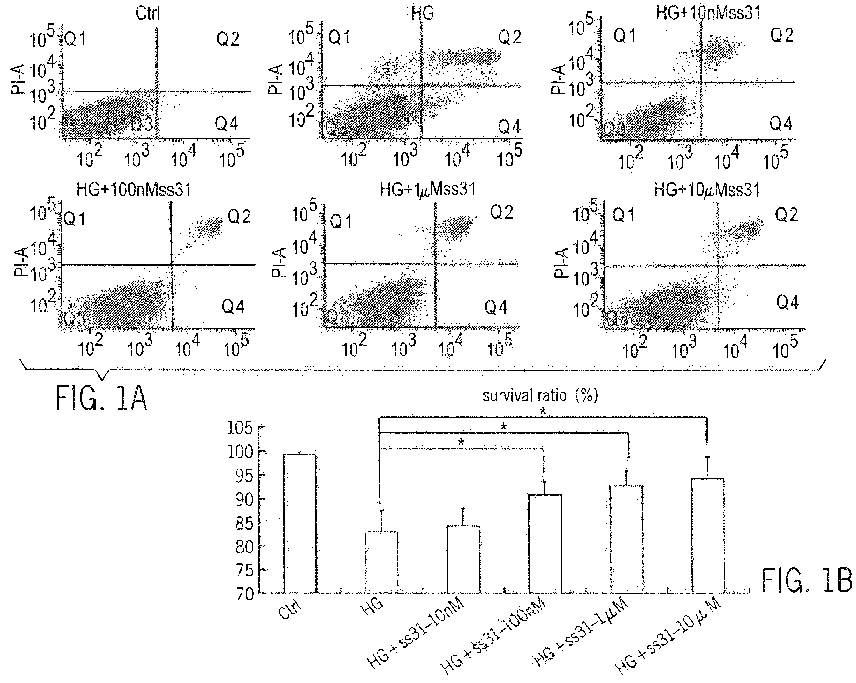

FIGs. 1A and 1B show the effects of different concentrations of D-Arg-2',6'- Dmt-Lys-Phe-NH₂ (SS-31) (10 nM, 100 nM, 1 µM and 10 µM) used as co-treatment with 30 mM glucose (HG). FIG. 1A shows analysis for apoptosis, as assessed by a Flow cytometry after Annexin V/PI staining, which showed that the survival ratios for HRECs (Q3) was 99.3%, 83.2%, 84.3%, 90.7%, 92.8%, and 94.3%, respectively 24 hours after treatment. FIG. IB is a graphic representation of the survival ratio for HRECs. Data for D-Arg-2',6'-Dmt-Lys-Phe-NH₂ (SS-31) concentrations of 100 nM, 1 µM, and 10 µM were significantly higher than that seen with high glucose exposed cells in the absence of co-treatment with D-Arg-2',6'-Dmt-Lys-Phe-NH₂ (SS-31). * p<0.05 vs. 30mM high glucose treated group.
FIGs. 2A-2F is a series of micrographs showing co-treatment with D-Arg-2',6'-Dmt-Lys-Phe-NH₂ (SS-31) reduced intracellular reactive oxygen species (ROS) in HRECs exposed to 30 mM glucose for 24 h and 48 h. Intracellular ROS was measured using dihydroethidium. 2A, 2D normal culture media; 2B, 2E 30 mM glucose; and 2C, 2F 30 mM glucose + D-Arg-2',6'-Dmt-Lys-Phe-NH₂ (SS-31) (100 nM) at 24 and 48 h, respectively.

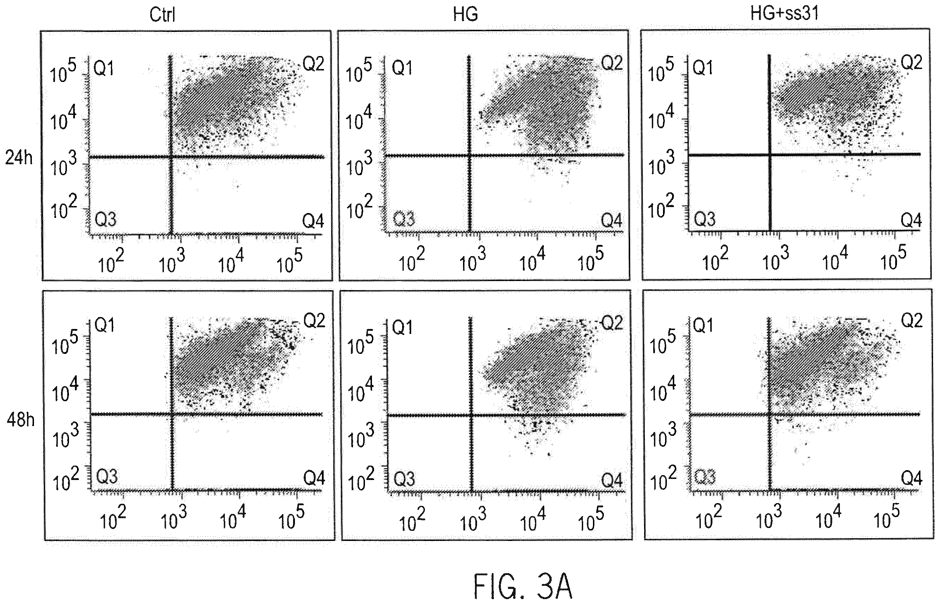

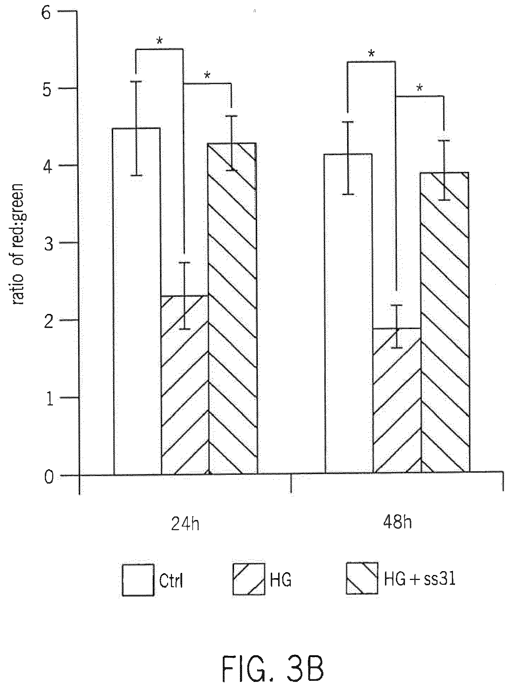

FIGs. 3A and 3B show that D-Arg-2',6'-Dmt-Lys-Phe-NH₂ (SS-31) prevents the mitochondrial potential loss of HRECs treated with high-glucose. FIG. 3A. The ΔΨm of HRECs was measured by flow cytometry after JC-1 fluorescent probe staining. High glucose (30 mM) treatment resulted in a rapid loss of mitochondrial membrane potential of the cultured HRECs at 24 and 48 hours. In contrast, flow cytometric analysis showed that 30mM glucose co-treated with D-Arg-2',6'-Dmt-Lys-Phe-NH₂ (SS-31) increased ΔΨm compared with the high glucose alone group. FIG. 3B. Quantitative analysis of ΔΨm in high glucose HRECs co-treated with D-Arg-2',6'-Dmt-Lys-Phe-NH₂ (SS-31) for 24 and 48 hours, High glucose alone adversely affected ΔΨm. In contrast, D-Arg-2',6'-Dmt-Lys-Phe-NH₂ (SS-31) restored ΔΨm to control levels. Values represent mean ± SD of six separate experiments performed in triplicate. **P<* 0.05.

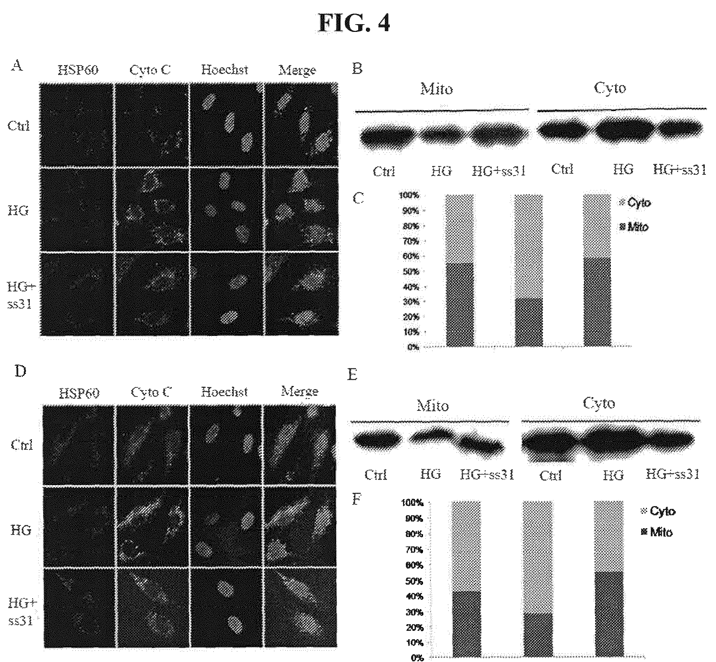

FIGs. 4A and 4D are confocal microscopic images showing that HRECs in the normal glucose group and the D-Arg-2',6'-Dmt-Lys-Phe-NH₂ (SS-31) co-treated group have more exact overlapping cytochrome c staining and HSP60 staining at 24 and 48 hours, indicating the co-localization of cytochrome c and mitochondria. Twenty four and 48 hours after treatment, cytochrome c was obviously increased in the cytoplasm of HRECs treated with 30 mM glucose. FIGs. 4B and 4E show the cytochrome c content in mitochondria and cytoplasm as determined by Western blot. FIGs. 4C and 4F show quantitative analysis of the percentage of cytochrome c content in mitochondria and cytoplasm of HRECs co-treated with high glucose and D-Arg-2',6'-Dmt-Lys-Phe-NH₂ (SS-31) for 24 and 48 h.

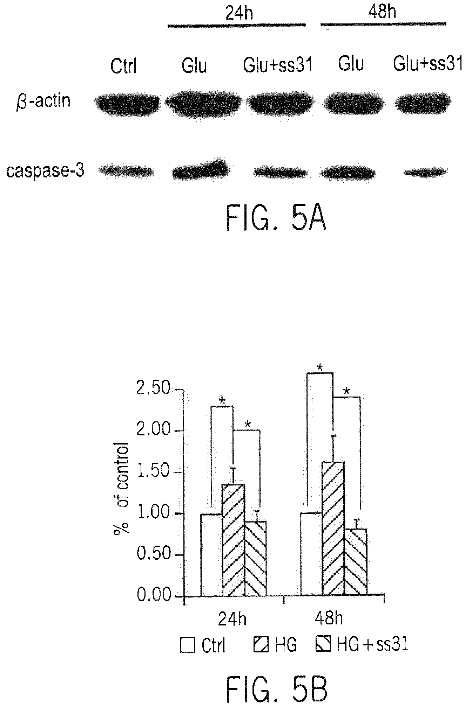

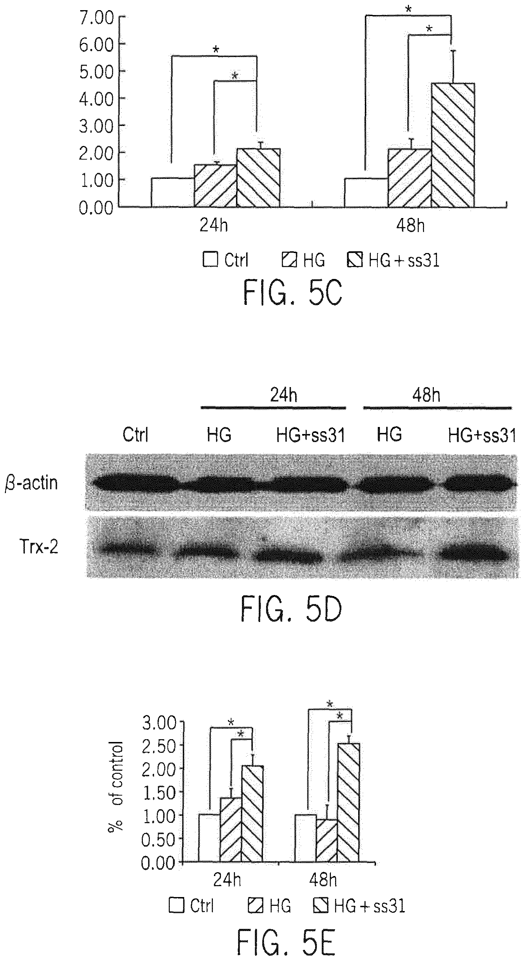

FIG. 5A and FIG. 5B show increased expression of caspase-3 in HRECs treated with high glucose (HG) was reduced by D-Arg-2',6'-Dmt-Lys-Phe-NH₂ (SS-31) co-treatment as detected by western blot. Caspase-3 expression was normalized to the expression of β-actin. FIGs. 5C-E show D-Arg-2',6'-Dmt-Lys-Phe-NH₂ (SS-31) increases the expression of Trx2 in the high glucose-treated HRECs. FIG. 5C shows the mRNA level of Trx2 in HRECs exposed to 30 mM glucose treated with D-Arg-2',6'-Dmt-Lys-Phe-NH₂ (SS-31) for 24 h and 48 h. FIG. 5D shows the level of Trx2 protein expression measured by Western blot. FIG. 5E shows quantitative analysis of the protein level of Trx2 in HRECs 24 and 48 h after high glucose with or without D-Arg-2',6'-Dmt-Lys-Phe-NH₂ (SS-31) co-treatment.
FIG. 6 is a photograph of the effects of D-Arg-2',6'-Dmt-Lys-Phe-NH₂ (SS-31) on the lenses of diabetic rats. Top row: lenses obtained from diabetic rats; bottom row: lenses obtained from diabetic rats treated with D-Arg-2',6'-Dmt-Lys-Phe-NH₂ (SS-31) or Phe-D-Arg-Phe-Lys-NH₂ (SS-20).
FIG. 7 is a series of photographs showing the effects of D-Arg-2',6'-Dmt-Lys-Phe-NH₂ (SS-31) and Phe-D-Arg-Phe-Lys-NH₂ (SS-20) on the lenses of diabetic rats. Diabetes was induced by high fat diet and streptozotocin (HFD/STZ) (top row) or streptozotocin (STZ) alone (bottom row).
FIG. 8 is a series of micrographs showing the lens epithelium from normal rats, diabetic rats, and diabetic rats treated with D-Arg-2',6'-Dmt-Lys-Phe-NH₂ (SS-31). Diabetes was induced by STZ.
FIG. 9 is a series of micrographs showing the lens epithelium from normal rats, diabetic rats, and diabetic rats treated with D-Arg-2',6'-Dmt-Lys-Phe-NH₂ (SS-31). Diabetes was induced by HFD/STZ.
FIG. 10A and 10B are charts showing the integrity of the blood-retinal barrier of normal rats (NRC), diabetic rats, and diabetic rats treated with Phe-D-Arg-Phe-Lys-NH₂ (SS-20) or D-Arg-2',6'-Dmt-Lys-Phe-NH₂ (SS-31), as analyzed by Evans blue extravasation. (A) diabetes induced by STZ; (B) diabetes induced by HFD/STZ.
FIG. 11 is a series of micrographs showing retinal microvessels of normal rats (NRC), diabetic rats (HFD/STZ), and diabetic rats treated with D-Arg-2',6'-Dmt-Lys-Phe-NH₂ (SS-31).
FIG. 12 is a series of micrographs showing retinal microvessels of normal rats, diabetic rats (STZ), and diabetic rats treated with D-Arg-2',6'-Dmt-Lys-Phe-NH₂ (SS-31).
FIGS. 13A-13D is a series of micrographs showing the distribution of the tight junction protein claudin-5 in retinal microvessels in normal rats (A), STZ rats (B), STZ/Phe-D-Arg-Phe-Lys-NH₂-treated rats (SS-20) (C), or STZ/D-Arg-2',6'-Dmt-Lys-Phe-NH₂-treated rats (SS-31) (D).
FIG. 14 is a chart showing the lack of cytotoxicity of D-Arg-2',6'-Dmt-Lys-Phe-NH₂ (SS-31) on trabecular meshwork cells from non-diseased individuals (HTM) and trabecular meshwork cells from glaucoma patients (GTM) administered D-Arg-2',6'-Dmt-Lys-Phe-NH₂ (SS-31).
FIG. 15 is a series of confocal micrographs showing co-treatment with D-Arg-2',6'-Dmt-Lys-Phe-NH₂ (SS-31) dose-dependently inhibited the decrease in mitochondrial potential (ΔΨm) elicited by 200 µM H₂O₂ in trabecular meshwork cells from glaucoma patients (GTM).
FIG. 16 is a series of charts showing co-treatment with D-Arg-2',6'-Dmt-Lys-Phe-NH₂ (SS-31) inhibited the decrease in mitochondrial membrane potential (ΔΨm), as measured by TMRM and flow cytometry, in trabecular meshwork cells from glaucoma patients (GTM) induced by 200 µM H₂O₂.
FIG. 17 is a chart comparing mitochondrial membrane potential (ΔΨm) in GTM and HTM cells.
FIG. 18 is a series of micrographs showing the morphology changes in GTM cells in response to D-Arg-2',6'-Dmt-Lys-Phe-NH₂ (SS-31) treatment as viewed using inverted phase contrast microscopy.
FIG. 19 is a series of micrographs showing co-treatment with D-Arg-2',6'-Dmt-Lys-Phe-NH₂ (SS-31) reduced the loss of mitochondrial membrane potential in GTM cells caused by 400 µM H₂O₂ in a dose-dependent manner as viewed using confocal microscopy.
FIG. 20 is a series of micrographs showing co-treatment with D-Arg-2',6'-Dmt-Lys-Phe-NH₂ (SS-31) reduced the loss of mitochondrial membrane potential (ΔΨm) in GTM cells caused by 400 µM H₂O₂ as viewed by TMRM and confocal microscopy (200x magnification).
FIG. 21 is a series of micrographs showing the morphology changes in GTM cells in response to D-Arg-2',6'-Dmt-Lys-Phe-NH₂ (SS-31) treatment as viewed using inverted phase contrast microscopy.
FIG. 22 is a chart showing that D-Arg-2',6'-Dmt-Lys-Phe-NH₂ (SS-31) had no effect on the viability of primary human retinal pigment epithelial (RPE) cells (as measured by the MTT assay).
FIG. 23A is a chart showing the effect of different concentrations of tBHP on the viability (as measured by an MTT assay) of RPE cells. FIG. 23B is a chart showing the effects of different concentrations of D-Arg-2',6'-Dmt-Lys-Phe-NH₂ (SS-31) on cell viability when exposed to increasing concentrations of tBHP.
FIG. 24A-24C is a series of micrographs illustrating the pathological effects in a choroidal neovascularization (CNV) mouse model. FIG. 24D is a graph showing CNV area in treated and control groups.
FIG. 25 is a series of micrographs illustrating different pathological findings in an oxygen-induced retinopathy (OIR) mouse model. Note areas of avascularity and new vascularization in a P 17 OIR mouse as compared to a P 17 normal mouse.
FIG. 26A-26D is a series of micrographs showing the effects of administering D-Arg-2',6'-Dmt-Lys-Phe-NH₂ (SS-31) in the OIR mouse model. FIG. 26E is a graph showing the neovascular area of the control and treated groups. D-Arg-2',6'-Dmt-Lys-Phe-NH₂ (SS-31) reduced the avascular area.
FIG. 27A is a chart showing the effect of different doses of tBHP on cell viability of a 661 W cone cell line derived from a mouse retinal tumor. FIG. 27B is a chart showing the effect of 1 µM D-Arg-2',6'-Dmt-Lys-Phe-NH₂ (SS-31) in reducing tBHP-induced 661W cell death.
FIG. 28 is a series of micrographs showing the thickness of the retinal outer nuclear layer (ONL) in a mouse model of retina degeneration in control and D-Arg-2',6'-Dmt-Lys-Phe-NH₂ (SS-31)-treated mice.
FIG. 29 is a series of micrographs showing the cone cell density in retinal flat mounts stained with peanut agglutinin (PNA), which selectively stains cone inner and outer segments in control and D-Arg-2',6'-Dmt-Lys-Phe-NH₂ (SS-31)-treated mice.
FIG. 30 is a series of micrographs showing staining for acolein, a marker for oxidative lipid damage in a mouse model of retina degeneration.
FIG. 31 is a series of graphs showing fluorescence intensity of intracellular ROS production in three groups of RPE cells using FACS analysis. FIG. 31A shows ROS production in control RPE cells; FIG. 31B shows ROS production in RPE cells treated with 500 µM tBHP for 3 h; FIG. 31C shows ROS production in RPE cells treated with 500 µM tBHP for 3 h and 1 µM D-Arg-2',6'-Dmt-Lys-Phe-NH₂ (SS-31). FIG. 31D is a bar graph showing ROS fluorescence.
FIG. 32 is a series of graphs showing analysis of MMP labeled by JC-1 in a FACS assay. Three different concentration of D-Arg-2',6'-Dmt-Lys-Phe-NH₂ (SS-31) groups were analyzed.
FIGs. 33A-33C is a series of graphs showing the effect of 1 µM D-Arg-2',6'-Dmt-Lys-Phe-NH₂ (SS-31) on MMP decline induced by tBHP. FIG. 33A: Control group; FIG. 33B: 500 µM tBHP for 3 h group; FIG. 33C: 1 µM D-Arg-2',6'-Dmt-Lys-Phe-NH₂ (SS-31) for 4 h + 500 µM tBHP for 3 h group. FIG. 33D is a chart comparing the fluorescence ratio for the different groups. *P<0.01, C vs. B.
FIGs. 34A-34C is a series of graphs showing the effect of D-Arg-2',6'-Dmt-Lys-Phe-NH₂ (SS-31) on cell apoptosis induced by 250 µM tBHP for 24 h. FIG. 34A: control group; FIG. 34B: 250 µM tBHP for 24 h group; FIG. 34C: 1 µM D-Arg-2',6'-Dmt-Lys-Phe-NH₂ (SS-31) for 4 h + 250 µM tBHP for 24 h group. FIG. 34D is a chart comparing the fluorescence ratio for the different groups. *P<0.05 C vs. B.
FIG. 35 is a chart showing the MDA level induced by tBHP in 3 groups of RPE cells. *P<0.05, 1 µM D-Arg-2',6'-Dmt-Lys-Phe-NH₂ (SS-31) for 4 h+250 µM tBHP for 24 h group vs 250 µM tBHP for 24 h.
FIG. 36 is a graph showing the fluorescence intensity of TMRM of GTM and HTM cells in control and D-Arg-2',6'-Dmt-Lys-Phe-NH₂ (SS-31)-treated groups, as measured using FACS analysis.
FIG. 37 is a graph showing the fluorescence intensity of ROS of GTM and HTM cells in control and D-Arg-2',6'-Dmt-Lys-Phe-NH₂ (SS-31)-treated groups, as measured using FACS analysis.
FIG. 38A-38D are graphs showing cell apoptosis of control and D-Arg-2',6'-Dmt-Lys-Phe-NH₂ (SS-31)-treated groups valued by percentage of cells in the Q2+Q4 quadrant.
FIG. 39A and 39B are graphs showing that D-Arg-2',6'-Dmt-Lys-Phe-NH₂ (SS-31) reduced intracellular ROS production in GTM3 and iHTM cells treated with H₂O₂.
FIG. 40A and 40B are graphs showing that D-Arg-2',6'-Dmt-Lys-Phe-NH₂ (SS-31) protected against H₂O₂-induced mitochondrial depolarization of GTM3 and iHTM cells.
FIG. 41 is a chart showing the spatial frequency (SPF) threshold in streptozotocin (STZ)-treated mice administered D-Arg-2',6'-Dmt-Lys-Phe-NH₂ (SS-31).

### DETAILED DESCRIPTION

It is to be appreciated that certain aspects, modes, embodiments, variations and features of the invention are described below in various levels of detail in order to provide a substantial understanding of the present invention.

In practicing the present invention, many conventional techniques in molecular biology, protein biochemistry, cell biology, immunology, microbiology and recombinant DNA are used. These techniques are well-known and are explained in, *e.g.,* Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989); DNA Cloning: A Practical Approach, Vols. I and II, Glover, Ed. (1985); Oligonucleotide Synthesis, Gait, Ed. (1984); Nucleic Acid Hybridization, Hames & Higgins, Eds. (1985); Transcription and Translation, Hames & Higgins, Eds. (1984); Animal Cell Culture, Freshney, Ed. (1986); Immobilized Cells and Enzymes (IRL Press, 1986); Perbal, A Practical Guide to Molecular Cloning*;* the series, Meth. Enzymol., (Academic Press, Inc., 1984); Gene Transfer Vectors for Mammalian Cells, Miller & Calos, Eds. (Cold Spring Harbor Laboratory, NY, 1987); and Meth. Enzymol., Vols. 154 and 155, Wu & Grossman, and Wu, Eds., respectively.

The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a", "an" and "the" include plural referents unless the content clearly dictates otherwise. For example, reference to "a cell" includes a combination of two or more cells, and the like.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the enumerated value.

As used herein, the "administration" of an agent, drug, or peptide to a subject includes any route of introducing or delivering to a subject a compound to perform its intended function. Administration can be carried out by any suitable route, including epicutaneously, orally, nasally, parenterally (intravenously, intramuscularly, intraperitoneally, or subcutaneously), topically, rectally, intracavernously, intradermally, transdermally, by inhalation, intraarterially, intracerebrally, interosseusly, intrathecally, intravesically, iontophoretically, ocularly, *etc.* Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g.,* homoserine, norleucine, methionine sulfoxide, methionine methyl sulfonium. Such analogs have modified R groups (*e.g.*, norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e.g.,* an amount which results in the prevention of, or a decrease in, the symptoms associated with an ophthalmic condition. The amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs or symptoms of an ophthalmic condition. For example, a "therapeutically effective amount" of the aromatic-cationic peptides is meant levels in which the physiological effects of an ophthalmic condition are, at a minimum, ameliorated.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide", "peptide" and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the terms "treating" or "treatment" or "alleviation" refers to both therapeutic treatment and prophylactic or preventative measures, wherein the object is to prevent or slow down (lessen) the targeted pathologic condition or disorder. A subject is successfully "treated" for an ophthalmic condition if, after receiving a therapeutic amount of the aromatic-cationic peptides according to the methods described herein, the subject shows observable and/or measurable reduction in or absence of one or more signs and symptoms of an ophthalmic condition. It is also to be appreciated that the various modes of treatment or prevention of medical conditions as described are intended to mean "substantial", which includes total but also less than total treatment or prevention, and wherein some biologically or medically relevant result is achieved.

As used herein, "prevention" or "preventing" of a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

### Aromatic-Cationic Peptides

The present technology relates to the treatment or prevention of an ophthalmic condition by administration of certain aromatic-cationic peptides. Without wishing to be limited by theory, the aromatic-cationic peptides may treat or prevent ophthalmic diseases or conditions by reducing the severity or occurrence of oxidative damage in the eye. The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, more preferably about nine, and most preferably about six.

The amino acids of the aromatic-cationic peptides can be any amino acid. As used herein, the term "amino acid" is used to refer to any organic molecule that contains at least one amino group and at least one carboxyl group. Typically, at least one amino group is at the position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid include hydroxyproline (Hyp).

The peptides optionally contain one or more non-naturally occurring amino acids. Suitably, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy *(i.e.,* alkoxy), amino, C₁-C₄ alkylamino and C₁-C₄ dialkylamino (*e.g*., methylamino, dimethylamino), nitro, hydroxyl, halo *(i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g.* methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are preferably resistant, and more preferably insensitive, to common proteases. Examples of non-naturally occurring amino acids that are resistant or insensitive to proteases include the dextrorotatory (D-) form of any of the above-mentioned naturally occurring L-amino acids, as well as Land/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, preferably less than four, more preferably less than three, and most preferably, less than two contiguous L-amino acids recognized by common proteases, irrespective of whether the amino acids are naturally or non-naturally occurring. Suitably, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid *(i.e.,* Glu) and four positively charged amino acids *(i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

| **TABLE 1. Amino acid number and net positive charges (3pₘ≤ p+1)** | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

| **TABLE 2. Amino acid number and net positive charges (2pₘ ≤ p+1)** | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, preferably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

| **TABLE 3. Aromatic groups and net positive charges (3a ≤ pₜ+1 or a= pₜ=1)** | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

| **TABLE 4. Aromatic groups and net positive charges (2a ≤ pt+1 or a= pₜ=1)** | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 2 | 2 | 3 | 3 | 4 | 4 | 5 | 5 | 6 | 6 | 7 | 7 | 8 | 8 | 9 | 9 | 10 | 10 |

In another embodiment, the number of aromatic groups (a) and the total number of net positive charges (pₜ) are equal.

Carboxyl groups, especially the terminal carboxyl group of a C-terminal amino acid, are preferably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

Aromatic-cationic peptides include, but are not limited to, the following peptide examples:
Lys-D-Arg-Tyr-NH₂
Phe-D-Arg-His
D-Tyr-Trp-Lys-NH₂
Trp-D-Lys-Tyr-Arg-NH₂
Tyr-His-D-Gly-Met
Phe-Arg-D-His-Asp
Tyr-D-Arg-Phe-Lys-Glu-NH₂
Met-Tyr-D-Lys-Phe-Arg
D-His-Glu-Lys-Tyr-D-Phe-Arg
Lys-D-Gln-Tyr-Arg-D-Phe-Trp-NH₂
Phe-D-Arg-Lys-Trp-Tyr-D-Arg-His
Gly-D-Phe-Lys-Tyr-His-D-Arg-Tyr-NH₂
Val-D-Lys-His-Tyr-D-Phe-Ser-Tyr-Arg-NH₂
Trp-Lys-Phe-D-Asp-Arg-Tyr-D-His-Lys
Lys-Trp-D-Tyr-Arg-Asn-Phe-Tyr-D-His-NH₂
Thr-Gly-Tyr-Arg-D-His-Phe-Trp-D-His-Lys
Asp-D-Trp-Lys-Tyr-D-His-Phe-Arg- D-Gly-Lys-NH₂
D-His-Lys-Tyr- D-Phe-Glu- D-Asp- D-His- D-Lys-Arg-Trp-NH₂
Ala-D-Phe-D-Arg-Tyr-Lys-D-Trp-His-D-Tyr-Gly-Phe
Tyr-D-His-Phe- D-Arg-Asp-Lys- D-Arg-His-Trp-D-His-Phe
Phe-Phe-D-Tyr-Arg-Glu-Asp-D-Lys-Arg-D-Arg-His-Phe-NH₂
Phe-Try-Lys-D-Arg-Trp-His-D-Lys-D-Lys-Glu-Arg-D-Tyr-Thr
Tyr-Asp-D-Lys-Tyr-Phe- D-Lys- D-Arg-Phe-Pro-D-Tyr-His-Lys
Glu-Arg-D-Lys-Tyr- D-Val-Phe- D-His-Trp-Arg-D-Gly-Tyr-Arg-D-Met-NH₂
Arg-D-Leu-D-Tyr-Phe-Lys-Glu- D-Lys-Arg-D-Trp-Lys- D-Phe-Tyr-D-Arg-Gly D-Glu-Asp-Lys-D-Arg-D-His-Phe-Phe-D-Val-Tyr-Arg-Tyr-D-Tyr-Arg-His-Phe-NH₂ Asp-Arg-D-Phe-Cys-Phe-D-Arg-D-Lys-Tyr-Arg-D-Tyr-Trp-D-His-Tyr-D-Phe-Lys-Phe His-Tyr-D-Arg-Trp-Lys-Phe-D-Asp-Ala-Arg-Cys-D-Tyr-His-Phe-D-Lys-Tyr-His-Ser-NH₂ Gly-Ala-Lys-Phe-D-Lys-Glu-Arg-Tyr-His-D-Arg-D-Arg-Asp-Tyr-Trp-D-His-Trp-His-D-Lys-Asp Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂

In one embodiment, a peptide that has mu-opioid receptor agonist activity has the formula Tyr-D-Arg-Phe-Lys-NH₂. Tyr-D-Arg-Phe-Lys-NH₂ has a net positive charge of three, contributed by the amino acids tyrosine, arginine, and lysine and has two aromatic groups contributed by the amino acids phenylalanine and tyrosine. The tyrosine of Tyr-D-Arg-Phe-Lys-NH₂ can be a modified derivative of tyrosine such as in 2',6'-dimethyltyrosine to produce the compound having the formula 2',6'-Dmt-D-Arg-Phe-Lys-NH₂. 2',6'-Dmt-D-Arg-Phe-Lys-NH2 has a molecular weight of 640 and carries a net three positive charge at physiological pH. 2',6'-Dmt-D-Arg-Phe-Lys-NH2 readily penetrates the plasma membrane of several mammalian cell types in an energy-independent manner (Zhao et al., J. Pharmacol Exp Ther. 304: 425-432, 2003).

Peptides that do not have mu-opioid receptor agonist activity generally do not have a tyrosine residue or a derivative of tyrosine at the N-terminus *(i.e.,* amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

D-Glu-Asp-Lys-D-Arg-D-His-Phe-Phe-D-Val-Tyr-Arg-Tyr-D-Tyr-Arg-His-Phe-
NH2

Asp-Arg-D-Phe-Cys-Phe-D-Arg-D-Lys-Tyr-Arg-D-Tyr-Trp-D-His-Tyr-D-Phe-
Lys-Phe

His-Tyr-D-Arg-Trp-Lys-Phe-D-Asp-Ala-Arg-Cys-D-Tyr-His-Phe-D-Lys-Tyr-His-
Ser-NH2

Gly-Ala-Lys-Phe-D-Lys-Glu-Arg-Tyr-His-D-Arg-D-Arg-Asp-Tyr-Trp-D-His-Trp-
His-D-Lys-Asp

Thr-Tyr-Arg-D-Lys-Trp-Tyr-Glu-Asp-D-Lys-D-Arg-His-Phe-D-Tyr-Gly-Val-Ile-
D-His-Arg-Tyr-Lys-NH2

An example of an aromatic-cationic peptide that does not have mu-opioid receptor agonist activity has the formula Phe-D-Arg-Phe-Lys-NH₂. Alternatively, the N-terminal phenylalanine can be a derivative of phenylalanine such as 2',6'-dimethylphenylalanine (2'6'-Dmp). Tyr-D-Arg-Phe-Lys-NH₂ containing 2',6'-dimethylphenylalanine at amino acid position 1 has the formula 2',6'-Dmp-D-Arg-Phe-Lys-NH₂. In one embodiment, the amino acid sequence of 2',6'-Dmt-D-Arg-Phe-Lys-NH2 is rearranged such that Dmt is not at the N-terminus. An example of such an aromatic-cationic peptide that does not have mu-opioid receptor agonist activity has the formula D-Arg-2'6'-Dmt-Lys-Phe-NH₂.

Aromatic-cationic peptides, and their derivatives can further include functional analogs. A peptide is considered a functional analog of an aromatic cationic peptide if the analog has the same function as the aromatic cationic peptide. The analog may, for example, be a substitution variant of the aromatic cationic peptide, wherein one or more amino acids are substituted by another amino acid.

Suitable substitution variants of an aromatic-cationic peptide include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

In some embodiments, the aromatic-cationic peptide has a formula as shown in Table 5.

| **TABLE 5. Peptide Analogs with Mu-Opioid Activity** | | | | |
|---|---|---|---|---|
| **Amino Acid Position 1** | **Amino Acid Position 2** | **Amino Acid Position 3** | **Amino Acid Position 4** | **C-Terminal Modification** |
| Tyr | D-Arg | Phe | Lys | **NH₂** |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arg | Phe | Lys | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Lys | NH₂ |
| 2'6'Dmt | D-Cit | Phe | Ahp | NH₂ |
| 2'6'Dmt | D-Arg | Phe | Orn | NH₂ |
| 2'6'Dmt | D-Arg | Phe | Dab | NH₂ |
| 2'6'Dmt | D-Arg | Phe | Dap | NH₂ |
| 2'6'Dmt | D-Arg | Phe | Ahp(2-aminoheptanoic acid) | NH₂ |
| Bio-2'6'Dmt | D-Arg | Phe | Lys | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lys | NH₂ |
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lys | NH₂ |
| 2'6'Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lvs | Phe | Dap | NH₂ |
| 3'5'Dmt | D-Lvs | Phe | Arg | NH₂ |
| Tyr | D-Lvs | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lys | Tyr | Lys | NH₂ |
| 2'6'Dmt | D-Lys | Tyr | Orn | NH₂ |
| 2'6'Dmt | D-Lvs | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lvs | 2'6'Dmt | Dab | NH₂ |
| 2'6'Dmt | D-Lys | 2'6'Dmt | Dap | NH₂ |
| 2'6'Dmt | D-Arg | Phe | dnsDap | NH₂ |
| 2'6'Dmt | D-Arg | Phe | atnDap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lvs | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = -dansyl-L- , -diaminopropionic acid atnDap = -anthraniloyl-L- , -diaminopropionic acid Bio = biotin | | | | |

Examples of other aromatic-cationic peptides that do not activate mu-opioid receptors include, but are not limited to, the aromatic-cationic peptides shown in Table 6.

**TABLE 6. Peptide Analogs Lacking Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| D-Arg | Dmt | Lys | Phe | NH₂ |
| D-Arg | Dmt | Phe | Lys | NH₂ |
| D-Arg | Phe | Lys | Dmt | NH₂ |
| D-Arg | Phe | Dmt | Lys | NH₂ |
| D-Arg | Lys | Dmt | Phe | NH₂ |
| D-Arg | Lys | Phe | Dmt | NH₂ |
| Phe | Lys | Dmt | D-Arg | NH₂ |
| Phe | Lys | D-Arg | Dmt | NH₂ |
| Phe | D-Arg | Phe | Lys | NH₂ |
| Phe | D-Arg | Dmt | Lys | NH₂ |
| Phe | D-Arg | Lys | Dmt | NH₂ |
| Phe | Dmt | D-Arg | Lys | NH₂ |
| Phe | Dmt | Lys | D-Arg | NH₂ |
| Lys | Phe | D-Arg | Dmt | NH₂ |
| Lys | Phe | Dmt | D-Arg | NH₂ |
| Lys | Dmt | D-Arg | Phe | NH₂ |
| Lys | Dmt | Phe | D-Arg | NH₂ |
| Lys | D-Arg | Phe | Dmt | NH₂ |
| Lvs | D-Arg | Dmt | Phe | NH₂ |
| D-Arg | Dmt | D-Arg | Phe | NH₂ |
| D-Arg | Dmt | D-Arg | Dmt | NH₂ |
| D-Arg | Dmt | D-Arg | Tyr | NH₂ |
| D-Arg | Dmt | D-Arg | Trp | NH₂ |
| Trp | D-Arg | Phe | Lys | NH₂ |
| Trp | D-Arg | Tyr | Lvs | NH₂ |
| Trp | D-Arg | Trp | Lys | NH₂ |
| Trp | D-Arg | Dmt | Lys | NH₂ |
| D-Arg | Trp | Lvs | Phe | NH₂ |
| D-Arg | Trp | Phe | Lys | NH₂ |
| D-Arg | Trp | Lys | Dmt | NH₂ |
| D-Arg | Trp | Dmt | Lys | NH₂ |
| D-Arg | Lys | Trp | Phe | NH₂ |
| D-Arg | Lys | Trp | Dmt | NH₂ |
| Cha | D-Arg | Phe | Lys | NH₂ |
| Ala | D-Arg | Phe | Lys | NH₂ |

| | | | | |
|---|---|---|---|---|
| Cha = cyclohexyl alanine | | | | |

The amino acids of the peptides shown in Table 5 and 6 may be in either the L- or the D- configuration. In addition to the peptides described above and any variants thereof, also provided are pharmaceutically acceptable salts of the peptides. Exemplary pharmaceutically acceptable salts include, without limitation, acetate salt and tri-flouro-acetate salt.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol. 289, Academic Press, Inc, New York (1997).

### Prophylactic and Therapeutic Uses of Aromatic-Cationic Peptides.

The eye appears to be at considerable risk from oxidative stress. There is mounting evidence indicating that dysfunctional mitochondria are the primary site of reactive oxygen species (ROS). More than 95% of O₂ produced during normal metabolism is generated by the electron transport chain in the inner mitochondrial membrane. Mitochondria are also the major target of ROS. There is need for mitochondrial targeting of compounds with universal types of antioxidant activity for treating a number of ROS-related ocular diseases and disorders. Babizhayev MA, Yegorov YE. *Reactive Oxygen Species and the Aging Eye: Specific Role of Metabolically Active Mitochondria in Maintaining Lens Function and in the Initiation of the Oxidation-Induced Maturity Onset Cataract-A Novel Platform of Mitochondria-Targeted Antioxidants With Broad Therapeutic Potential for Redox Regulation and Detoxification of Oxidants in Eye Diseases.* Am J Ther. 2010 Oct 22; Barnstable CJ. *Mitochondria and the regulation of free radical damage in the eye.* J Ocul Biol Dis Infor. 2009 Sep;2(3):145-148; Jarrett SG, et al., The importance of mitochondria in age-related and inherited eye disorders. Ophthalmic Res. 2010;44(3):179-90; Neroev W, et al., Mitochondria-targeted plastoquinone derivatives as tools to interrupt execution of the aging program. Age-related eye disease. SkQ1 returns vision to blind animals. Biochemistry (Mosc). 2008 Dec;73(12):1317-28; Zhao C, et al., Acetaminophen cytotoxicity in mouse eye: mitochondria in anterior tissues are the primary target. J Ocul Pharmacol Ther. 1997 Jun;13(3):269-76.

The aromatic-cationic peptides described herein are useful to prevent or treat disease. Specifically, the disclosure provides for both prophylactic and therapeutic methods of treating a subject at risk of (or susceptible to) an ophthalmic disease or condition, such as diabetic macular edema. Accordingly, the present methods provide for the prevention and/or treatment of an ophthalmic condition in a subject by administering an effective amount of an aromatic-cationic peptide to a subject in need thereof. For example, a subject can be administered an aromatic-cationic peptide compositions in an effort to improve one or more of the factors contributing to an ophthalmic disease or condition.

One aspect of the technology includes methods of reducing an ophthalmic condition such as diabetic macular edema in a subject for therapeutic purposes. In therapeutic applications, compositions or medicaments are administered to a subject suspected of, or already suffering from such a disease or disorder in an amount sufficient to cure, or at least partially arrest, the symptoms of the disease, including its complications and intermediate pathological phenotypes in development of the disease. As such, the disclosure provides methods of treating an individual afflicted with an ophthalmic condition.

In some embodiments, the technology provides a method of treating or preventing specific ophthalmic diseases or disorders in a mammal by administering an aromatic cationic peptide. That is, the aromatic cationic peptides of the present technology are useful to prevent or treat diseases and disorders of the eye including, but not limited to, *e.g.,* the following:

Disorders of eyelid, lacrimal system and orbit, which include, but are not limited to, *e.g.,* hordeolum ("stye" or "sty"); chalazion; blepharitis; entropion and trichiasis; ectropion; lagophthalmos; blepharochalasis; ptosis; xanthelasma of eyelid; parasitic infestation of eyelid in leishmaniasis, loiasis, onchocerciasis, and phthiriasis; dermatitis of eyelid due to Demodex species; involvement of eyelid in herpesviral (herpes simplex) infection, leprosy, *Molluscum contagiosum,* tuberculosis, yaws, zoster; involvement of eyelid in impetigo, Dacryoadenitis, Epiphora, Dysthyroid exophthalmos.

Disorders of conjunctiva, which include, but are not limited to, *e.g.,* Pterygium; subconjunctival hemorrhage; conjunctivitis due to/ or type, *e.g.,* Acanthamoeba, adenoviral follicular (acute), chlamydial, diphtheritic, gonococcal, haemorrhagic (acute)(epidemic), herpesviral (herpes simplex), meningococcal, Newcastle, zoster.

Disorders of sclera, cornea, iris and ciliary body, which include, but are not limited to, *e.g.,* scleritis; keratitis; corneal ulcer; corneal abrasion; snow blindness; Arc eye; Thygeson's superficial punctate keratopathy; corneal neovascularization; Fuchs' dystrophy; keratoconus; keratoconjunctivitis sicca; iritis; uveitis; and sympathetic ophthalmia.

Disorders of lens, which include, but are not limited to, *e.g.,* cataract.

Disorders of choroid and retina, which include, but are not limited to, *e.g.,* chorioretinal inflammation; focal, disseminated, and unspecified chorioretinal inflammation (*e.g*., chorioretinitis, choroiditis, retinitis, retinochoroiditis) ; exudative retinopathy; posterior cyclitis; Harada's disease; unspecified chorioretinal inflammation.

Other disorders of choroid, which include, but are not limited to, *e.g.,* chorioretinal scars; macula scars of posterior pole (postinflammatory) (post-traumatic); solar retinopathy; choroidal degeneration (*e.g.*, atrophy, sclerosis); angioid streaks; hereditary choroidal dystrophy; choroideremia; dystrophy (*e.g.*, choroidal (central arcolar) (generalized) (peripapillary)); gyrate atrophy, choroid; ornithinaemia; choroidal haemorrhage and rupture (not otherwise specified and expulsive); and choroidal detachment.

Chorioretinitis including, *e.g.,* syphilitic, late, toxoplasma, and tuberculous types.

Retinal detachments and breaks including retinoschisis.

Other retinal disorders which include, but are not limited to, *e.g.,* retinal vascular occlusions; hypertensive retinopathy; diabetic retinopathy; retinopathy, referring to non-inflammatory damage to the retina; retinopathy of prematurity; age-related macular degeneration; epiretinal membrane; peripheral retinal degeneration; hereditary retinal dystrophy; retinitis pigmentosa; retinal haemorrhage; separation of retinal layers; macular edema, retinal disorder (unspecified).

Retinal neuropathy such as glaucoma.

Disorders of vitreous body and globe, which include, but are not limited to, *e.g.,* floaters.

Disorders of optic nerve and visual pathways, which include, but are not limited to, *e.g.,* Leber's hereditary optic neuropathy; and optic disc drusen.

Disorders of ocular muscles, binocular movement, accommodation and refraction, which include, but are not limited to, *e.g.,* strabismus (Crossed eye/Wandering eye/Walleye); ophthalmoparesis; progressive external ophthalmoplegia; esotropia; exotropia; disorders of refraction and accommodation, *e.g.,* hypermetropia (farsightedness); myopia (nearsightedness); astigmatism; anisometropia; presbyopia; internal ophthalmoplegia.

Visual disturbances and blindness, which include, but are not limited to, *e.g.,* amblyopia (lazy eye); Leber's congenital amaurosis; scotoma (blind spot); anopsia; color blindness; achromatopsia/maskun; nyctalopia (nightblindness); blindness; river blindness; micro-opthalmia/coloboma,

Other disorders of eye and adnexa, which include, but are not limited to, adnexa, Argyll Robertson pupil. Other diseases such as keratomycosis, xerophthalmia, restasis, and aniridia.

In one embodiment, an aromatic-cationic peptide is administered to a subject to treat or prevent diabetic retinopathy. Diabetic retinopathy is characterized by capillary microaneurysms and dot hemorrhaging. Thereafter, microvascular obstructions cause cotton wool patches to form on the retina. Moreover, retinal edema and/or hard exudates may form in individuals with diabetic retinopathy due to increased vascular hyperpermeability. Subsequently, neovascularization appears and retinal detachment is caused by traction of the connective tissue grown in the vitreous body. Iris rubeosis and neovascular glaucoma may also occur which, in turn, can lead to blindness. The symptoms of diabetic retinopathy include, but are not limited to, difficulty reading, blurred vision, sudden loss of vision in one eye, seeing rings around lights, seeing dark spots, and/or seeing flashing lights.

In one embodiment, an aromatic-cationic peptide is administered to a subject to treat or prevent cataracts. Cataracts is a congenital or acquired disease characterized by a reduction in natural lens clarity. Individuals with cataracts may exhibit one or more symptoms, including, but not limited to, cloudiness on the surface of the lens, cloudiness on the inside of the lens, and/or swelling of the lens. Typical examples of congenital cataract-associated diseases are pseudo-cataracts, membrane cataracts, coronary cataracts, lamellar cataracts, punctuate cataracts, and filamentary cataracts. Typical examples of acquired cataract-associated diseases are geriatric cataracts, secondary cataracts, browning cataracts, complicated cataracts, diabetic cataracts, and traumatic cataracts. Acquired cataracts is also inducible by electric shock, radiation, ultrasound, drugs, systemic diseases, and nutritional disorders. Acquired cataracts further includes postoperative cataracts.

In one embodiment, an aromatic-cationic peptide is administered to a subject to treat or prevent retinitis pigmentosa. Retinitis pigmentosa is a disorder that is characterized by rod and/or cone cell damage. The presence of dark lines in the retina is typical in individuals suffering from retinitis pigmentosa. Individuals with retinitis pigmentosa also present with a variety of symptoms including, but not limited to, headaches, numbness or tingling in the extremities, light flashes, and/or visual changes. *See, e.g.,* Heckenlively et al., Clinical findings and common symptoms in retinitis pigmentosa. Am JOphthalmol. 105(5): 504-511 (1988).

In one embodiment, an aromatic-cationic peptide is administered to a subject to treat or prevent glaucoma. Glaucoma is a genetic disease characterized by an increase in intraocular pressure, which leads to a decrease in vision. Glaucoma may emanate from various ophthalmologic conditions that are already present in an individual, such as, wounds, surgery, and other structural malformations. Although glaucoma can occur at any age, it frequently develops in elderly individuals and leads to blindness. Glaucoma patients typically have an intraocular pressure in excess of 21 mmHg. However, normal tension glaucoma, where glaucomatous alterations are found in the visual field and optic papilla, can occur in the absence of such increased intraocular pressures, *i.e.,* greater than 21 mmHg. Symptoms of glaucoma include, but are not limited to, blurred vision, severe eye pain, headache, seeing haloes around lights, nausea, and/or vomiting.

In one embodiment, an aromatic-cationic peptide is administered to a subject to treat or prevent macular degeneration. Macular degeneration is typically an age-related disease. The general categories of macular degeneration include wet, dry, and non-aged related macular degeneration. Dry macular degeneration, which accounts for about 80-90 percent of all cases, is also known as atrophic, nonexudative, or drusenoid macular degeneration. With dry macular degeneration, drusen typically accumulate beneath the retinal pigment epithelium tissue. Vision loss subsequently occurs when drusen interfere with the function of photoreceptors in the macula. Symptoms of dry macular generation include, but are not limited to, distorted vision, center-vision distortion, light or dark distortion, and/or changes in color perception. Dry macular degeneration can result in the gradual loss of vision.

Wet macular degeneration is also known as neovascularization, subretinal neovascularization, exudative, or disciform degeneration. With wet macular degeneration, abnormal blood vessels grow beneath the macula. The blood vessels leak fluid into the macula and damage photoreceptor cells. Wet macular degeneration can progress rapidly and cause severe damage to central vision. Wet and dry macular degeneration have identical symptoms. Non-age related macular degeneration, however, is rare and may be linked to heredity, diabetes, nutritional deficits, injury, infection, or other factors. The symptoms of non-age related macular degeneration also include, but are not limited to, distorted vision, center-vision distortion, light or dark distortion, and/or changes in color perception.

In one embodiment, an aromatic-cationic peptide is administered to a subject to treat or prevent maculopathy, in particular, diabetic macular edema.

### Diabetes and maculopathy

Diabetes now affects 25.8 million people (8.3%) of the U.S. population. In 2005-2008, 4.2 million (28.5%) people with diabetes aged 40 years or older had diabetic retinopathy, and of these, 655,000 (4.4% of those with diabetes) had advanced diabetic retinopathy that could lead to severe vision loss. In a recent epidemiological study on 913 cases of non-insulin-dependent diabetes mellitus, about 8% (about 600,000 patients) of patients with diabetes mellitus are reported to have maculopathy. It is estimated that as the number of patients with diabetes mellitus increases, the number of patients with diabetic maculopathy also increases.

Diabetes is the leading cause of new cases of blindness among adults aged 20-74 years. Diabetic maculopathy, together with diabetic retinopathy, is considered to be important as one of the retinal diseases in patients with diabetes mellitus. Diabetic maculopathy is classified into macular edema, ischemic maculopathy, retinal pigment epitheliopathy and macular traction. An object of diabetic retinopathy therapy is to prevent blindness (loss of visual acuity), while an object of diabetic maculopathy therapy is to prevent and ameliorate deterioration of visual acuity.

The macula lutea is significantly different in form from other parts of the retina so as to attain high central visual acuity (sharpest and high visual acuity), and have a special structure (absent inner plexiform layer and inner nuclear layer) with a high concentration of color-sensitive cone cells. Accordingly, the clinically problematic deterioration of central visual acuity is due to maculopathy. The development of photocoagulation and vitrectomy made it possible to almost prevent blindness attributable to retinopathy, but is not satisfactory for maculopathy, so a therapy that is different from retinopathy therapy is needed for maculopathy. This is also notable as many patients have maculopathy but do not have retinopathy. Indeed, a recent increase in pan-photocoagulation as treatment for diabetic retinopathy is estimated to worsen macular edema in diabetic maculopathy, *e.g.,* to cause further deterioration of visual acuity. Accordingly, a focus of therapy includes a shift toward improvement of quality of life (QOL) of patients by maintaining and improving visual acuity.

Macular edema caused by leakage of the blood-retinal barrier comprised of retinal vascular endothelial cells and retinal pigment epithelial cells accounts for about 90% of maculopathy and is a major cause for deterioration of visual acuity in maculopathy. This deterioration of visual acuity does not lead to blindness, but causes extreme deterioration of visual acuity referred to as social blindness making usual living difficult. Given that the average life span has increased due to the advancement of medical technology, such a deterioration of visual acuity is a serious problem that cannot be neglected in consideration of QOL.

Therapies for preventing or ameliorating deterioration of visual acuity include photocoagulation, vitrectomy and chemotherapy. While photocoagulation and vitrectomy have been examined for their effectiveness in clinical studies in some contexts, the effectiveness and safety for macular edema have still not been established. Indeed, there are cases where complications of neovascular glaucoma and worsening edema occur. Therapies including steroids (with anti-inflammatory action) and carbonic anhydrase (sometimes known as carbonate dehydratase) inhibitors (anti-glaucoma activity) are used in symptomatic therapy, but their effectiveness is not established and their administration over a long period of time leads to the occurrence of side effects, and thus, the continuous use thereof in chronic diseases such as diabetes mellitus is typically not desirable.

### Diabetic macular edema

As noted above, diabetic retinopathy (DR) and diabetic macular edema (DME) are common microvascular complications in patients with diabetes and may have a sudden and debilitating impact on visual acuity, and can eventually lead to blindness. Advanced stages of DR are characterized by the growth of abnormal retinal blood vessels secondary to ischemia. These blood vessels grow in an attempt to supply oxygenated blood to the hypoxic retina. At any time during the progression of DR, patients with diabetes can also develop DME, which involves retinal thickening from swelling in the macular area. DME occurs after breakdown of the blood-retinal barrier because of leakage of plasma from dilated hyperpermeable capillaries and microaneurysms.

Macular edema in common in diabetes. The lifetime risk for diabetics to develop macular edema is about 10%. It is estimated that close to one million people in the United States alone currently have DME and approximately 300,000 new cases develop annually.

DME is closely associated with the degree of diabetic retinopathy (retinal disease). DME is the major cause of vision loss in people with diabetic retinopathy. Hypertension (high blood pressure) and fluid retention also increase the hydrostatic pressure within capillaries which drives fluid from within the vessels into the retina. A common cause of fluid retention in diabetes is kidney disease with loss of protein in the urine (proteinuria).

Diabetic macular edema (DME) manifests as swelling of the retina in diabetes mellitus due to leaking of fluid from blood vessels within the macula. These leaks cause the macula to thicken and swell, progressively distorting acute vision. While the swelling may or may not lead to blindness, the effect can cause a severe loss in central vision. As macular edema develops, blurring occurs in the middle or just to the side of the central visual field. Visual loss from diabetic macular edema can progress over a period of months and make it impossible to focus clearly.

DME is classified into two types (1) focal macular edema: caused by vascular abnormalities (primarily microaneurysms), which tend to leak fluid; (2) diffuse macular edema: caused by dilated capillaries in the retina. A correlation has been identified between diabetic macular edema and peripheral retinal ischemia.

As note above, one of the current standards of care for the treatment of DME is laser photocoagulation. Laser photocoagulation is a retinal procedure in which a laser is used to cauterize leaky blood vessels or to apply a pattern of bums to reduce edema. This procedure has undesirable side effects including partial loss of peripheral and night vision. As a result of these side effects and a desire for improved visual outcomes, retinal specialists have supplemented laser photocoagulation with non-FDA approved pharmacologic therapies for the treatment of DME, including injections of corticosteroids and anti-vascular endothelial growth factor (anti-VEGF) agents. Both of these therapies are limited by a need for multiple injections to maintain a therapeutic effect.

Disclosed herein are methods and compositions useful for the treatment, prevention or amelioration of maculopathy, and in particular, diabetic macular edema. In some embodiments, a therapeutically effective amount of an aromatic-cationic peptide such as D-Arg-2',6'-Dmt-Lys-Phe-NH₂ or a pharmaceutically acceptable salt thereof, such as acetate salt or trifluoroacetate salt is administered to a subject suffering from or at risk of maculopathy, and/or suffering from or at risk of diabetic macular edema. In some embodiments, the aromatic-cationic peptide such as D-Arg-2',6'-Dmt-Lys-Phe-NH₂ or a pharmaceutically acceptable salt thereof, is administered orally, parenterally, or topically, directly to the eye *e.g.,* as eye drops or as a cream, lotion or salve.

In one embodiment, an aromatic-cationic peptide is administered to a subject to treat or prevent choroidal neovascularization. Choroidal neovascularization (CNV) is a disease characterized by the development of new blood vessels in the choroid layer of the eye. The newly formed blood vessels grow in the choroid, through the Bruch membrane, and invade the subretinal space. CNV can lead to the impairment of sight or complete loss of vision. Symptoms of CNV include, but are not limited to, seeing flickering, blinking lights, or gray spots in the affected eye or eyes, blurred vision, distorted vision, and/or loss of vision.

In one embodiment, an aromatic-cationic peptide is administered to a subject to treat or prevent retinal degeneration. Retinal degeneration is a genetic disease that relates to the break-down of the retina. Retinal tissue may degenerate for various reasons, such as, artery or vein occlusion, diabetic retinopathy, retinopathy of prematurity, and/or retrolental fibroplasia. Retinal degradation generally includes retinoschisis, lattic degeneration, and is related to progressive macular degeneration. The symptoms of retina degradation include, but are not limited to, impaired vision, loss of vision, night blindness, tunnel vision, loss of peripheral vision, retinal detachment, and/or light sensitivity.

In one embodiment, an aromatic-cationic peptide is administered to a subject to treat or prevent oxygen-induced retinopathy. Oxygen-induced retinopathy (OIR) is a disease characterized by microvascular degeneration. OIR is an established model for studying retinopathy of prematurity. OIR is associated with vascular cell damage that culminates in abnormal neovascularization. Microvascular degeneration leads to ischemia which contributes to the physical changes associated with OIR. Oxidative stress also plays an important role in the vasoobliteration of OIR where endothelial cells are prone to peroxidative damage. Pericytes, smooth muscle cells, and perivascular astrocytes, however, are generally resistant to peroxidative injury. *See, e.g.,* Beauchamp et al., Role of thromboxane in retinal microvascular degeneration in oxygen-induced retinopathy, J Appl Physiol. 90: 2279-2288 (2001). OIR, including retinopathy of prematurity, is generally asymptomatic. However, abnormal eye movements, crossed eyes, severe nearsightedness, and/or leukocoria, can be a sign of OIR or retinopathy of prematurity.

In one aspect, an aromatic-cationic peptide is administered to a subject to treat or prevent or treat microvascular dysfunction in the eye, *e.g.,* microvascular damage associated with ocular ischemia/reperfusion injury or vascular complications due to diabetes and metabolic disorders. In one embodiment, an aromatic-cationic peptide is administered to a subject to treat or prevent or treat microvascular dysfunction due to no re-flow phenomenon associated with ocular ischemia.

### General

Diabetic macular edema (DME) and choroidal neovascularization (CNV) associated with age-related macular degeneration (AMD) are the leading causes of vision loss in the industrialized world.

*Macular edema (ME).* The macula is a small, avascular central portion of the retina rich in cones upon which sharp daytime color vision depends. Fluid can leak into the macular causing it to swell and blur vision.

*Diabetic macular edema (DME).* Diabetes is the leading cause of new blindness in the United States, and clinically significant macular edema (CSME) contributes greatly to this vision loss. About 50% of people with advanced diabetic retinopathy (proliferative retinopathy) also have macular edema, and DME is the leading cause of decreased vision from diabetic retinopathy.

DME (ICD-9-CM 362.07) is broadly classified into focal and diffuse types. This is an important difference because the two types differ in treatment. Focal macular edema is caused by foci of vascular abnormalities, primarily microaneurysms, which tend to leak fluid, whereas diffuse retinal thickening (DRT) is caused by leaking retinal capillaries throughout the retina. By the ophthalmological technique of optical coherence tomography (OCT), five morphological patterns have been described for DME: DRT (diffuse retinal thickening), cystoid macular edema (CME), posterior hyaloidal traction (PHT), serous retinal detachment (SRD) and traction retinal detachment (TRD). In one series of 119 DME patients 164 eyes 97% showed DRT and 55% were CME; other patterns were each <13%. This is consistent with the observation of that ME in the diabetic frequently takes on a pattern of CME. For all patterns, increasing retinal thickness was significantly correlated with worse visual acuity (P < .005).

*DME Pathophysiology.* DME is believed to result from diabetes-linked microvascular abnormalities, particularly leakage of retinal capillaries and reduction in the number of capillary-supporting pericytes. Hypoxia consequent to swelling is hypothesized to stimulate the production of vascular endothelial growth factor (VEGF), also known as vascular permeability factor.

*DME treatment.* The Early Treatment Diabetic Retinopathy Study (ETDRS) set the guidelines for the treatment of diabetic macular edema (DME) (7). Since that time, the standard of treatment for diabetic macular edema has been glycemic control as demonstrated by the Diabetes Control and Complications Trial (DCCT), optimal blood pressure control as demonstrated by the United Kingdom Prospective Diabetes Study (UKPDS), and macular focal/grid laser photocoagulation. In ETDRS, laser photocoagulation reduced the risk of moderate visual loss from diabetic macular edema by 50% (from 24% to 12% 3 years after initiation of treatment). Over the past few years, research has started to focus on the use of anti-vascular endothelial growth factor (VEGF) therapy to treat DME. There are currently no approved drugs for DME, but trials are underway. There are currently 51 DME studies listed in clinical trials that are known to be actively recruiting or have not yet started recruiting. Most are anti-VEGF or anti-inflammatory (steroids).

*Cystoid macular edema (CME).* CME is a morphological description of a type of ME and a final common pathway of many intraocular diseases, usually involving the retinal vasculature.

*CME Definition.* (ICD-9-CM 362.53) is any type of macular edema that involves cystoid changes in the foveal region. CME is a common, painless condition and is usually associated with blurred or distorted vision. It is frequently associated with various ocular conditions, such as cataract surgery, age-related macular degeneration (AMD), uveitis, eye injury, diabetes (DME), retinal vein occlusion, or drug toxicity.

*CME Pathophysiology.* CME associated with uveitis or following cataract surgery is most likely caused by the cytokines, *e.g.,* VEGF, prostaglandins, released by activated inflammatory cells. Other causes include diabetes (DME), wet, but not dry, AMD which cause CME by the growth of choroidal neovascular vessels (CNV) which are inherently leaky. Retinal vein occlusion (RVO) can also cause CME resulting from increased intravascular hydrostatic pressure.

*CME Treatment.* See underlying disease, *e.g.,* DME or wet AMD.

*Age*-*related Macular Degeneration (AMD, also abbreviated by some as ARMD).* AMD is classified under two forms, "dry" (ICD-9-CM 362.51) which constitute ∼85-90% of all cases, and "wet" (ICD-9-CM 362.52), which comprise ∼10-15% of all AMD cases, and is considered to be the advanced stage of dry AMD. "Dry" AMD results from the slow loss of function of the photoreceptor cells in the macula and a gradually blurring of central vision. Dry AMD goes through three stages, early, intermediate, advanced. The early and intermediate stages are associated with "drusen," yellow deposits under the retina that increase with age. However, it is unclear if the drusen are etiologically related to AMD.

*Dry AMD Pathophysiology.* The causes of dry AMD are unknown.

*Treatment of dry AMD.* A special mix of vitamins called "AREDS vitamins," that proved helpful in a clinical trial are used to prevent or delay progression from intermediate stage to the advanced stage (wet AMD).

*"Wet" AMD.* Wet AMD is considered to be advanced dry AMD. It affects central vision, lines can look distinctively "wavy" and vision loss can proceed relatively rapidly. Wet AMD pathophysiology. It is commonly associated with chroidal neovascularization (CNV) which occurs when choroidal capillaries behind the retina penetrate Bruch's membrane and begin to grow under the macula. Fluid from these leaky new blood vessels fluid raises the macula from its normal place at the back of the eye.

*Wet AMD Treatment.* Wet AMD is treated with laser surgery, photodynamic therapy, and injections into the eye of anti-inflammatory agents (steroids) and anti-VEGF agents that are injected intravitreally. Ranibizumab (Lucentis) and pegaptanib (Macugen) were approved by the FDA for wet AMD, and ranibizymab was approved for RVO in 2010. Bevacizumab (Avastin), another form of anti-VEGF antibody, has been used off label for wet AMD and shows similar efficacy as ranibizumab. Both agents are also reported to show comparable efficacy in DME. While no agent has been approved yet for DME, a variety of clinical trials with anti-VEGF antibody therapies against DME are currently underway.

Vascular disease of the eye can take many forms, including but not limited to:

Diabetes: Capillaries in the retina may be affected by diabetes and result in leakage of blood and serum exudates. Capillaries may also occlude (capillary dropout) resulting in poor circulation to the retina. Damage to the circulation of the retina is known as diabetic retinopathy. In advanced diabetic eye disease, new and fragile blood vessels may develop in the retina and cause hemorrhages in the retina or the center of the eye (vitreous).

Temporal Arteritis: Inflammation of blood vessels with a tendency to affect the important blood vessels that nourish and feed the retina. Unless the inflammation in temporal arteritis is controlled, it may affect the central retinal artery and cause sudden and permanent visual loss.

Branch Vein Occlusion: A branch from the major vein that drains the retina may be occluded by a clot. The degree of visual impairment depends on the extent and length of blockage.

Central Vein Occlusion: Blockage of the major vein that drains blood from the retina. This condition is often associated with high blood pressure.

Central Retinal Artery Occlusion: Blockage of the major artery that supplies blood to the retina.

### Vascular Damage Due to Diabetes or Metabolic Disease

It is well known that diabetes damages the vascular system. Vascular complications arising from diabetes are the leading cause of blindness, kidney failure and cardiovascular problems in the U.S. And yet, the physiological mechanisms that link diabetes, which afflicts 26 million Americans, to sickly blood vessels are poorly understood. Recent studies have shown that endothelial dysfunction leads to lethal vascular complications in diabetes and related metabolic disorders. Wei X, et al., De novo lipogenesis maintains vascular homeostasis through endothelial nitric-oxide synthase (eNOS) palmitoylation. J Biol Chem. 2011 Jan 28;286(4):2933-45. Researchers found that NOS requires palmitate synthesized by the fatty-acid synthase (FAS), an enzyme that is regulated by insulin. Without FAS, NOS cannot properly attach to the endothelium. People with diabetes have low levels of FAS due to insulin deficiency or resistance, and this FAS deficit may be at the root of their increased vulnerability to blood vessel damage. As such, disrupting eNOS bioavailability through impaired lipogenesis is a novel mechanism coordinating nutritional status and tissue repair that may contribute to diabetic vascular disease.

### Vascular Damage Due to Ischemia/Reperfusion Related Microvascular Damage

In embodiment, the present technology relates to the treatment or prevention of ocular ischemia-reperfusion injury in mammals through administration of therapeutically effective amounts of aromatic-cationic peptides to subjects in need thereof. In one embodiment, the present technology relates to method useful in the treatment, prevention or amelioration of damage to the microvessels (e.g., capillaries) of the eye following ocular ischemia/reperfusion.

In one aspect, the present technology provides a method for preventing, treating or ameliorating microvascular damage/injury in the ocular tissue of a mammalian subject in need thereof, the method comprising administering to the subject a therapeutically effective amount of a aromatic-cationic peptide of the present technology, *e.g.,* D-Arg-2'6'-Dmt-Lys-Phe-NH₂, or a pharmaceutically acceptable salt thereof, thereby preventing or treating microvasular injury in the subject. In one embodiment, the method further comprises the step of performing a revascularization procedure on the subject. In one embodiment, the damage/injury to the microvessels of the eye include, but are not limited to, *e.g.,* occlusion, weakening, disruption resulting in leakage; destructions; and/or proliferation of new vessels. In one embodiment, the mammalian subject is at risk for, or suffering from ocular ischemia. In one embodiment, the subject is at risk for, or suffering from, an anatomic zone of no re-flow associated with ocular tissue. In one embodiment, the anatomic zone of no re-flow has a disruption or obstruction of the microvasculature of the subject. In one embodiment, the subject is administered the aromatic-cationic peptide of the present technology prior to ocular ischemia. In one embodiment, the subject is administered the aromatic peptide of the present technology after ocular ischemia.

In one embodiment, the subject is administered the aromatic-cationic peptide prior to the revascularization procedure. In another embodiment, the subject is administered the aromatic-cationic peptide after the revascularization procedure. In another embodiment, the subject is administered the aromatic-cationic peptide during and after the revascularization procedure. In yet another embodiment, the subject is administered the aromatic-cationic peptide continuously before, during, and after the revascularization procedure.

In one embodiment, the subject is administered the aromatic-cationic peptide for at least 3 hours, at least 5 hours, at least 8 hours, at least 12 hours, or at least 24 hours after the revascularization procedure. In one embodiment, the subject is administered the aromatic-cationic peptide starting at least 8 hours, at least 4 hours, at least 2 hours, at least 1 hour, or at least 10 minutes prior to the revascularization procedure.

In various embodiments, the subject is suffering from an ocular infarction, a stroke, or is in need of a revascularization procedure. In one embodiment, the revascularization procedure is removal of an occlusion. In one embodiment, the revascularization procedure is administration of one or more thrombolytic agents. In one embodiment, the one or more thrombolytic agents are selected from the group consisting of: tissue plasminogen activator; urokinase; prourokinase; streptokinase; acylated form of plasminogen; acylated form of plasmin; and acylated streptokinase-plasminogen complex.

In one aspect, the invention provides a method for preventing, in a subject, an ophthalmic condition by administering to the subject an aromatic-cationic peptide that modulates one or more signs or markers of an ophthalmic condition. Subjects at risk for an ophthalmic condition can be identified by, *e.g.,* any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. Depending upon the type of aberrancy, *e.g.,* an aromatic-cationic peptide which acts to enhance or improve mitochondrial function or reduce oxidative damage can be used for treating the subject. The appropriate compound can be determined based on screening assays described herein.

*Ocular Administration of Aromatic-Cationic Peptides of the Technology to Prevent or Treat Neurodegenrative Disease of Disorders.* In one aspect, the technology includes methods of using the ocular route of administration of a therapeutically effective amount of a one or more aromatic-cationic peptides of the present invention (*e.g*., D-Arg-2',6'-Dmt-Lys-Phe-NH₂ and/or a pharmaceutically acceptable salt thereof) for treating a neurodegenerative disease or condition to a subject in need thereof. In one embodiment, ocular route of administration is topical application of an aromatic-cationic peptide of the present invention suitably formulated for such application to ocular tissue of a subject in need therof. In therapeutic applications, compositions or medicaments are administered ocularly to a subject suspected of, or already suffering from such a disease or condition in an amount sufficient to cure, or at least partially arrest, the symptoms of the disease or condition, including its complications and intermediate pathological phenotypes in development of the disease or condition. As such, the disclosure provides methods of treating an individual afflicted with a neurodegenerative disease or condition. The methods of the present invention can also be used in reducing oxidative damage associated with any neurodegenerative disease or condition. The neurodegenerative disease can affect any cell, tissue or organ of the central and peripheral nervous system. Examples of such cells, tissues and organs include, the brain, spinal cord, neurons, ganglia, Schwann cells, astrocytes, oligodendrocytes and microglia.

The neurodegenerative condition can be an acute condition, such as a stroke or a traumatic brain or spinal cord injury. In another embodiment, the neurodegenerative disease or condition can be a chronic neurodegenerative condition. In a chronic neurodegenerative condition, the free radicals can, for example, cause damage to a protein. An example of such a protein is amyloid .beta.-protein. Examples of chronic neurodegenerative diseases associated with damage by free radicals include Parkinson's disease, Alzheimer's disease, Huntington's disease and Amyotrophic Lateral Sclerosis (also known as Lou Gherig's disease).

In some embodiments, the technology provides a method of treating or preventing specific neurodegenarative diseases, such as Alzheimer's disease, Parkinson's disease, Huntington's disease, and amyotrophic lateral sclerosis (ALS), in a mammal by ocularly administering an aromatic cationic peptide.

*Determination of the Biological Effect of the Aromatic-Cationic Peptide-Based Therapeutic.* In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative cells of the type(s) involved in the subject's disorder, to determine if a given aromatic-cationic peptide-based therapeutic exerts the desired effect upon the cell type(s). Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects. In one embodiment, administration of an aromatic-cationic peptide to a subject exhibiting symptoms associated with an ophthalmic condition will cause an improvement in one or more of those symptoms.

### Modes of Administration and Effective Dosages

Any method known to those in the art for contacting a cell, organ or tissue with a peptide may be employed. Suitable methods *include in vitro, ex vivo, or in vivo* methods. *In vivo* methods typically include the administration of an aromatic-cationic peptide, such as those described above, to a mammal, preferably a human. When used *in vivo* for therapy, the aromatic-cationic peptides are administered to the subject in effective amounts (*i.e.,* amounts that have desired therapeutic effect). The dose and dosage regimen will depend upon the degree of the ophthalmic condition in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods of the present invention, preferably in a pharmaceutical composition, may be administered to a mammal in need thereof by any of a number of well-known methods for administering pharmaceutical compounds. In some embodiments, the peptide is administered systemically. In some embodiments, the peptide is administered locally. In some embodiments, the peptide is administered epicutaneously, orally, nasally, parenterally (intravenously, intramuscularly, intraperitoneally, or subcutaneously), topically, rectally, intracavernously, intradermally, transdermally, by inhalation, intraarterially, intracerebrally, interosseusly, intrathecally, intravesically, iontophoretically, ocularly, *etc.* Administration includes self-administration and the administration by another.

For opthalmic applications, the therapeutic aromatic-cationic peptide is delivered in a therapeutically effective amount to select parts of the eye, including posterior chamber, ora serrata, ciliary muscle , ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina. In some embodiments, a therapeutically effective amount of an aromatic-cationic peptide delivered to a part of the eye is a concentration 10⁻¹³ to 10⁻⁶ molar, e.g., approximately 10⁻⁷ molar. In some embodiments, a therapeutically effective amount of an aromatic-cationic peptide delivered to a part of the eye is a concentration 10⁻¹¹ to 10⁻⁶; 10⁻⁹ to 10⁻⁶; or 10⁻⁷ to 10-⁶ molar; 10⁻⁶ to 10⁻¹ molar; 10⁻⁴ to 10⁻¹ molar; or 10⁻² to 10⁻¹ molar. In some embodiment, a therapeutically effective amount of an aromatic-cationic peptide delivered to a part of the eye is a concentration 10⁻¹ molar to 10¹ molar. The schedule of doses would be optimized to maintain the therapeutic concentration at the target tissue, most preferably by single daily or weekly administration, but also including continuous administration (*e.g.,* parenteral infusion or transdermal application).

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (e.g., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g.,* vials of drug, vials of diluent, syringes and needles) for a treatment course.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it may be desirable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

For ophthalmic applications, the therapeutic compound is formulated into solutions, suspensions, and ointments appropriate for use in the eye. For ophthalmic formulations generally, see Mitra (ed.), Ophthalmic Drug Delivery Systems, Marcel Dekker, Inc., New York, N.Y. (1993) and also Havener, W. H., Ocular Pharmacology, C.V. Mosby Co., St. Louis (1983). Ophthalmic pharmaceutical compositions may be adapted for topical administration to the eye in the form of solutions, suspensions, ointments, creams or as a solid insert. For a single dose, from between 0.1 ng to 5000 µg, 1 ng to 500 µg, or10 ng to 100 µg of the aromatic-cationic peptides can be applied to the human eye.

A topical formulation may be in any form suitable for topical administration, including, without being limited thereto, an ophthalmic solution (e.g. eye drops), an ophthalmic gel or an ophthalmic ointment or oily lotion. Topical administration of the aromatic-cationic peptide of the present technology also comprises the use of ophthalmic patches carrying a one or more aromatic-cationic peptide in a suitable drug containing layer and to be placed on top of the eyelid as well as to ocular inserts which are devices containing the one or more aromatic-cationic peptide and placed into the inferior or superior conjunctival sacs (see for example WO0059420).

Eye drops may be prepared by dissolving a one or more aromatic-cationic peptide of the present technology in a sterile aqueous solution such as saline, buffering solution *etc.,* or by combining powder compositions to be dissolved before use. Other additives may be included in the eye drops such as isotonizing agents *(e.g.,* sodium chloride, *etc.),* buffer agent *(e.g.,* boric acid, sodium monohydrogen phosphate, sodium dihydrogen phosphate, *etc.),* preservatives *(e.g.,* benzalkonium chloride, benzethonium chloride, chlorobutanol, *etc.),* thickeners *(e.g.,* saccharide such as lactose, mannitol, maltose, *etc.; e.g.,* hyaluronic acid or its salt such as sodium hyaluronate, potassium hyaluronate, *etc*.; *e.g.,* mucopolysaccharide such as chondroitin sulfate, *etc*.; *e.g.,* sodium polyacrylate, carboxyvinyl polymer, crosslinked polyacrylate, *etc.).*

Eye ointments may be prepared by mixing a one or more aromatic-cationic peptide D-Arg-2',6'-Dmt-Lys-Phe-NH₂, or a pharmaceutically acceptable salt thereof, into a base. Examples of the base for eye ointment include petrolatum, selen 50, Plastibase, macrogol, *etc.,* but are not limited thereto.

Some exemplary ophthalmic viscosity enhancers that can be used in the present formulation include: carboxymethyl cellulose sodium; methylcellulose; hydroxypropyl cellulose; hydroxypropylmethyl cellulose; hydroxyethyl cellulose; polyethylene glycol 300; polyethylene glycol 400; polyvinyl alcohol; and providone.

Some natural products, such as veegum, alginates, xanthan gum, gelatin, acacia and tragacanth, may also be used to increase the viscosity of ophthalmic solutions.

A tonicity is important because hypotonic eye drops cause an edema of the cornea, and hypertonic eye drops cause deformation of the cornea. The ideal tonicity is approximately 300 mOsM. The tonicity can be achieved by methods described in Remington: The Science and Practice of Pharmacy, known to those versed in the art.

The aromatic cationic peptides of the present technology are useful in the treatment of dry eye syndrome. The present technology provides topical formulations comprising the aromatic-cationic peptides which are useful in the prevention, treatment or amelioration of dry eye in a subject in need thereof.

The aromatic cationic peptides of the present technology are useful in the treatment of maculopathy, including but not limited to, *e.g.,* diabetic macular edema. The present technology provides topical formulations comprising the aromatic-cationic peptides which are useful in the-prevention, treatment or amelioration of maculopathy, including but not limited to diabetic macular edema, in a subject in need thereof.

In one embodiment, the present technology provided an ophthalmic delivery system suitable for ocular delivery of aromatic-cationic peptide of the present technology, e.g., for the treatment, prevention or amelioration of maculopathy, including but not limited to diabetic macular edema, in an isotonic unit dose application can be formulated. An illustrative embodiment of such a delivery system using D-Arg-2',6'-Dmt-Lys-Phe-NH₂ or a pharmaceutically acceptable salt thereof, is described in Example 9.

In one embodiment, the present technology provides an ophthalmic delivery system: isotonic solution for multiple dose ophthalmic application using an aromatic-cationic peptide of the present technology, e.g., for the treatment, prevention or amelioration of maculopathy, including but not limited to diabetic macular edema. An illustrative embodiment of such a delivery system using D-Arg-2',6'-Dmt-Lys-Phe-NH₂ or a pharmaceutically acceptable salt thereof, is provided in Example 10.

In one embodiment, the present technology provides an ophthalmic delivery system: isotonic solution for multiple or single dose use ophthalmic application stabilized against heavy metals using an aromatic-cationic peptide of the present technology, e.g., for the treatment, prevention or amelioration of maculopathy, including but not limited to diabetic macular edema. An illustrative embodiment of such a delivery system using D-Arg-2',6'-Dmt-Lys-Phe-NH₂ or a pharmaceutically acceptable salt thereof is provided in Example 11.

In one embodiment, the present technology provides an ophthalmic delivery system: isotonic solution for multiple or single dose use ophthalmic application with extended life using an aromatic-cationic peptide of the present technology e.g., for the treatment, prevention or amelioration of maculopathy, including but not limited to diabetic macular edema. An illustrative embodiment of such a delivery system using D-Arg-2',6'-Dmt-Lys-Phe-NH₂ or a pharmaceutically acceptable salt thereof is provided in Example 12.

In one embodiment, the present technology provides an ophthalmic delivery system utilizing viscous solutions or thermosetting gel for unit or multiple dose ophthalmic application using an aromatic-cationic peptide of the present technology *e.g.,* for the treatment, prevention or amelioration of maculopathy, including but not limited to diabetic macular edema. An illustrative embodiment of such a delivery system using D-Arg-2',6'-Dmt-Lys-Phe-NH₂ or a pharmaceutically acceptable salt thereof is provided in Example 13.

In one embodiment, the present technology provides an ophthalmic delivery system utilizing a liposomal emulsion to protect the aromatic-cationic peptide of the present technology from proteolysis *e.g.,* for the treatment, prevention or amelioration of maculopathy, including but not limited to diabetic macular edema. An illustrative embodiment of such a system using D-Arg-2',6'-Dmt-Lys-Phe-NH₂ or a pharmaceutically acceptable salt thereof is shown in Example 14.

In one embodiment, the present technology provides an ophthalmic delivery system comprising aromatic-cationic peptide of the present technology entrapped in albumin microspheres for slow release e.g., for the treatment, prevention or amelioration of maculopathy, including but not limited to diabetic macular edema. An illustrative embodiment of such a system using D-Arg-2',6'-Dmt-Lys-Phe-NH₂ or a pharmaceutically acceptable salt thereof is shown in Example 15.

In one embodiment, the present technology provides an ophthalmic delivery system comprising aromatic-cationic peptides of the present technology entrapped in injectable PLA/PGA microspheres for depot release of the aromatic-cationic peptide in the ophthalmic tissues *e.g.,* for the treatment, prevention or amelioration of maculopathy, including but not limited to diabetic macular edema. An illustrative embodiment of such a system using D-Arg-2',6'-Dmt-Lys-Phe-NH₂ or a pharmaceutically acceptable salt thereof is shown in Example 16.

In one embodiment, the present technology provides an ophthalmic delivery system comprising an aromatic-cationic peptide of the present technology in a slowly eroding, biodegradable film to deliver slow release of the aromatic-cationic peptide topically or via implant e.g., for the treatment, prevention or amelioration of maculopathy, including but not limited to diabetic macular edema. An illustrative embodiment of such a system using D-Arg-2',6'-Dmt-Lys-Phe-NH₂ or a pharmaceutically acceptable salt thereof is shown in Example 17.

All the above referenced delivery systems may comprise one or more aromatic-cationic peptides in combination with one or more other therapeutically effective drugs.

In some embodiments, a therapeutic formulation includes an aromatic-cationic peptide of the present technology or a pharmaceutically acceptable salt thereof, such as an acetate salt or a tri-fluoro-acetate salt, and a pharmaceutical excipient. In some embodiments, a formulation includes D-Arg-2',6'-Dmt-Lys-Phe-NH₂ or a pharmaceutically acceptable salt thereof, such as actetate salt or tri-fluoro-acetate salt and one or more pharmaceutical excipients (*e.g.,* a pharmacologically inactive substance used as a carrier for the peptide).

The ophthalmic preparation *e.g.,* for the treatment, prevention or amelioration of maculopathy, including but not limited to diabetic macular edema, may contain non-toxic auxiliary substances such as antibacterial components which are non-injurious in use, for example, thimerosal, benzalkonium chloride, methyl and propyl paraben, benzyldodecinium bromide, benzyl alcohol, or phenylethanol; buffering ingredients such as sodium chloride, sodium borate, sodium acetate, sodium citrate, or gluconate buffers; and other conventional ingredients such as sorbitan monolaurate, triethanolamine, polyoxyethylene sorbitan monopalmitylate, ethylenediamine tetraacetic acid, and the like.

The ophthalmic solution or suspension *e.g.,* for the treatment, prevention or amelioration of maculopathy, including but not limited to diabetic macular edema, may be administered as often as necessary to maintain an acceptable level of the aromatic-cationic peptide in the eye. Administration to the mammalian eye may be about once or twice daily.

Oral compositions *e.g.,* for the treatment, prevention or amelioration of maculopathy, including but not limited to diabetic macular edema,-generally include an inert diluent or an edible carrier. For the purpose of oral therapeutic administration, the active compound can be incorporated with excipients and used in the form of tablets, troches, or capsules, *e.g.,* gelatin capsules. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation,-*e.g*., for the treatment, prevention or amelioration of maculopathy, including but not limited to diabetic macular edema, the compounds can be delivered in the form of an aerosol spray from a pressurized container or dispenser which contains a suitable propellant, *e.g.,* a gas such as carbon dioxide, or a nebulizer. Such methods include those described in U.S. Pat. No. 6,468,798.

Systemic administration of a therapeutic compound as described herein e.g., for the treatment, prevention or amelioration of maculopathy, including but not limited to diabetic macular edema,-can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed by iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. *(See* Lichtenberg et al., Methods Biochem. Anal., 33:337-462 (1988); Anselem et al., Liposome Technology, CRC Press (1993)). Liposomal formulations can delay clearance and increase cellular uptake *(See* Reddy, Ann. Pharmacother., 34 (7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34 (7-8):915-923 (2000)). A polymer formulation for human growth hormone (hGH) has been used in clinical trials. *(See* Kozarich and Rich, Chemical Biology, 2:548-552 (1998)).

A one or more aromatic-cationic peptide of the present technology is suitable for formulation with one or more of the excipients detailed in Younic et al., An assessment of the ocular safety of inactive excipients following sub-tenon injection in rabbits .J Ocul Pharmacol Ther. 2008 Apr;24(2):206-16.

Examples of polymer microsphere sustained release formulations are described in PCT publication WO 99/15154 (Tracy et al.), U.S. Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e.g.,* from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods 4 (3) 201-9 (1994); and Gregoriadis, "Engineering Liposomes for Drug Delivery: Progress and Problems," Trends Biotechnol. 13 (12):527-37 (1995). Mizguchi et al., Cancer Lett. 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Preferably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. Intervals can also be irregular as indicated by measuring blood levels of glucose or insulin in the subject and adjusting dosage or administration accordingly. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹¹ to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, most preferably by single daily or weekly administration, but also including continuous administration (*e.g.,* parenteral infusion or transdermal application).

In some embodiments, the dosage of the aromatic-cationic peptide is provided at a "low," "mid," or "high" dose level. In one embodiment, the low dose is provided from about 0.0001 to about 0.5 mg/kg/h, suitably from about 0.01 to about 0.1 mg/kg/h. In one embodiment, the mid-dose is provided from about 0.1 to about 1.0 mg/kg/h, suitably from about 0.1 to about 0.5 mg/kg/h. In one embodiment, the high dose is provided from about 0.5 to about 10 mg/kg/h, suitably from about 0.5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In a preferred embodiment, the mammal is a human.

### Combination Therapy with an Aromatic-Cationic Peptide and Other Therapeutic Agents

In certain instances of treating or preventing or ameliorating maculopathy, including but not limited to diabetic macular edema, it may be appropriate to administer at least one of the aromatic-cationic peptides described herein (or a pharmaceutically acceptable salt, ester, amide, prodrug, or solvate) in combination with another therapeutic agent. By way of example only, if one of the side effects experienced by a patient upon receiving one of the aromatic-cationic peptides herein is inflammation, then it may be appropriate to administer an anti-inflammatory agent in combination with the initial therapeutic agent. Or, by way of example only, the therapeutic effectiveness of one of the compounds described herein may be enhanced by administration of an adjuvant (*i.e.,* by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit in the prevention or treatment of ophthalmic conditions. By way of example only, in a treatment for macular degeneration involving administration of one of the aromatic-cationic peptides described herein, increased therapeutic benefit may result by also providing the patient with other therapeutic agents or therapies for macular degeneration. In any case, regardless of the ophthalmic disease, disorder or condition being treated, the overall benefit experienced by the patient may simply be additive of the two therapeutic agents or the patient may experience a synergistic benefit.

Specific, non-limiting examples of possible combination therapies include use of at least one aromatic-cationic peptide with nitric oxide (NO) inducers, statins, negatively charged phospholipids, antioxidants, minerals, anti-inflammatory agents, anti-angiogenic agents, matrix metalloproteinase inhibitors, and carotenoids. In several instances, suitable combination agents may fall within multiple categories (by way of example only, lutein is an antioxidant and a carotenoid). Further, the aromatic-cationic peptides may also be administered with additional agents that may provide benefit to the patient, including by way of example only cyclosporin A.

In addition, the aromatic-cationic peptides may also be used in combination with procedures that may provide additional or synergistic benefit to the patient, including, by way of example only, the use of extracorporeal rheopheresis (also known as membrane differential filtration), the use of implantable miniature telescopes, laser photocoagulation of drusen, and microstimulation therapy.

The use of antioxidants has been shown to benefit patients with macular degenerations and dystrophies. *See, e.g.,* Arch. Ophthalmol., 119: 1417-36 (2001); Sparrow, et al., J. Biol. Chem., 278:18207-13 (2003). Examples of suitable antioxidants that could be used in combination with at least one aromatic-cationic peptide include vitamin C, vitamin E, beta-carotene and other carotenoids, coenzyme Q, 4-hydroxy-2,2,6,6-tetramethylpiperidine-N-oxyl (also known as Tempol), lutein, butylated hydroxytoluene, resveratrol, a trolox analogue (PNU-83836-E), and bilberry extract.

The use of certain minerals has also been shown to benefit patients with macular degenerations and dystrophies. *See, e.g.,* Arch. Ophthalmol., 119: 1417-36 (2001). Examples of suitable minerals that could be used in combination with at least one aromatic-cationic peptide include copper-containing minerals, such as cupric oxide; zinc-containing minerals, such as zinc oxide; and selenium-containing compounds.

The use of certain negatively-charged phospholipids has also been shown to benefit patients with macular degenerations and dystrophies. *See, e.g.,* Shaban & Richter, Biol. Chem., 383:537-45 (2002); Shaban, et al., Exp. Eye Res., 75:99-108 (2002). Examples of suitable negatively charged phospholipids that could be used in combination with at least one aromatic-cationic peptide include cardiolipin and phosphatidylglycerol. Positively-charged and/or neutral phospholipids may also provide benefit for patients with macular degenerations and dystrophies when used in combination with aromatic-cationic peptides.

The use of certain carotenoids has been correlated with the maintenance of photoprotection necessary in photoreceptor cells. Carotenoids are naturally-occurring yellow to red pigments of the terpenoid group that can be found in plants, algae, bacteria, and certain animals, such as birds and shellfish. Carotenoids are a large class of molecules in which more than 600 naturally occurring carotenoids have been identified. Carotenoids include hydrocarbons (carotenes) and their oxygenated, alcoholic derivatives (xanthophylls). They include actinioerythrol, astaxanthin, canthaxanthin, capsanthin, capsorubin, β-8'-apo-carotenal (apo-carotenal), β-12'-apo-carotenal, α-carotene, β-carotene, "carotene" (a mixture of α- and β-carotenes), γ-carotenes, β-cyrptoxanthin, lutein, lycopene, violerythrin, zeaxanthin, and esters of hydroxyl- or carboxyl-containing members thereof. Many of the carotenoids occur in nature as cis- and trans-isomeric forms, while synthetic compounds are frequently racemic mixtures.

In humans, the retina selectively accumulates mainly two carotenoids: zeaxanthin and lutein. These two carotenoids are thought to aid in protecting the retina because they are powerful antioxidants and absorb blue light. Studies with quails establish that groups raised on carotenoid-deficient diets had retinas with low concentrations of zeaxanthin and suffered severe light damage, as evidenced by a very high number of apoptotic photoreceptor cells, while the group with high zeaxanthin concentrations had minimal damage. Examples of suitable carotenoids for in combination with at least one aromatic-cationic peptide include lutein and zeaxanthin, as well as any of the aforementioned carotenoids.

Suitable nitric oxide inducers include compounds that stimulate endogenous NO or elevate levels of endogenous endothelium-derived relaxing factor (EDRF) *in vivo* or are substrates for nitric oxide synthase. Such compounds include, for example, L-arginine, L-homoarginine, and N-hydroxy-L-arginine, including their nitrosated and nitrosylated analogs (e.g., nitrosated L-arginine, nitrosylated L-arginine, nitrosated N-hydroxy-L-arginine, nitrosylated N-hydroxy-L-arginine, nitrosated L-homoarginine and nitrosylated L-homoarginine), precursors of L-arginine and/or physiologically acceptable salts thereof, including, for example, citrulline, ornithine, glutamine, lysine, polypeptides comprising at least one of these amino acids, inhibitors of the enzyme arginase (*e.g.,* N-hydroxy-L-arginine and 2(S)-amino-6-boronohexanoic acid) and the substrates for nitric oxide synthase, cytokines, adenosine, bradykinin, calreticulin, bisacodyl, and phenolphthalein. EDRF is a vascular relaxing factor secreted by the endothelium, and has been identified as nitric oxide or a closely related derivative thereof (Palmer et al, Nature, 327:524-526 (1987); Ignarro et al, Proc. Natl. Acad. Sci. USA, 84:9265-9269 (1987)).

Statins serve as lipid-lowering agents and/or suitable nitric oxide inducers. In addition, a relationship has been demonstrated between statin use and delayed onset or development of macular degeneration. G. McGwin, et al., British Journal of Ophthalmology, 87:1121-25 (2003). Statins can thus provide benefit to a patient suffering from an ophthalmic condition (such as the macular degenerations and dystrophies, and the retinal dystrophies) when administered in combination with aromatic-cationic peptides. Suitable statins include, by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin.

Suitable anti-inflammatory agents with which the aromatic-cationic peptides may be used include, by way of example only, aspirin and other salicylates, cromolyn, nedocromil, theophylline, zileuton, zafirlukast, montelukast, pranlukast, indomethacin, and lipoxygenase inhibitors; non-steroidal antiinflammatory drugs (NSAIDs) (such as ibuprofen and naproxin); prednisone, dexamethasone, cyclooxygenase inhibitors (*i.e.,* COX-1 and/or COX-2 inhibitors such as Naproxen™, or Celebrex™); statins (by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin); and disassociated steroids.

Suitable matrix metalloproteinases (MMPs) inhibitors may also be administered in combination with aromatic-cationic peptides in order to treat ophthalmic conditions or symptoms associated with macular or retinal degenerations. MMPs are known to hydrolyze most components of the extracellular matrix. These proteinases play a central role in many biological processes such as normal tissue remodeling, embryogenesis, wound healing and angiogenesis. However, excessive expression of MMP has been observed in many disease states, including macular degeneration. Many MMPs have been identified, most of which are multidomain zinc endopeptidases. A number of metalloproteinase inhibitors are known (see for example the review of MMP inhibitors by Whittaker M. et al, Chemical Reviews 99(9):2735-2776 (1999)). Representative examples of MMP Inhibitors include Tissue Inhibitors of Metalloproteinases (TIMPs) *(e.g.,* TIMP-1, TIMP-2, TIMP-3, or TIMP-4), α-2-macroglobulin, tetracyclines (*e.g.,* tetracycline, minocycline, and doxycycline), hydroxamates (*e.g.,* BATIMASTAT, MARIMISTAT and TROCADE), chelators (*e.g.,* EDTA, cysteine, acetylcysteine, D-penicillamine, and gold salts), synthetic MMP fragments, succinyl mercaptopurines, phosphonamidates, and hydroxaminic acids. Examples of MMP inhibitors that may be used in combination with aromatic cationic peptides include, by way of example only, any of the aforementioned inhibitors.

The use of antiangiogenic or anti-VEGF drugs has also been shown to provide benefit for patients with macular degenerations and dystrophies. Examples of suitable antiangiogenic or anti-VEGF drugs that could be used in combination with at least one aromatic-cationic peptide include Rhufab V2 (Lucentis™), Tryptophanyl-tRNA synthetase (TrpRS), Eye001 (Anti-VEGF Pegylated Aptamer), squalamine, Retaane™ 15 mg (anecortave acetate for depot suspension; Alcon, Inc.), Combretastatin A4 Prodrug (CA4P), Macugen™, Mifeprex™ (mifepristone--ru486), subtenon triamcinolone acetonide, intravitreal crystalline triamcinolone acetonide, Prinomastat (AG3340--synthetic matrix metalloproteinase inhibitor, Pfizer), fluocinolone acetonide (including fluocinolone intraocular implant, Bausch & Lomb/Control Delivery Systems), VEGFR inhibitors (Sugen), and VEGF-Trap (Regeneron/Aventis).

Other pharmaceutical therapies that have been used to relieve visual impairment can be used in combination with at least one aromatic-cationic peptide. Such treatments include but are not limited to agents such as Visudyne™ with use of a non-thermal laser, PKC 412, Endovion (NeuroSearch A/S), neurotrophic factors, including by way of example Glial Derived Neurotrophic Factor and Ciliary Neurotrophic Factor, diatazem, dorzolamide, Phototrop, 9-cis-retinal, eye medication (including Echo Therapy) including phospholine iodide or echothiophate or carbonic anhydrase inhibitors, AE-941 (AEterna Laboratories, Inc.), Sirna-027 (Sirna Therapeutics, Inc.), pegaptanib (NeXstar Pharmaceuticals/Gilead Sciences), neurotrophins (including, by way of example only, NT-4/5, Genentech), Cand5 (Acuity Pharmaceuticals), ranibizumab (Genentech), INS-37217 (Inspire Pharmaceuticals), integrin antagonists (including those from Jerini AG and Abbott Laboratories), EG-3306 (Ark Therapeutics Ltd.), BDM-E (BioDiem Ltd.), thalidomide (as used, for example, by EntreMed, Inc.), cardiotrophin-1 (Genentech), 2-methoxyestradiol (Allergan/Oculex), DL-8234 (Toray Industries), NTC-200 (Neurotech), tetrathiomolybdate (University of Michigan), LYN-002 (Lynkeus Biotech), microalgal compound (Aquasearch/Albany, Mera Pharmaceuticals), D-9120 (Celltech Group p1c), ATX-S10 (Hamamatsu Photonics), TGF-beta 2

(Genzyme/Celtrix), tyrosine kinase inhibitors (Allergan, SUGEN, Pfizer), NX-278-L (NeXstar Pharmaceuticals/Gilead Sciences), Opt-24 (OPTIS France SA), retinal cell ganglion neuroprotectants (Cogent Neurosciences), N-nitropyrazole derivatives (Texas A&M University System), KP-102 (Krenitsky Pharmaceuticals), and cyclosporin A.

In any case, the multiple therapeutic agents may be administered in any order or even simultaneously. If simultaneously, the multiple therapeutic agents may be provided in a single, unified form, or in multiple forms (by way of example only, either as a single solution or as two separate solutions). One of the therapeutic agents may be given in multiple doses, or both may be given as multiple doses. If not simultaneous, the timing between the multiple doses may vary from more than zero weeks to less than about four weeks, less than about six weeks, less than about 2 months, less than about 4 months, less than about 6 months, or less than about one year. In addition, the combination methods, compositions and formulations are not to be limited to the use of only two agents. By way of example only, an aromatic-cationic peptide may be provided with at least one antioxidant and at least one negatively charged phospholipid; or an aromatic-cationic peptide may be provided with at least one antioxidant and at least one inducer of nitric oxide production; or an aromatic-cationic peptide may be provided with at least one inducer of nitric oxide productions and at least one negatively charged phospholipid; and so forth.

In addition, an aromatic-cationic peptide may also be used in combination with procedures that may provide additional or synergistic benefits to the patient. Procedures known, proposed or considered to relieve visual impairment include but are not limited to "limited retinal translocation", photodynamic therapy (including, by way of example only, receptor-targeted PDT, Bristol-Myers Squibb, Co.; porfimer sodium for injection with PDT; verteporfin, QLT Inc.; rostaporfin with PDT, Miravent Medical Technologies; talaporfin sodium with PDT, Nippon Petroleum; motexafin lutetium, Pharmacyclics, Inc.), antisense oligonucleotides (including, by way of example, products tested by Novagali Pharma SA and ISIS-13650, Isis Pharmaceuticals), laser photocoagulation, drusen lasering, macular hole surgery, macular translocation surgery, implantable miniature telescopes, Phi-Motion Angiography (also known as Micro-Laser Therapy and Feeder Vessel Treatment), Proton Beam Therapy, microstimulation therapy, Retinal Detachment and Vitreous Surgery, Scleral Buckle, Submacular Surgery, Transpupillary Thermotherapy, Photosystem I therapy, use of RNA interference (RNAi), extracorporeal rheopheresis (also known as membrane differential filtration and Rheotherapy), microchip implantation, stem cell therapy, gene replacement therapy, ribozyme gene therapy (including gene therapy for hypoxia response element, Oxford Biomedica; Lentipak, Genetix; PDEF gene therapy, GenVec), photoreceptor/retinal cells transplantation (including transplantable retinal epithelial cells, Diacrin, Inc.; retinal cell transplant, Cell Genesys, Inc.), and acupuncture.

Further combinations that may be used to benefit an individual include using genetic testing to determine whether that individual is a carrier of a mutant gene that is known to be correlated with certain ophthalmic conditions. By way of example only, defects in the human ABCA4 gene are thought to be associated with five distinct retinal phenotypes including Stargardt disease, cone-rod dystrophy, age-related macular degeneration and retinitis pigmentosa. See *e.g.,* Allikmets et al., Science, 277:1805-07 (1997); Lewis et al., Am. J. Hum. Genet., 64:422-34 (1999); Stone et al., Nature Genetics, 20:328-29 (1998); Allikmets, Am. J Hum. Gen., 67:793-799 (2000); Klevering, et al., Ophthalmology, 11 1:546-553 (2004). In addition, an autosomal dominant form of Stargardt Disease is caused by mutations in the ELOV4 gene. *See* Karan, et al., Proc. Natl. Acad. Sci. (2005). Patients possessing any of these mutations are expected to find therapeutic and/or prophylactic benefit in the methods described herein.

### EXAMPLES

The present invention is further illustrated by the following examples, which should not be construed as limiting in any way.

### Example 1 -Ocular Pharmacokinetics and Disposition of [dimethyltyrosine-2-¹⁴C] D-Arg-2',6'-Dmt-Lys-Phe-NH₂ Following Subcutaneous Dosing and Topical Instillation Into the Eyes of New Zealand White Rabbits

This example demonstrates the ocular pharmacokinetics and disposition of [dimethyltyrosine-2-¹⁴C] D-Arg-2',6'-Dmt-Lys-Phe-NH₂ and its derived total radioactivity following subcutaneous dosing and topical instillation into the eyes of New Zealand White rabbits.

Sixteen New Zealand White rabbits were dosed with a formulation consisting of 10 mg/mL of ¹⁴C-labeled D-Arg-2',6'-Dmt-Lys-Phe-NH₂. Animals were at least twelve weeks old and weighed 2.30-3.43 kg at the time of dosing. Animals were randomized to treatment groups according to Table 7.

Dosing solutions were prepared by combining 14.70 mg (correction factor of 0.782) of non-radiolabeled D-Arg-2',6'-Dmt-Lys-Phe-NH₂, and 17.49 mg (correction factor of 0.770) of ¹⁴C-labeled D-Arg-2',6'-Dmt-Lys-Phe-NH₂, and dissolving in 2.5 mL of 0.01M PBS, pH 7.4 to achieve a concentration of 9.97 mg/mL (1 %, free peptide) and a radiolabeled concentration of 460.1 Ci/mL. Three aliquots (100 µL) of dosing solution were brought to a volume of 25 mL with saline. Duplicate 100 µL aliquots of the diluted solutions were quantified for radioactivity by liquid scintillation counting (LSC). The measured radioactivity was found to be within 5% of the theoretical value. The dosing solutions were stored at 2-8°C for up to 24 hours prior to use, or at-70°C for future use.

| **TABLE 7. Ocular Pharmacokinetics and Disposition of [dimethyltyrosine-2-¹⁴C] D-Arg-2',6'-Dmt-Lys-Phe-NH₂ Following Subcutaneous Dosing and Topical Instillation Into the Eves of New Zealand White Rabbits** | | | | | | |
|---|---|---|---|---|---|---|
| Group | N | Treatment | D-Arg-2',6'-Dmt-Lys-Phe-NH₂ Dose Both Eyes (mg)¹ or Total Dose² | Dose Volume/eye¹ or Total Dose Volume² | Route | Blood Collection³ |
| A | 2 | ¹⁴C-D-Arg-2',6'-Dmt-Lys-Phe-NH₂ (OU) | 0.80 | 40 µl | Topical | 15 min. |
| B | 2 | ¹⁴C-D-Arg-2',6'-Dmt-Lys-Phe-NH₂ (OU) | 0.80 | 40 µl | Topical | 30 min. |
| C | 2 | ¹⁴C-D-Arg-2',6'-Dmt-Lys-Phe-NH₂ (OU) | 0.80 | 40 µl | Topical | 2 hrs. |
| D | 2 | ¹⁴C-D-Arg-2',6'-Dmt-Lys-Phe-NH₂ (OU) | 0.80 | 40 µl | Topical | 4 hrs. |
| E | 2 | ¹⁴C-D-Arg-2',6'-Dmt-Lys-Phe-NH₂ (OU) | 0.80 | 40 µl | Topical | 6 hrs. |
| F | 2 | ¹⁴C-D-Arg-2',6'-Dmt-Lys-Phe-NH₂ (OU) | 0.80 | 40 µl | Topical | 8 hrs. |
| G | 2 | ¹⁴C-D-Arg-2',6'-Dmt-Lys-Phe-NH₂ (OU) | 0.80 | 40 µl | Topical | 24 hrs. |
| H | 1 | ¹⁴C-D-Arg-2',6'-Dmt-Lys-Phe-NH₂ | 1 mg/kg | 0.1 mL/kg | Subcutaneous | 1 hr. |
| I | 1 | ¹⁴C-D-Arg-2',6'-Dmt-Lys-Phe-NH₂ | 1 mg/kg | 0.1 mL/kg | Subcutaneous | 2 hrs. |
| ¹Dose administered by topical instillation to eyes (Groups A-G), delivered as 1% (10 ²mg/ml) D-Arg-2',6'-Dmt-Lys-Phe-NH₂ solution and ≈18.4 µCi/eye | | | | | | |
| ²Dose administered by subcutaneous injection (Groups H-I) | | | | | | |
| ³ Immediately following blood collection, animals were euthanized OU = both eyes | | | | | | |

On Day 1, 40 µL of the 1% (10 mg/mL) D-Arg-2',6'-Dmt-Lys-Phe-NH₂ dosing solution was topically instilled into both eyes of each animal in Groups A-G. Each animal in Groups H-I received a single subcutaneous dose of 0.1 mL/kg (1 mg/kg) of the dosing solution into the dorsal scapular region. At the designated time, each animal was anesthetized and blood was collected via cardiac puncture. Animals were euthanized following cardiac puncture. Approximately 3 ± 0.5 mL of whole blood was collected in a K2EDTA tube. Four aliquots (100 µL) were directly transformed to combustion cones. Blood aliquots were stored at ≤-20°C until analyzed.

For Groups A-G, the right globe and one left globe from each group (N = 3 globes) were collected and necropsied. The remaining left eye (except for Group G) was collected for possible 3D-QWBA (Quantitative whole body autoradiography) analysis and the conjunctiva remained intact to the eyes. For Groups H-I, both globes were collected from each animal and necropsied. All eyes designated for necropsy were rinsed with normal saline and aqueous humor samples were collected. The globes were enucleated and frozen in liquid nitrogen and stored at ≤ -80°C prior to dissection. The vitreous humor, cornea, conjunctiva, iris/ciliary body, lens, retina, choroid, and sclera were collected from the appropriate eye, weighed, and stored at ≤-20°C prior to analysis. Aqueous humor samples were also stored at ≤-20°C until analyzed.

All radioactivity measurements were performed using a Beckman Liquid Scintillation Spectrometer. Two blood aliquots were combusted and two aliquots were reserved for combustion if needed. From each eye, the cornea, conjunctiva, iris/ciliary body, lens, retina, choroid, and sclera from each eye were weighed into combustion cones and combusted. Duplicate aliquots of each aqueous humor sample and each vitreous humor sample were transferred to scintillation vials using Insta-Gel as the scintillation fluid, and the amount of radioactivity was determined.

### Results

After topical instillation of ¹⁴C-labeled D-Arg-2',6'-Dmt-Lys-Phe-NH₂ to both eyes, radioactivity was observed in all tissues of the eye and the blood of each animal from the first time point to the last, 15 minutes through 24 hours post-dosing. The results demonstrate the rapid increase in the equivalent drug concentration in the tissues of the eye, in particular the conjunctiva (7425 ± 5344 ng-equi/g), cornea (1976 ± 359 ng-equi/g), and sclera (917 ± 466 ng-equi/g), where they reached a maximum at 30 minutes post-dosing. Other tissues of the eye including the choroid, lens and retina reached lesser maximum equivalent drug concentrations, 208 ± 95 ng- equi/g, 185 ± 75 ng- equi/g, and 96.7 ± 22.7 ng- equi/g, respectively. These maxima were also reached over a greater time period, at 4 hours post-dosing. At their peak concentration, the equivalent drug content of the conjunctiva, cornea and sclera amounted to 0.293±0.198%, 0.028±0.005%, and 0.051±0.035%, respectively, of the total dose administered to the eye.

After subcutaneous dosing of ¹⁴C-labeled D-Arg-2',6'-Dmt-Lys-Phe-NH₂, radioactivity was present in blood at concentrations of 356 ng- equi/g at 1 hour and 87.9 ng- equi/g at 2 hours after dosing. At 1 hour post-dosing, the equivalent drug concentration was greatest in the conjunctiva (650 ± 12 ng- equi/g), choroid (558 ± 251 ng- equi/g), sclera (530 ± 47 ng- equi/g), iris/ciliary body (513 ± 2 ng- equi/g), retina (336 ± 3 ng- equi/g) and cornea (209 ng- equi/g). D-Arg-2',6'-Dmt-Lys-Phe-NH₂-derived radioactivity was present at lesser concentrations in the other tissues of the eye. The concentrations in the tissues diminished to varying degrees at 2 hours, except for the aqueous humor and the lens, which increased moderately. A summary of the pharmacokinetics parameters after ocular administration is presented in Table 8.

| **TABLE 8 Pharmacokinetic Parameters of Total Radioactivity Following Instillation of 14C-D-Arg-2',6'-Dmt-Lys-Phe-NH₂ (1% solution, 40 µL/eye) in the Eyes of New Zealand White Rabbits** | | | | | |
|---|---|---|---|---|---|
| Tissue | Cₘₐₓ (ng-equi/g) | Tₘₐₓ(hr) | T_{1/2} (hr) | AUC₀₋₂₄ₕ (hr*ng-equi/g) | AUC_{inf} (hr*ng-equi/g) |
| Aqueous humor | 314 | 4.0 | 4.60 | 2600 | 2680 |
| Blood | 116 | 2.0 | 4.36 | 1210 | 1250 |
| Choroid | 208 | 4.0 | 8.21 | 1900 | 2190 |
| Conjunctiva | 7425 | 0.5 | 13.6 | 41,400 | 56,000 |
| Cornea | 1976 | 0.5 | ND | 14,000 | ND |
| Iris/Ciliary Body | 343 | 4.0 | 8.81 | 2750 | 3279 |
| Lens | 185 | 4.0 | ND | 2720 | ND |
| Retina | 97.0 | 4.0 | 7.01 | 1030 | 1160 |
| Sclera | 917 | 0.5 | 18.3 | 5780 | 8910 |
| Vitreous humor | 20.0 | 4.0 | 7.50 | 221 | 253 |

| | | | | | |
|---|---|---|---|---|---|
| Cₘₐₓ = maximum concentration Tmax = time Of Cₘₐₓ T_{1/2} = apparent terminal half-life AUC₀₋₂₄ₕ = area under curve from first to last data points AUC_{inf} = area under curve from zero time to infinite time ND: not determined, insufficient data to characterize the terminal phase | | | | | |

These results demonstrate that the methods and compositions described herein are useful for the ocular instillation of aromatic-cationic peptides for the treatment or prevention of ophthalmic conditions. The results show that aromatic-cationic peptides of the present technology, such as D-Arg-2',6'-Dmt-Lys-Phe-NH₂, may be administered ocularly for the treatment or prevention of ophthalmic conditions, such as diabetic macular edema.

### Example 2 -Prevention of High Glucose Induced Injury of Human Retinal Epithelial Cells

The effects of the aromatic-cationic peptides of the invention in preventing high glucose induced injury in human retinal epithelial cells (HREC) were investigated in cultured HRECs.

Methods of HREC culture useful in the studies of the present invention are known. *See generally,* Li B, Tang SB, Zhang G, Chen JH, Li BJ. Culture and characterization of human retinal capillary endothelial cell. Chin Ophthal Res 2005; 23: 20-2; Premanand C, Rema M, Sameer MZ, Sujatha M, Balasubramanyam M. Effect of curcumin on proliferation of human retinal endothelial cells under in vitro conditions. Invest Ophthalmol Vis Sci 2006; 47: 2179-84.

Briefly, HREC cells were divided into three groups: a normal control group; a group administered 30 mM glucose; and a group administered 30 mM glucose + D-Arg-2',6'-Dmt-Lys-Phe-NH₂. Survival of HRECs in high glucose co-treated with different concentrations of D-Arg-2',6'-Dmt-Lys-Phe-NH₂ (10 nM, 100 nM, 1 µM, 10 µM) was measured using an Annexin V+PI assay and flow cytometry. *See generally,* Koopman, G., Reutelingsperger, C. P., Kuijten, G. A. M., Keehnen, R. M. J., Pals, S. T., and van Oers, M. H. J. 1994. Annexin V for flow cytometric detection of phosphatidylserine expression on B cells undergoing apoptosis. Blood 84: 1415; Homburg, C. H., de Haas, M., von dem Borne, A. E., Verhoeven, A. J., Reutelingsperger, C. P., and Roos, D. 1995. Human neutrophils lose their surface Fc gamma RIII and acquire Annexin V binding sites during apoptosis in vitro. Blood 85: 532; Vermes, I., Haanen, C., Steffens-Nakken, H., and Reutelingsperger, C. 1995. A novel assay for apoptosis - flow cytometric detection of phosphatidylserine expression on early apoptotic cells using fluorescein labelled Annexin V. J. Immunol. Meth. 184: 39; Fadok, V. A., Voelker, D. R., Campbell, P. A., Cohen, J. J., Bratton, D. L., and Henson, P. M. 1992. Exposure of phosphatidylserine on the surface of apoptotic lymphocytes triggers specific recognition and removal by macrophages. J. Immunol. 148: 2207.

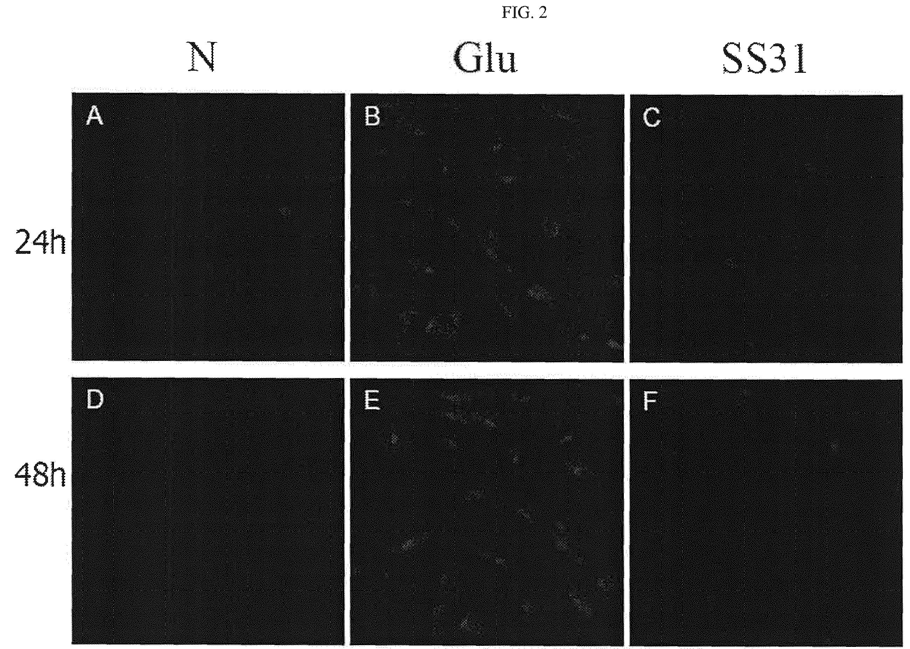

The survival of HRECs in high glucose co-treated with D-Arg-2',6'-Dmt-Lys-Phe-NH₂ was tested at 24 h and 48 h. The results are shown in FIG. 1 and indicate that survival of HRECs was significantly improved with the administration of D-Arg-2',6'-Dmt-Lys-Phe-NH₂, with a reduction in apoptotic and necrotic cells. The treatment of D-Arg-2',6'-Dmt-Lys-Phe-NH₂ also reduced the production of ROS (FIG. 2).

Assessment of D-Arg-2',6'-Dmt-Lys-Phe-NH₂ as a protectant against mitochondrial potential loss of HRECs treated with high-glucose was examined. To determine if a mitochondrial-mediated pathway was important in the protective effect of D-Arg-2',6'-Dmt-Lys-Phe-NH₂ against high glucose-induced cell death, ΔΨm was measured by flow cytometry. After treating the HRECs with high-glucose without D-Arg-2',6'-Dmt-Lys-Phe-NH₂ for 24 or 48 hours, a rapid loss of mitochondrial membrane potential was detected by JC-1 fluorescent probe as indicated by a significant decrease in the ratio of red to green fluorescence observed in the high glucose group. In contrast, ΔΨm in the 100 nM D-Arg-2',6'-Dmt-Lys-Phe-NH₂ co-treated group remained virtually unchanged and was comparable to the normal glucose control group (FIG. 3). These data suggest that D-Arg-2',6'-Dmt-Lys-Phe-NH₂ prevented the mitochondrial membrane potential loss caused by exposure to a high glucose environment.

Glucose (30 mmol/L) induced cytochrome c release from the mitochondria of HRECs. Fixed HRECs were immunolabeled with a cytochrome c antibody and a mitochondrial specific protein antibody (HSP60). Confocal microscopic analysis showed that HRECs in normal culture and in D-Arg-2',6'-Dmt-Lys-Phe-NH₂ co-treated with glucose have overlapping cytochrome c staining and mitochondria staining, indicating colocalization of cytochrome c and mitochondria (FIG. 4). After treatment with 30 mmol/L glucose for 24 h or 48 h, some cytochrome c was observed in the cytoplasm of HRECs, indicating that glucose induces the release of cytochrome c from the mitochondria to cytoplasm in HREC cells, but D-Arg-2',6'-Dmt-Lys-Phe-NH₂ can decrease such translocation between mitochondria and cytoplasm.

The prevention of cytochrome c release from mitochondria resulted in reduced caspase-3 activity. As shown in FIG. 5, D-Arg-2',6'-Dmt-Lys-Phe-NH₂ decreased the protein expression of caspase-3 in high glucose-treated HRECs. The level of cleaved caspase-3 protein expression was measured by Western blot (FIG. 5A). When HRECs were exposed to 30 mM glucose for 24 h and 48 h, the level of caspase-3 expression increased dramatically. At the same time, in the D-Arg-2',6'-Dmt-Lys-Phe-NH₂ co-treated group, it displayed a marked decrease in the caspase-3 protein level (*p<0.05). FIG. 5B shows a quantitative analysis the level of caspase-3 expression of HRECs in high glucose co-treated with D-Arg-2',6'-Dmt-Lys-Phe-NH₂ for 24 and 48h.

D-Arg-2',6'-Dmt-Lys-Phe-NH₂ increased the expression of Trx2 in the high glucose-treated HRECs. FIG. 5C shows the mRNA level of Trx2 in HRECs exposed to 30 mM glucose co-treated with D-Arg-2',6'-Dmt-Lys-Phe-NH₂ for 24 h and 48 h. The mRNA expression level of Trx2 was measured by quantitative real-time PCR. Relative mRNA levels of Trx2 were normalized by 18S mRNA levels (* p<0.05 vs. the normal glucose medium group and the 30 mM high glucose treated group). Three independent samples were used for each time point. FIG. 5D shows the level of Trx2 protein expression as measured by Western blot. The protein expression of Trx2 in the high glucose co-treated with D-Arg-2',6'-Dmt-Lys-Phe-NH₂ group significantly increased comparing to the normal glucose group (*p<0.05). FIG. 5E shows quantitative analysis of the protein level of Trx2 in HRECs 24 and 48 h after high glucose without or with D-Arg-2',6'-Dmt-Lys-Phe-NH₂ co-treatment.

These results show that D-Arg-2',6'-Dmt-Lys-Phe-NH₂ is useful for promoting the survival of HREC cells in a high glucose environment. These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of high glucose-induced injury of human retinal epithelial cells in mammals in need thereof.

### Example 3 - Prevention of Diabetic Retinopathy in Rats Fed a High Fat Diet

The effects of the aromatic-cationic peptides of the invention in preventing the development of diabetic retinopathy were investigated in a Sprague-Dawley rat model. The example describes the results of such experiments.

A rat model of diabetes was established by combination of 6-week HFD and low dose of STZ (30 mg/kg) injection or a single high dose of STZ (65 mg/kg) in SD rats. *See generally,* K. Srinivasan, B. Viswanad, Lydia Asrat, C.L. Kaul and P. Ramarao, Combination of high-fat diet-fed and low-dose streptozotocin-treated rat: A model for type 2 diabetes and pharmacological screening, Pharmacological Research, 52(4): 313-320, 2005. Rats of the same batch fed with normal chow (NRC) were used as a control. Tables 9-12 show the therapeutic schedule and experimental protocol.

| **TABLE 9. Treatment Groups - HFD/STZ Model** | | | | |
|---|---|---|---|---|
| **Group** | **Number of Rats** | **Model** | **Treatment** | **Dosage and Route** |
| A | 12 | HFD/STZ | D-Arg-2',6'-Dmt-Lys-Phe-NH₂ | 10 mg/kg s.c |
| B | 12 | HFD/STZ | D-Arg-2',6'-Dmt-Lys-Phe-NH₂ | 3 mg/kg s.c. |
| C | 12 | HFD/STZ | D-Arg-2',6'-Dmt-Lys-Phe-NH₂ | 1 mg/kg s.c. |
| D | 10 | HFD/STZ | Phe-D-Arg-Phe-Lys-NH₂ | 10 mg/kg s.c. |
| E | 10 | HFD/STZ | Phe-D-Arg-Phe-Lys-NH₂ | 3 mg/kg s.c. |
| F | 10 | HFD/STZ | Saline | Equal vol. s.c. |
| G | 10 | NRC | Saline | Equal vol. s.c. |

| **TABLE 10. Therapeutic Schedule - HFD/STZ Model** | | | |
|---|---|---|---|
| **Duration** | **Objective** | **Diabetic Groups (A,B,C,D,E,F)** | **Control Group (G)** |
| 1^{st} Week | Acclimation | Normal rat chow | |
| 2^{nd} - 7^{th} Week | Diet Manipulation | High Fat Diet | Normal Rat Chow |
| End of 7^{th} Week | STZ Injection | STZ 30 mg/kg, i.p., once | Citrate buffer |
| 8^{th} - 27^{th} Week | Induction of Diabetes | High fat diet until 21^{st} week, then switched to normal rat chow | Normal rat chow |
| 28^{th} - 37^{th} Week | Peptide Treatment | Peptide treatment (see Table 9) | Group G: 2 mL/kg, s.c. |
| 38^{th} Week | Collected 24h urine and blood samples, and harvested vital organs | | |

| **TABLE 11. Treatment Groups -STZ Model** | | | | |
|---|---|---|---|---|
| **Group** | **Number of Rats** | **Model** | **Treatment** | **Dosage and Route** |
| A | 11 | Diabetes | D-Arg-2',6'-Dmt-Lys-Phe-NH₂ | 10 mg/kg s.c |
| B | 11 | Diabetes | Phe-D-Arg-Phe-Lys-NH₂ | 10 mg/kg s.c. |
| C | 10 | Diabetes | Saline | Equal vol. s.c. |
| D | 10 | Normal | Saline | Equal vol. s.c. |

| **TABLE 12. Therapeutic Schedule -STZ Model** | | | |
|---|---|---|---|
| **Duration** | **Objective** | **Diabetic Groups (A,B,C)** | **Control Group (D)** |
| 1^{st} - 3^{rd} Week | Acclimation | Normal rat chow | |
| End of 3^{rd} Week | STZ Injection | STZ 30 mg/kg, i.p., once | Citrate buffer |
| 4^{th} - 18^{th} Week | Induction of Diabetic Complications | Normal Rat Chow | |
| 19^{h} - 28^{th} Week | Peptide Treatment | Peptide treatment (see Table 11) | Group D: 2 mL/kg, s.c. |
| 29^{th} Week | Collected 24h urine and blood samples, and harvested vital organs | | |

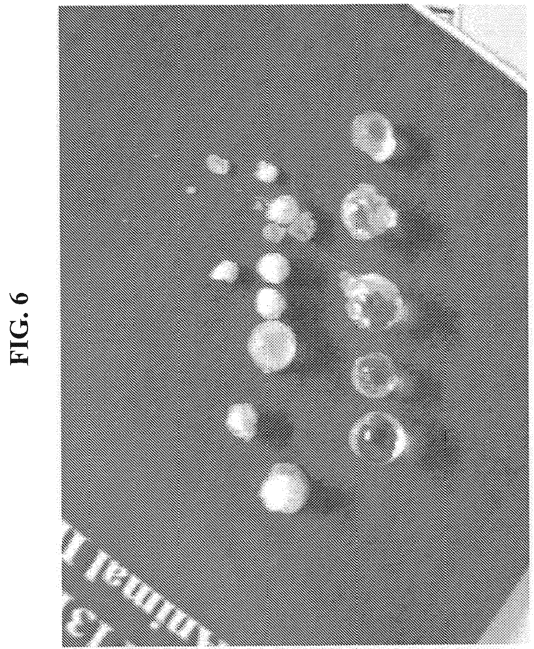

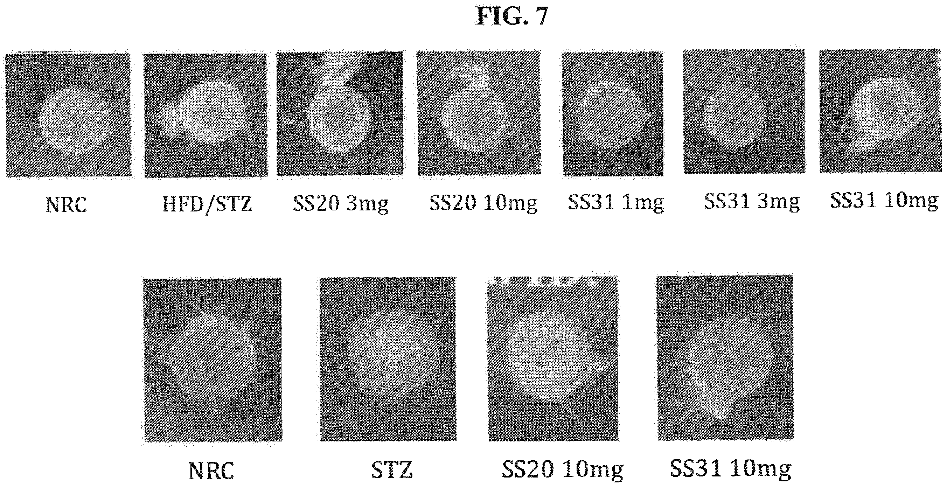

In accordance with the experimental protocol just described, the effects of the aromatic-cationic peptides in treating conditions associated with diabetes in a SD rat model were demonstrated. Administration of Phe-D-Arg-Phe-Lys-NH₂ and D-Arg-2',6'-Dmt-Lys-Phe-NH₂ resulted in a prevention or reversal of cataract formation in the lenses of diabetic rats (FIGs. 6 and 7, Tables 13 and 14).

| **TABLE 13. HFD/STZ Rat Model** | | | | | | | |
|---|---|---|---|---|---|---|---|
| Group | Turbidity degree | | | | | Percentage of opacity (%) | Percentage of severe opacity (%) |
| | - | + | ++ | +++ | ++++ | | |
| NRC | 4 | 0 | 0 | 0 | 0 | 0 | 0 |
| HFD/STZ | 1 | 0 | 2 | 3 | 0 | 83.3 | 0 |
| Phe-D-Arg-Phe-Lys-NH₂ 3mg | 1 | 1 | 1 | 0 | 1 | 75.0 | 25.0 |
| Phe-D-Arg-Phe-Lys-NH₂ 10mg | 1 | 2 | 1 | 0 | 0 | 75.0 | 0 |
| D-Arg-2',6'-Dmt-Lys-Phe-NH₂ 1mg | 1 | 1 | 1 | 0 | 0 | 67.7 | 0 |
| D-Arg-2',6'-Dmt-Lys-Phe-NH₂ 3mg | 3 | 1 | 0 | 0 | 1 | 20.0 | 20.0 |
| D-Arg-2',6'-Dmt-Lys-Phe-NH₂ 10mg | 6 | 1 | 0 | 0 | 0 | 14.3 | 0 |

| **TABLE 14. STZ Rat Model** | | | | | | | |
|---|---|---|---|---|---|---|---|
| Group | Opacity degree | | | | | Percentage of opacity (%) | Percentage of severe opacity (%) |
| | - | + | ++ | +++ | ++++ | | |
| NRC | 6 | 0 | 0 | 0 | 0 | 0 | 0 |
| STZ | 1 | 0 | 0 | 1 | 3 | 80.0 | 60.0 |
| Phe-D-Arg-Phe-Lys-NH₂ 10mg | 2 | 0 | 2 | 0 | 1 | 60.0 | 20.0 |
| D-Arg-2',6'-Dmt-Lys-Phe-NH₂ 10mg | 2 | 2 | 0 | 0 | 1 | 60.0 | 20.0 |

| | | | | | | | |
|---|---|---|---|---|---|---|---|
| -: transparent; +: mildly opaque; ++: opaque; +++: moderately opaque; ++++: severely opaque | | | | | | | |

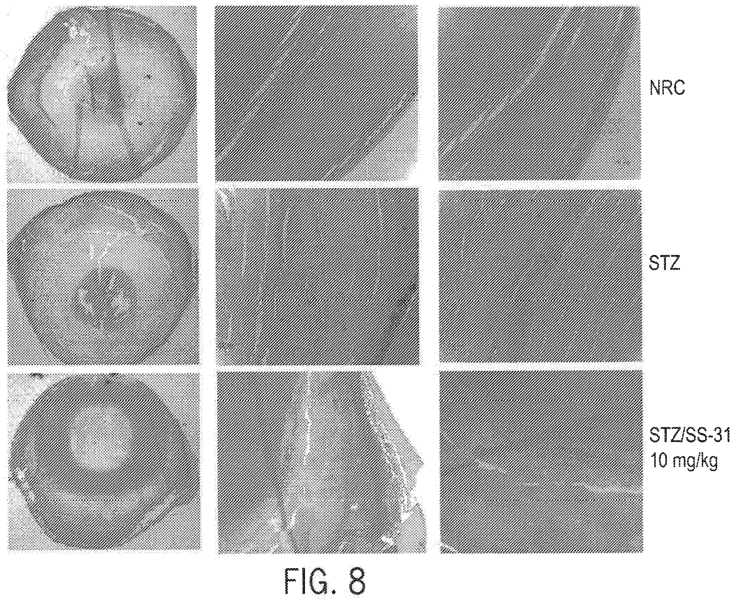

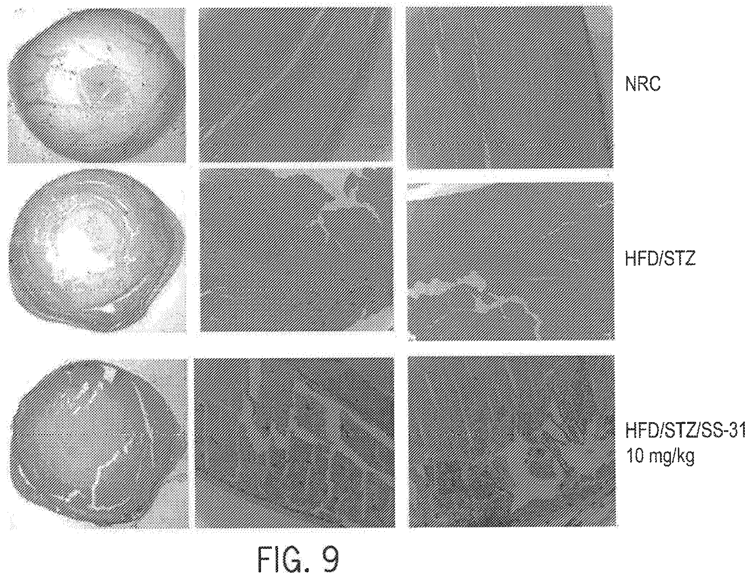

The effect of the aromatic cationic peptides on lens epithelium in the SD rat model was investigated. Administration of D-Arg-2',6'-Dmt-Lys-Phe-NH₂ reduced epithelial cellular changes in both STZ rat model (FIG. 8) and HFD/STZ rat model (FIG. 9).

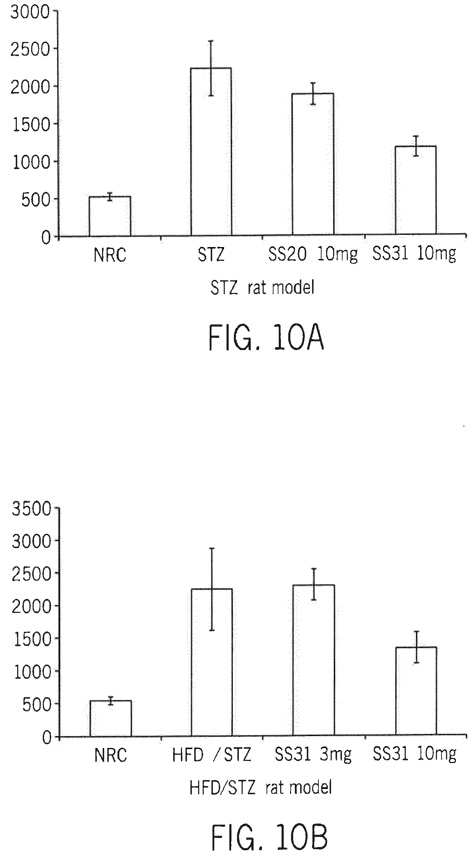

The effect of the aromatic-cationic peptides on the inner blood-retinal barrier function in the SD rat model was investigated. Administration of Phe-D-Arg-Phe-Lys-NH₂ and D-Arg-2',6'-Dmt-Lys-Phe-NH₂ resulted in improved inner blood-retinal barrier function compared to rats on a HFD not administered Phe-D-Arg-Phe-Lys-NH₂ or D-Arg-2',6'-Dmt-Lys-Phe-NH₂ (FIG. 10).

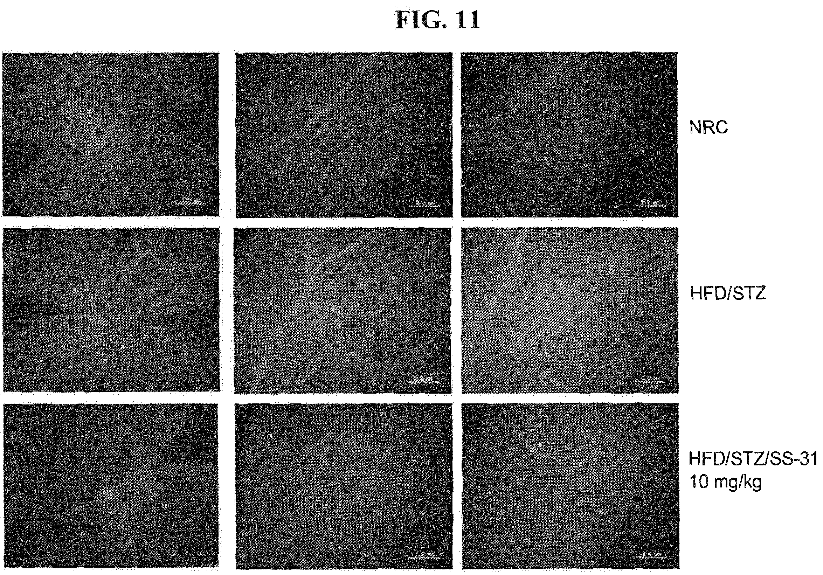

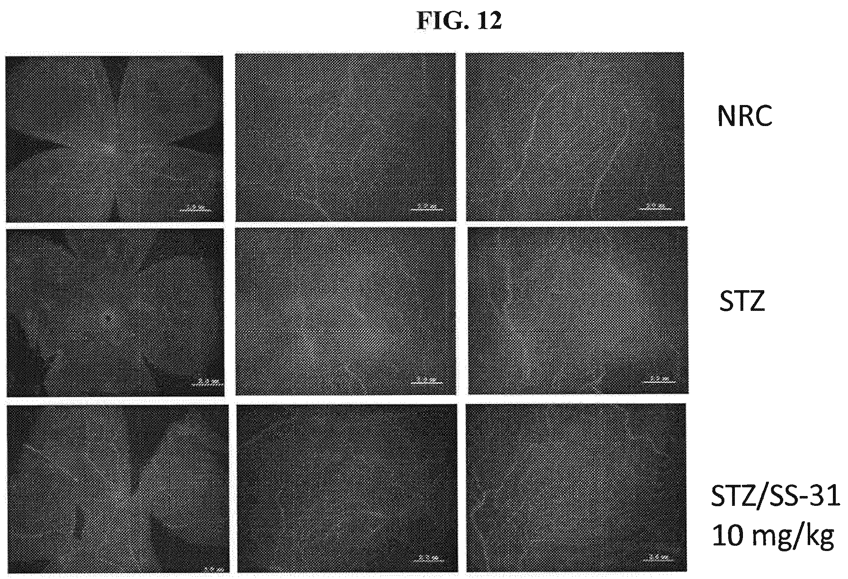

The effect of the aromatic-cationic peptides on retinal microvessels in the SD rat model was investigated (FIGs. 11-12). Administration of D-Arg-2',6'-Dmt-Lys-Phe-NH₂ reduced retinal microvasular changes observed in STZ or HFD/STZ rats.

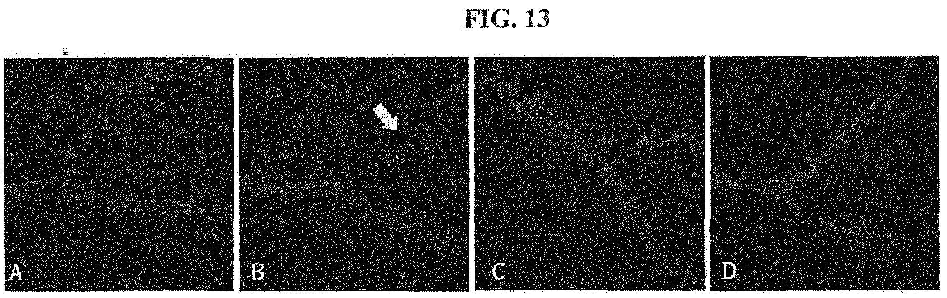

The effect of the aromatic-cationic peptides on the distribution of tight junction protein claudin-5 in retinal microvessels in the SD rat model was investigated. Distribution of tight junction protein claudin-5 was detected under a confocal microscope (FIG. 13). Claudin-5 was distributed along the retinal vessels smoothly, linearly, and uniformly in normal rats (A), but the linear shape was broken in the STZ rat (B, arrow). Distribution of claudin-5 on retinal vessels in STZ rats treated with Phe-D-Arg-Phe-Lys-NH₂ (10 mg/kg) or D-Arg-2',6'-Dmt-Lys-Phe-NH₂ (10 mg/kg) was similar to that of normal rat (Panels C and D, respectively).

In summary, these findings collectively establish that aromatic-cationic peptides, either prevent or compensate for the negative effects of diabetes in the eye, e.g., cataracts and microvasculature. As such, administration of the aromatic-cationic peptides of the present invention is useful in methods of preventing or treating ophthalmic conditions associated with diabetes in human subjects. In particular, the results show that aromatic-cationic peptides of the present technology, such as D-Arg-2',6'-Dmt-Lys-Phe-NH₂, are useful for the prevention or treatment of diabetic retinopathy in mammalian subjects in need thereof.

### Example 4 - D-Ars-2',6'-Dmt-Lys-Phe-NH₂ prevents oxidative stress in glaucomatous trabecular meshwork cells.

The effects of the aromatic-cationic peptides of the invention in preventing or treating glaucoma were investigated by studying the effects of the peptides in glaucomatous trabecular meshwork cells. Glaucoma is the second leading cause of irreversible blindness worldwide. Primary open-angle glaucoma (POAG) is the major subtype of glaucoma. In POAG, there is no visible abnormality of the trabecular meshwork. However, it is believed that the ability of the cells in the trabecular meshwork to carry out their normal function is impaired.

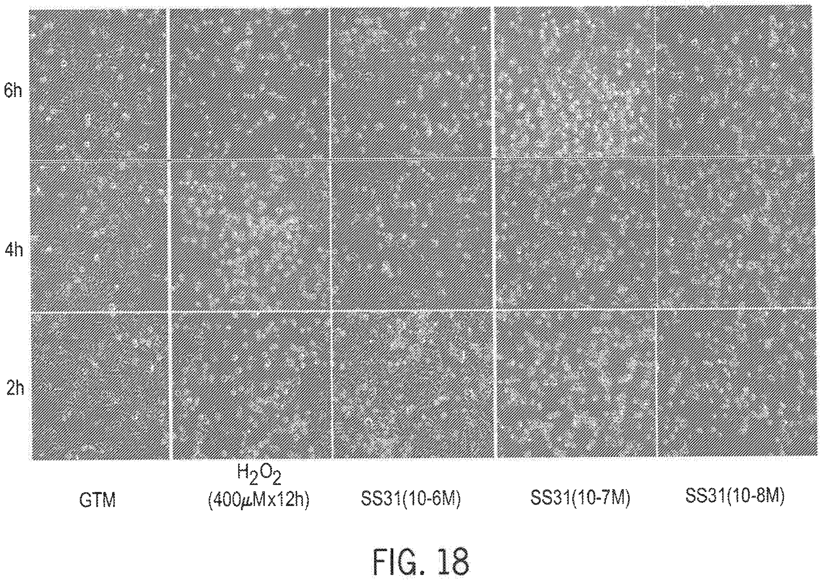

In this Example, the effects of the aromatic-cationic peptides of the invention were compared between trabecular meshwork cells from POAG patients (GTM) and trabecular meshwork cells from non-diseased individuals (HTM). Methods useful in the studies of the present invention have been described. *See generally,* He Y, Ge J, Tombran-Tink J., Mitochondrial defects and dysfunction in calcium regulation in glaucomatous trabecular meshwork cells. Invest Ophthalmol Vis Sci. 2008, 49(11):4912-22; He Y, Leung KW, Zhang YH, Duan S, Zhong XF, Jiang RZ, Peng Z, Tombran-Tink J, Ge J. Mitochondrial complex I defect induces ROS release and degeneration in trabecular meshwork cells of POAG patients: protection by antioxidants. Invest Ophthalmol Vis Sci. 2008, 49(4):1447-58. GTM cells show a significant impairment of mitochondrial membrane potential compared to HTM cells (FIG. 18).

The cells were divided into three groups: "Group A" cells were exposed to hydrogen peroxide prior to administration of D-Arg-2',6'-Dmt-Lys-Phe-NH₂. "Group B" cells were exposed to D-Arg-2',6'-Dmt-Lys-Phe-NH₂ prior to administration of hydrogen peroxide. "Group C" cells were administered D-Arg-2',6'-Dmt-Lys-Phe-NH₂ and hydrogen peroxide simultaneously.

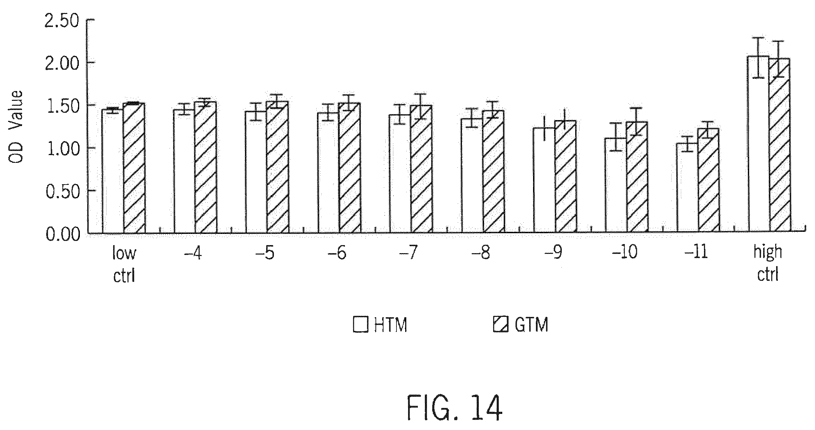

To assess whether D-Arg-2',6'-Dmt-Lys-Phe-NH₂ had cytotoxic effects of HTM or GTM cells, various concentrations of D-Arg-2',6'-Dmt-Lys-Phe-NH₂ were administered to cells and the cytotoxicity was measured using an LDH assay. A LDH cytotoxicity assay is a colorimetric method of assaying cellular cytotoxicity. The assay quantitatively measures the stable, cytosolic, lactate dehydrogenase (LDH) enzyme, which is released from damaged cells. The released LDH is measured with a coupled enzymatic reaction that results in the conversion of a tetrazolium salt (iodonitrotetrazolium (INT)) into a red color formazan by diaphorase. Methods to detect LDH from cells useful in the studies of the present invention are known. *See generally,* Haslam, G. et al. (2005) Anal. Biochem. 336: 187; Tarnawski, A. (2005) Biochem. Biophys. Res. Comm. 333: 207; Round, J. L et al. (2005) J. Exp. Med. 201: 419; Bose, C. et al. (2005) Am. J. Physiol. Gastr. L. 289: G926; Chen, A. and Xu, J. (2005) Am. J. Physiol. Gastr. L. 288: G447. The LDH activity is determined as NADH oxidation or INT reduction over a defined time period. The results are shown in FIG. 14 and indicate that D-Arg-2',6'-Dmt-Lys-Phe-NH₂ does not affect the viability of HTM and GTM cells.

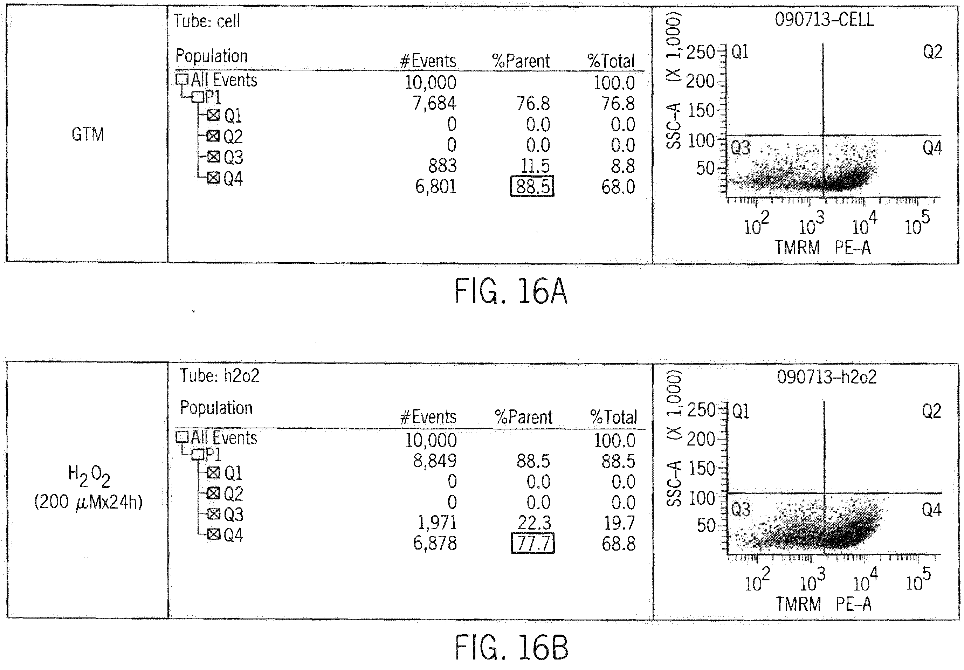

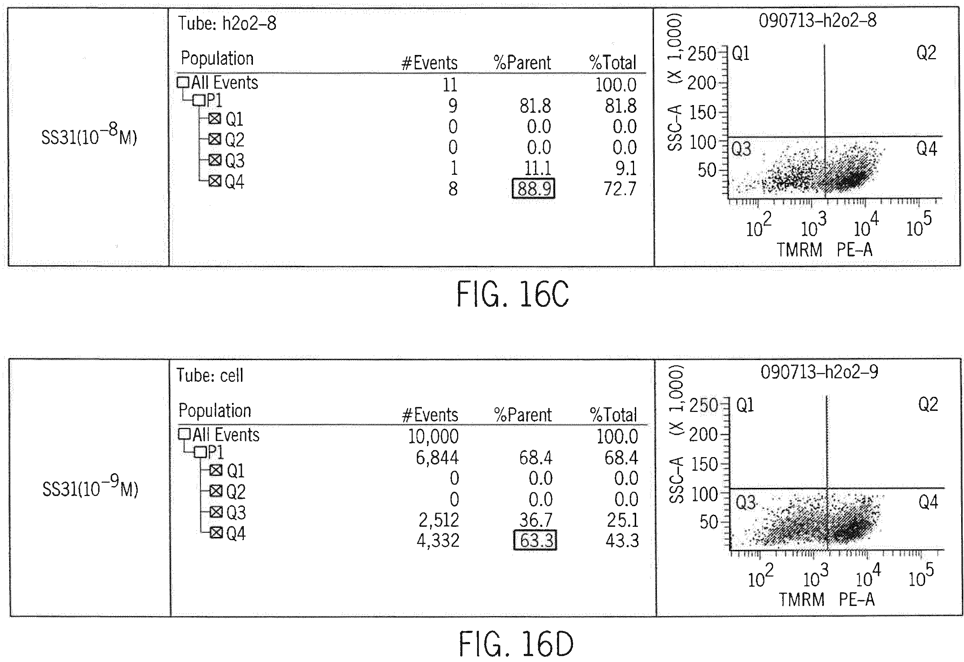

Methods to measure mitochondrial membrane potential using TMRM useful in the studies of the present invention have been described by Andrea Rasola and Massimo Geuna, A flow cytometry assay simultaneously detects independent apoptotic parameters, Cytometry 45:151-157, 2001; Mitoprobe™ JC-1 Kit for Flow Cytometry, Molecular Probes, Invitrogen, USA. FIG. 16 shows the results in GTM cells. Collectively, these results establish that treatment with D-Arg-2',6'-Dmt-Lys-Phe-NH₂ improves the mitochondrial membrane potential of cells that were exposed to hydrogen peroxide prior to administration of D-Arg-2',6'-Dmt-Lys-Phe-NH₂.

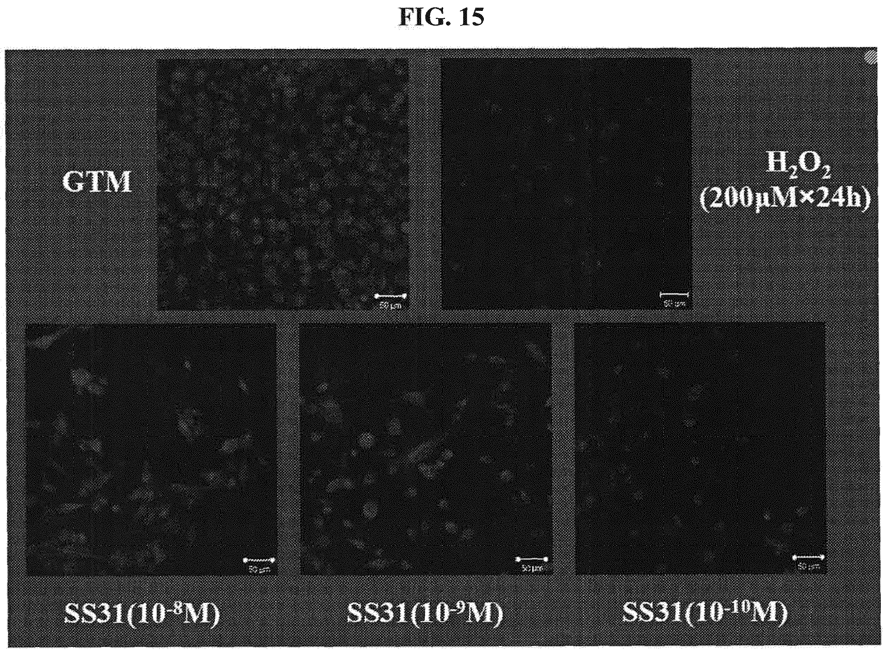

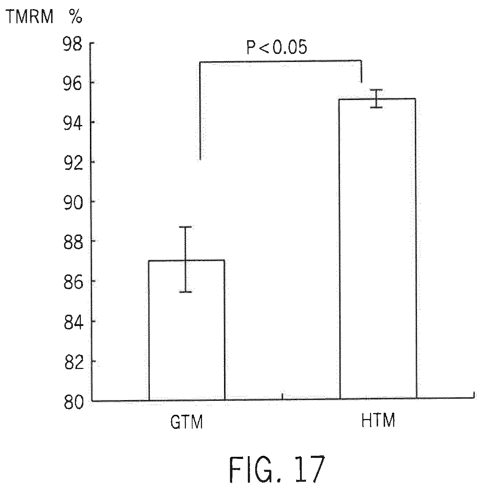

*Group A.* The mitochondrial membrane potential (ΔΨm) of HTM and GTM cells was investigated when those cells were exposed to hydrogen peroxide prior to administration of D-Arg-2',6'-Dmt-Lys-Phe-NH₂. First, the mitochondrial membrane potential was measured using confocal microscopy of cells labeled with tetramethylrhodamine methyl ester (TMRM, 500 nM x 30 min) (FIG. 15). The mitochondrial membrane potential was also measured using flow cytometry (FIGs. 16-17) by labeling cells with the mitochondrion-selective probe tetramethylrhodamine methyl ester (TMRM, 500 nM x 30 min).

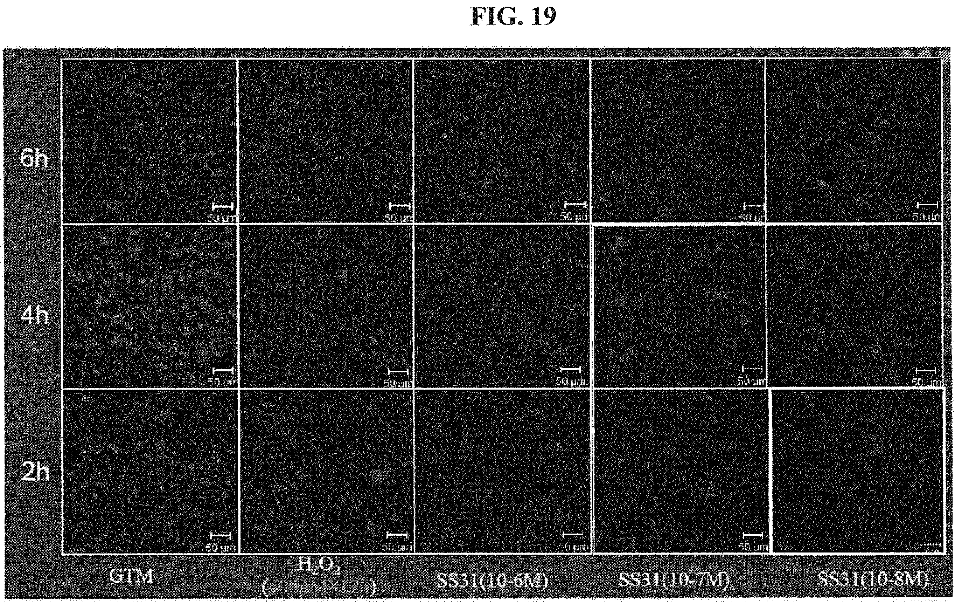

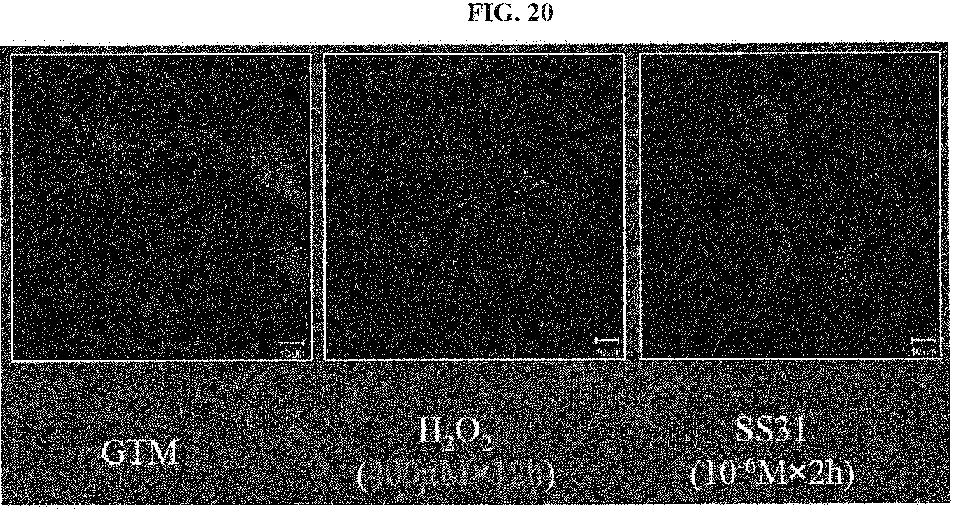

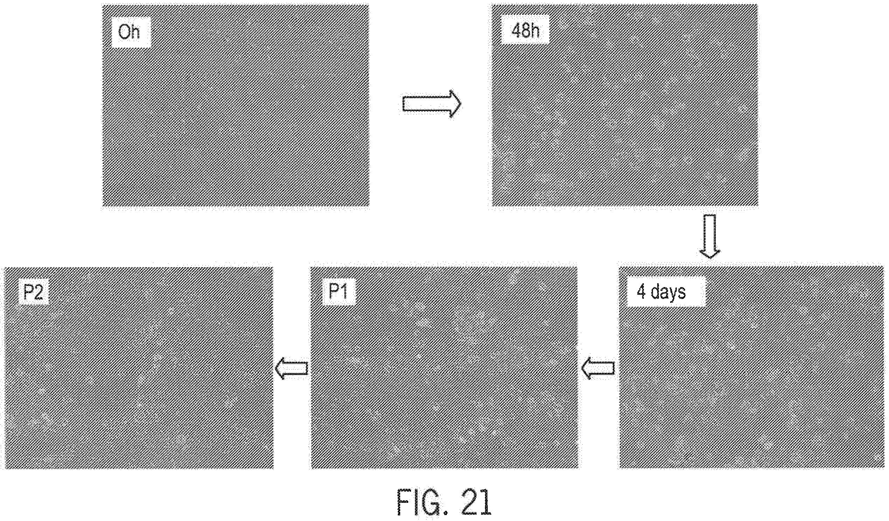

*Group B.* The morphology of GTM cells was investigated when those cells were exposed to D-Arg-2',6'-Dmt-Lys-Phe-NH₂ prior to administration of hydrogen peroxide. FIG. 18 shows the results of inverted phase contrast microscopy of cells administered various concentrations of D-Arg-2',6'-Dmt-Lys-Phe-NH₂. The results indicate that D-Arg-2',6'-Dmt-Lys-Phe-NH₂ protects cells from hydrogen peroxide-mediated morphlogical changes in a concentration-dependent and time-dependent manner. That is, hydrogen peroxide mediated cell loss and rounding was diminished in cells exposed to D-Arg-2',6'-Dmt-Lys-Phe-NH₂ peptide. The mitochondrial membrane potential (ΔΨm) of HTM and GTM cells was also investigated when those cells were exposed to D-Arg-2',6'-Dmt-Lys-Phe-NH₂ prior to administration of hydrogen peroxide. The mitochondrial membrane potential was measured using confocal microscopy of cells labeled with tetramethylrhodamine methyl ester (TMRM, 500 nM x 30 min) (FIG. 19-21). These results show that pre-treatment with D-Arg-2',6'-Dmt-Lys-Phe-NH₂ dose-dependently improves the mitochondrial membrane potential of cells that were exposed to hydrogen peroxide. As such, D-Arg-2',6'-Dmt-Lys-Phe-NH₂ provides a protective effect against oxidative stress in GTM cells.

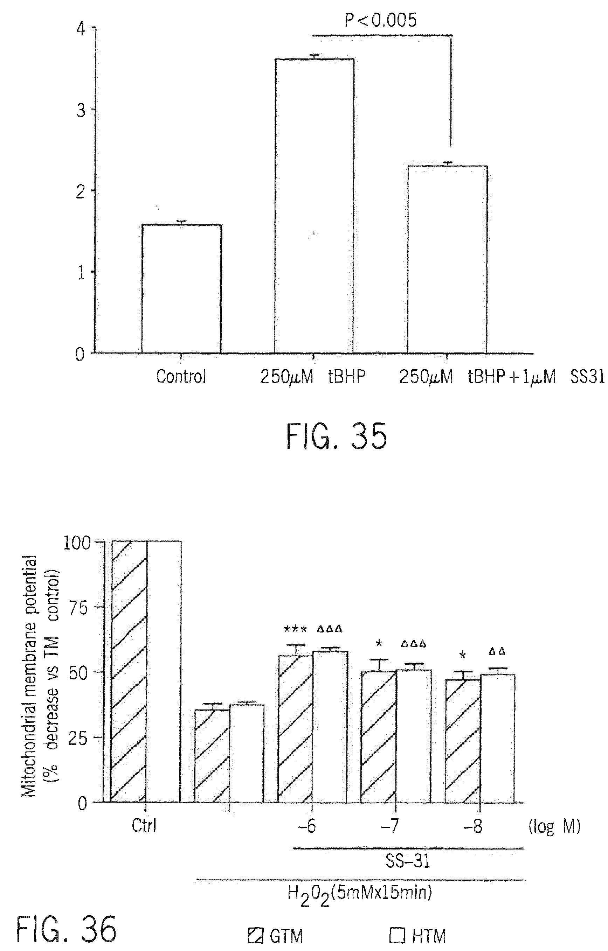

The effects of D-Arg-2',6'-Dmt-Lys-Phe-NH₂ at mitigating acute oxidative injury in GTM and HTM cells was investigated. FIG. 36 shows the fluorescence intensity of TMRM of GTM and HTM cells using FACS analysis. The percentage of fluorescence intensity compared to GTM control in H₂O₂, D-Arg-2',6'-Dmt-Lys-Phe-NH₂ 10⁻⁶M, D-Arg-2',6'-Dmt-Lys-Phe-NH₂ 10⁻⁷M, D-Arg-2',6'-Dmt-Lys-Phe-NH₂ 10⁻⁸M were 35.2±2.12%, 56.2±4.04%, 50.3±4.46%, 47.5±2.82% respectively, n=4; the HTM groups were 37.4±0.725%, 57.7±1.80%, 50.6±3.06%, 49.4±2.27% respectively, n=4. ** means P<0.01 compared to GTM H₂O₂ group; * means P<0.05 compared to GTM H₂O₂ group; ▲▲▲ means P<0.001 compared to HTM H₂O₂ group.

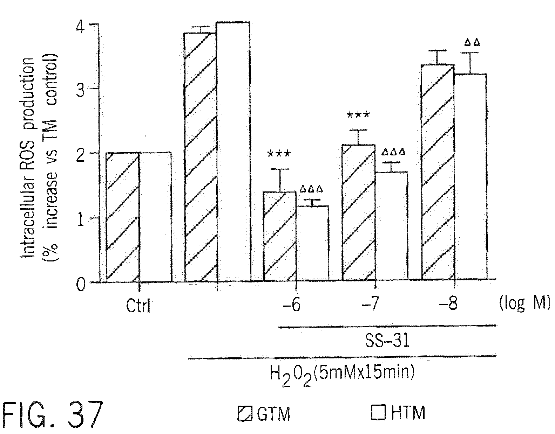

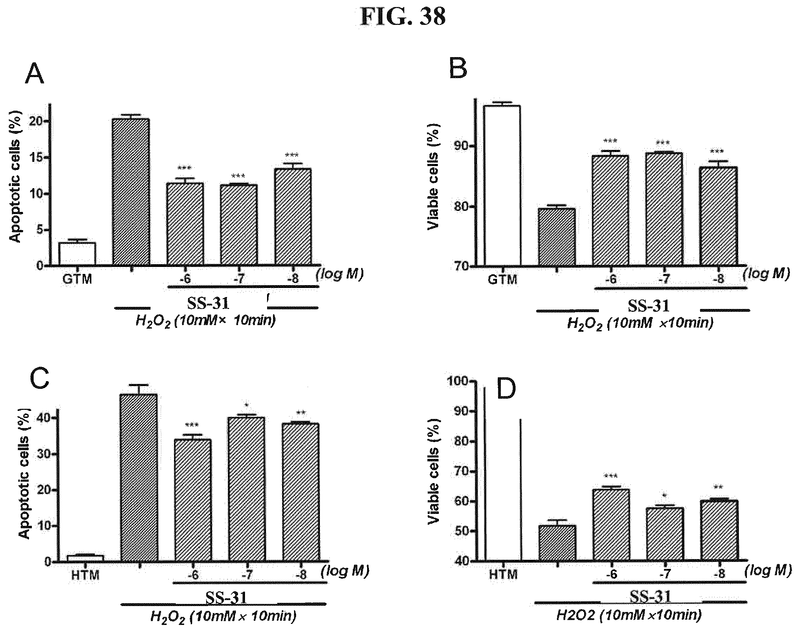

FIG. 37 shows the fluorescence intensity of ROS of GTM and HTM cells in control and D-Arg-2',6'-Dmt-Lys-Phe-NH₂-treated groups using FACS analysis. The percentage of intracellular ROS production compared to GTM control in GTM H₂O₂, D-Arg-2',6'-Dmt-Lys-Phe-NH₂ 10⁻⁶M, D-Arg-2',6'-Dmt-Lys-Phe-NH₂ 10⁻⁷M, D-Arg-2',6'-Dmt-Lys-Phe-NH₂ 10⁻⁸M groups were 146.0 ± 2.27%, 84.5±8.75%, 102.0±5.69%, 133.0±5.17% respectively (n=3); the HTM groups were 153.0±3.46%, 79±2.39%, 91.8±3.49%, 129.0±8.24% respectively (n=4). P<0.001 GTM and HTM H₂O₂ Group compared to control; *** means P<0.001 compared to GTM H₂O₂ group; ▲▲▲ Means P<0.001 compared to HTM H₂O₂ group; ▲▲ means P<0.01 compared to HTM H₂O₂ group. FIG. 38 shows that D-Arg-2',6'-Dmt-Lys-Phe-NH₂ reduced the amount of cell apoptosis induced by H₂O₂.

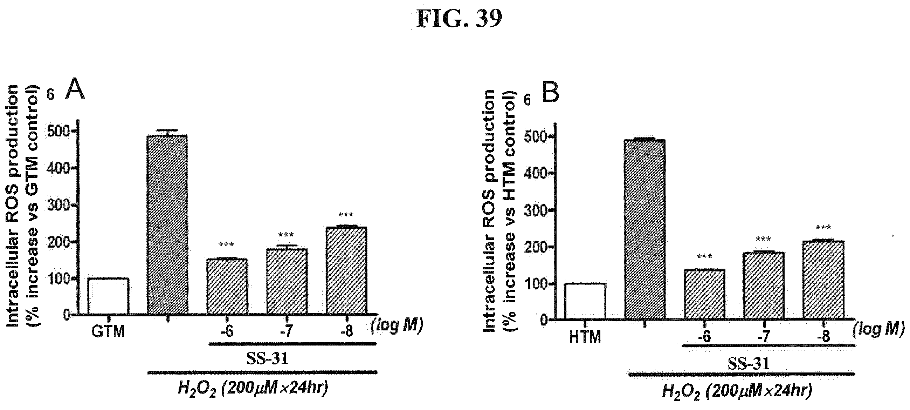

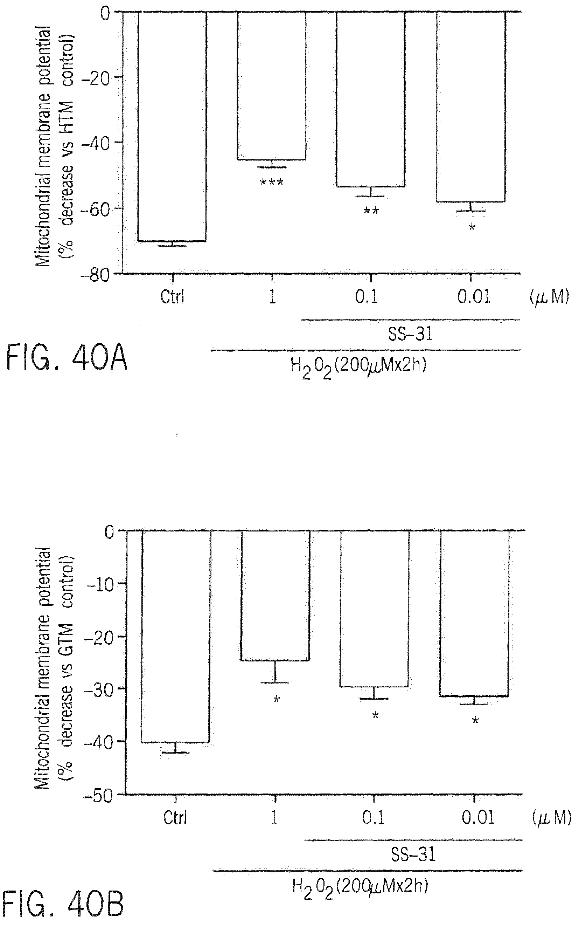

The effects of D-Arg-2',6'-Dmt-Lys-Phe-NH₂ on sustained oxidative injury of GTM and HTM cells was examined. Cells were pre-treated with 10⁻⁶, 10⁻⁷, 10⁻⁸ M of D-Arg-2',6'-Dmt-Lys-Phe-NH₂ for 1 h, and then incubated with 200 µM H₂O₂ for 24 h to investigate the protective effect of D-Arg-2',6'-Dmt-Lys-Phe-NH₂ in sustained oxidative stress. FIG. 39 and Table 15 shows the effects of D-Arg-2',6'-Dmt-Lys-Phe-NH₂ on ROS production from sustained oxidative injury of GTM and HTM cells. FIG. 40 and Table 16 shows the MMP change in GTM and HTM cells in each treatment group.

| **TABLE 15. ROS Production in GTM3 and iHTM cells treated with H₂O₂.** | | | | |
|---|---|---|---|---|
| | H₂O₂-Ctrl (%) | D-Arg-2',6'-Dmt-Lys-Phe-NH₂ | | |
| | | 1µM (%) | 0.1µM (%) | 0.01µM (%) |
| GTM 3 | 376.80±17.47 | 47.40±1.81** * | 68.91±8.62** * | 133.70±3.24** * |
| iHTM | 388.50±5.54 | 36.91±1.47** * | 82.89±3.70** * | 114.30±3.89** * |

| **TABLE 16. MMP Decline in GTM3 and iHTM cells treated with H₂O₂.** | | | | |
|---|---|---|---|---|
| | H2O2-Ctrl (%) | D-Arg-2',6'-Dmt-Lys-Phe-NH₂ | | |
| | | 1µM (%) | 0.1µM(%) | 0.01µM (%) |
| GTM3 | -39.67±2.33 | -24.33±4.18* | -29.33±2.19* | -31.33±1.20* |
| iHTM | -69.53±2.01 | -44.99±2.19*** | -53.24±2.52** | -58.24±2.62* |

Collectively, these results demonstrate that D-Arg-2',6'-Dmt-Lys-Phe-NH₂ has no cytotoxicity at 10⁻⁴ M for both GTM and HTM cells and that sustained and acute oxidative stress induced by hydrogen peroxide can be prevented by D-Arg-2',6'-Dmt-Lys-Phe-NH₂ (>10⁻⁹ M). These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of glaucoma in mammalian subjects in need thereof.

### Example 5 - D-Arg-2',6'-Dmt-Lys-Phe-NH₂ prevents oxidative stress in primary retinal pigment epithelial cells.

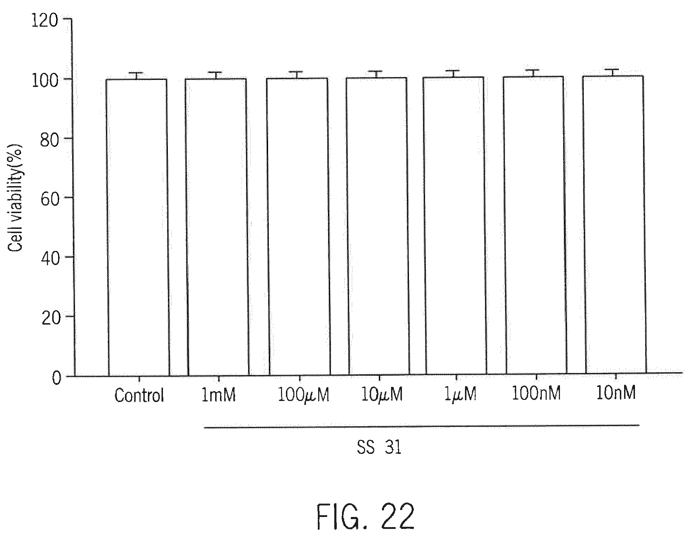

Primary retinal pigment epithelial (RPE) cells were cultured to test the effects of the aromatic-cationic peptides of the invention in preventing or reducing oxidative damage in these cells. Methods useful for the study of primary retinal pigment epithelial cells have been described. *See,* Dunn et al., ARPE-19, A Human Retinal Pigment Epithelial Cell Line with Differentiated Properties, Experimental Eye Research, 1996,62(2): 155-170. First, it was shown that D-Arg-2',6'-Dmt-Lys-Phe-NH₂ did not adversely effect these cells. Primary cultured human RPE cells were incubated with different concentrations of D-Arg-2',6'-Dmt-Lys-Phe-NH₂ alone for a period of 24 h, and cell viability was determined by a MTT assay (FIG. 22).

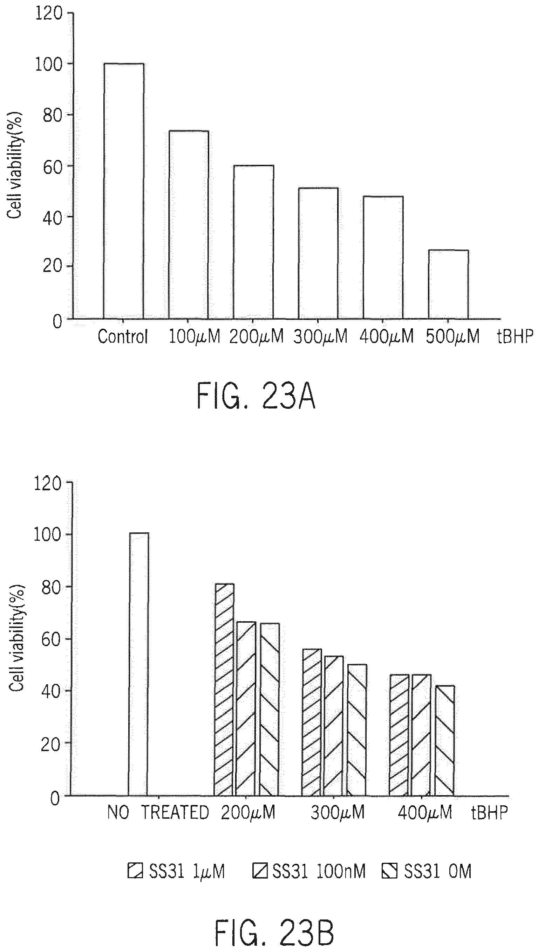

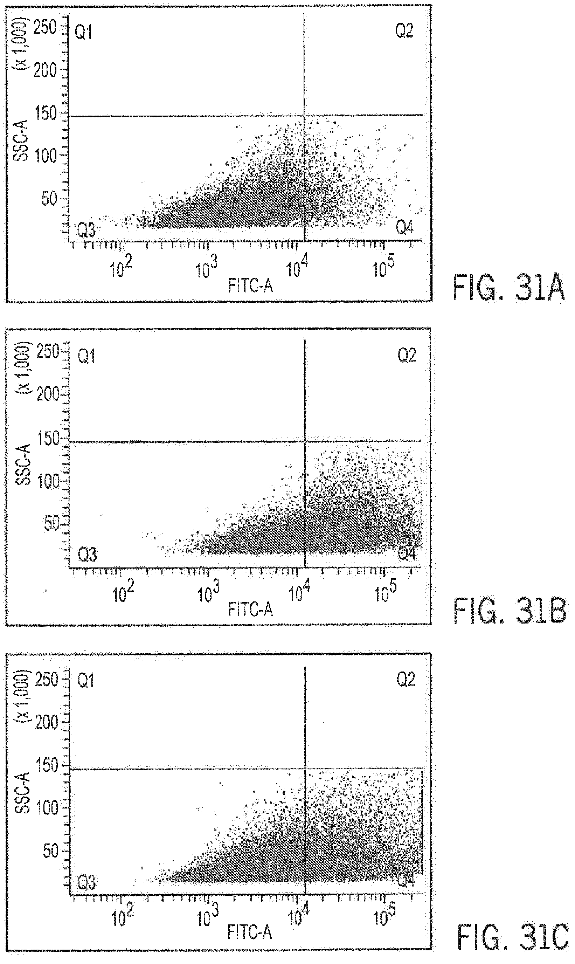

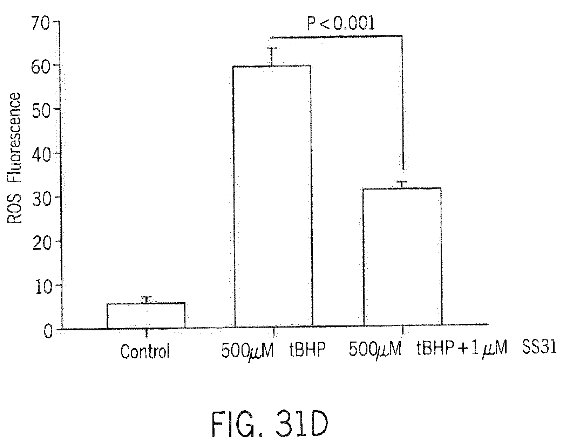

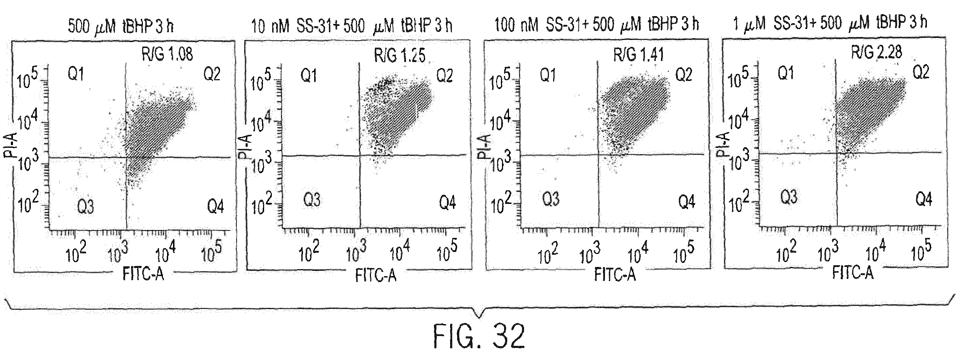

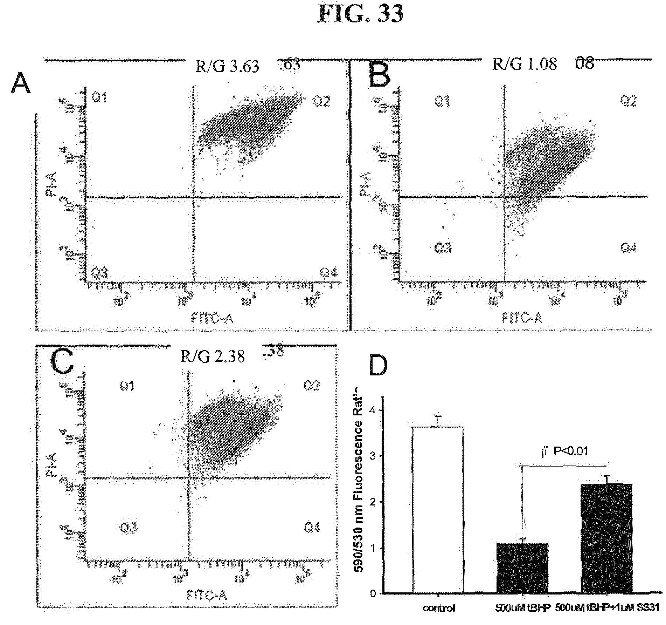

Next, the viability of primary RPE cells was tested in the presence of tBHP and various concentrations of D-Arg-2',6'-Dmt-Lys-Phe-NH₂. Cells were plated at 10,000 cells per well in a 96-well plate and cultured for 24 h, then starved for 24 h. After that, cells were exposed to increasing concentrations of tBHP (FIG. 23A), or preincubated for 4 h with different concentrations of D-Arg-2',6'-Dmt-Lys-Phe-NH₂, then stimulated with tBHP for 6 h (FIG. 23B). These results indicate that D-Arg-2',6'-Dmt-Lys-Phe-NH₂ enhanced cell viability in response to tBHP administration. Intracellular ROS production in three groups of RPE cells was also examined using FACS analysis. FIG. 31A shows ROS production in control RPE cells; FIG. 31B shows ROS production in RPE cells treated with 500 µM tBHP for 3 h; and FIG. 31C shows ROS production in RPE cells treated with 500 µM tBHP for 3 h and 1 µM D-Arg-2',6'-Dmt-Lys-Phe-NH₂. FIG. 32 shows MMP labeled by JC-1 in a FACS analysis. Three different concentration of D-Arg-2',6'-Dmt-Lys-Phe-NH₂ groups were analyzed. The ratio of red to green in 500 µM tBHP for the 3 h group is 1.08, the ratio of red to green in 10 nM D-Arg-2',6'-Dmt-Lys-Phe-NH₂ for 4 h + 500 µM tBHP for 3 h group is 1.25; the ratio of red to green in 100 nM D-Arg-2',6'-Dmt-Lys-Phe-NH₂ for 4hr + 500 µM tBHP for 3 h group is 1.4; and the ratio of red to green in 1 µM D-Arg-2',6'-Dmt-Lys-Phe-NH₂ for 4 h + 500 µM tBHP for 3 h group is 2.28. FIG. 33 shows the effect of 1 µM D-Arg-2',6'-Dmt-Lys-Phe-NH₂ on MMP decline induced by tBHP. FIG. 33A: Control group, R/G is 3.63±0.24; FIG. 33B: 500 µM tBHP for 3 h group, R/G is 1.08±0.11; FIG. 33C: 1 µM D-Arg-2',6'-Dmt-Lys-Phe-NH₂ for 4 h + 500 µM tBHP for 3 h group, R/G is 2.38±0.18. FIG. 33D is a chart comparing the fluorescence ratio for the different groups. *P<0.01, C vs. B.

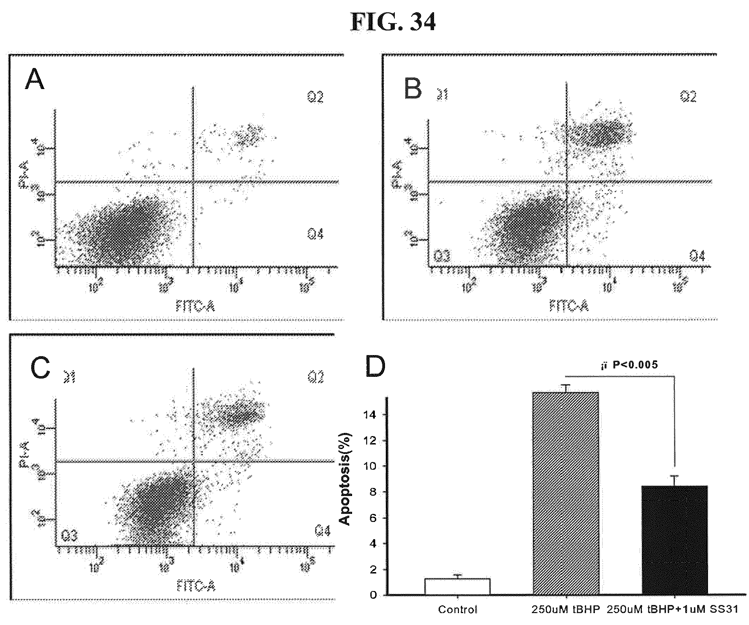

FIG. 34 shows the effect of D-Arg-2',6'-Dmt-Lys-Phe-NH₂ on cell apoptosis induced by 250 µM tBHP for 24 h. FIG. 34A: control group;(Q2+Q4)%=1.27±0.3%; FIG. 34B: 250 µM tBHP for 24 h group; (Q2+Q4)%=15.7±0.6%; FIG. 34C: 1 µM D-Arg-2',6'-Dmt-Lys-Phe-NH₂ for 4 h + 250 µM tBHP for 24 h group; (Q2+Q4)%=8.4±0.8%. FIG. 34D is a chart comparing the fluorescence ratio for the different groups. *P<0.05 C vs. B. FIG. 35 is a chart showing the MDA level induced by tBHP in 3 groups of RPE cells. (*P<0.05).

Collectively, these results demonstrate that D-Arg-2',6'-Dmt-Lys-Phe-NH₂ prevents oxidative stress in primary retinal pigment epithelial cells. These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of damage to retinal cells in mammalian subjects in need thereof.

### Example 6 - Prevention and Treatment of Choroidal Neovascularization by Aromatic-Cationic Peptides of the Invention in a CNV Mouse Model

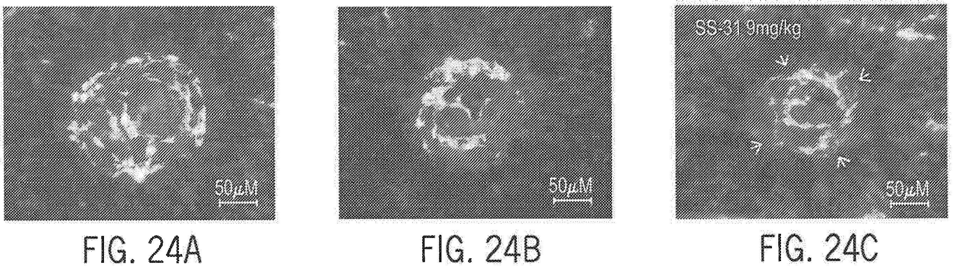

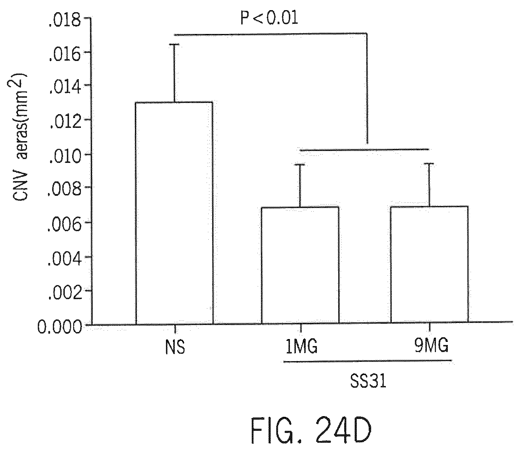

To further demonstrate the prevention of choroidal neovascularization (CNV) on the one hand, and treatment of CNV on the other hand, the aromatic-cationic peptides of the invention were tested on a mouse model of CNV (FIG. 24). CNV were induced in the eye with laser bums. Methods useful in the present studies have been described by Reich, Mol Vis 2003; 9:210-216.

Briefly, five to six-week-old C57BL/6 male mice were anesthetized with chloral hydrate and the pupils were dilated with tropicamide. With a coverslip used as a contact lens, four laser spots (532 nm, 260 mw, 0.01s, 50 µm; Novus Spectra, Lumenis, USA) were applied to the fundus in a circle around the optic disc in the right eye. Daily intraperitoneal injections of 1 mg/kg, 9 mg/kg D-Arg-2',6'-Dmt-Lys-Phe-NH₂ (D-Arg-2',6'-Dmt-Lys-Phe-NH₂) or vehicle were started the day prior to laser photocoagulation.

After one week, mice were deeply anaesthetized and perfused through the left ventricle with 1 ml (50 mg/ml) of PBS-buffered fluorescein-dextran. Eyes were enucleated and fixed in 4% paraformaldehyde for 2 h. The eyes were sectioned at the equator, and the anterior half and retina were removed. The posterior eye segment containing the sclera and choroid was dissected into quarters by four to five radial cuts and mounted on a slide. All flatmounts were examined by a fluorescence microscope (AxioCam MRC;Carl Zeiss). Image-Pro Plus software (Media Cybernetics, Silver Spring, MD) was used to measure the area of each CNV lesion.

There were 48 locations of neovascularization in each group. The area of neovascularization was calculated using IMAGE-PROPLUS6.0 software. The area of neovascularization in the CNV model, 1 mg/kg D-Arg-2',6'-Dmt-Lys-Phe-NH₂ and 9 mg/kg D-Arg-2',6'-Dmt-Lys-Phe-NH₂ groups were 0.0130±0.0034, 0.0068±0.0025, 0.0067±0, respectively. These results indicate that the two concentrations of D-Arg-2',6'-Dmt-Lys-Phe-NH₂ significantly reduced the area of choroidal neovasculatization (P<0.05) (FIG. 24).

These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of choroidal neovasculatization in mammalian subjects in need thereof.

### Example 7 - Prevention and Treatment of Oxygen-Induced Retinopathy (OIR) by Aromatic-Cationic Peptides of the Invention in an OIR Mouse Model

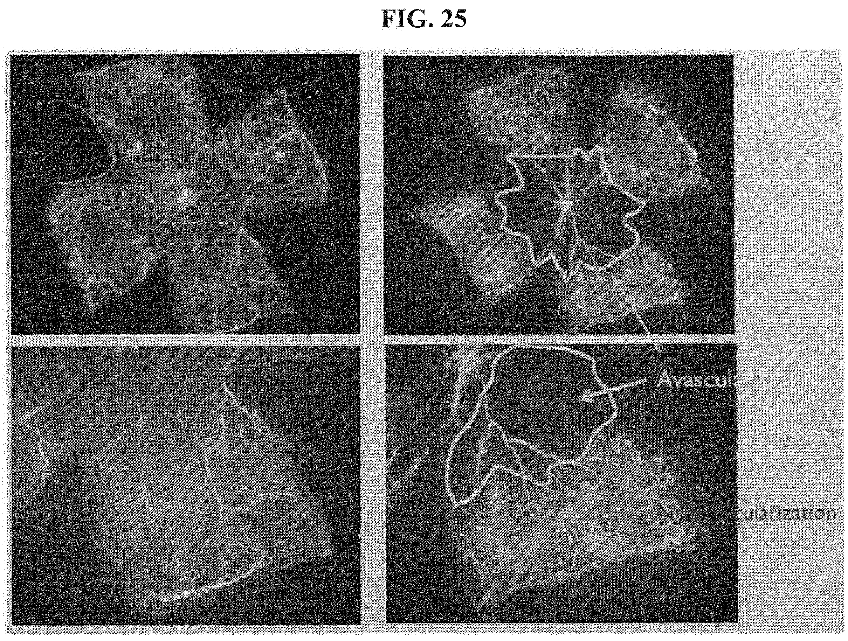

To further demonstrate the prevention of oxygen-induced retinopathy (OIR), the aromatic-cationic peptides of the invention were tested on a mouse model of OIR (FIG. 25). In this model, 7-day-old mouse pups with partially developed retinal vasculature were subjected to hyperoxia (75% oxygen) for 5 days, which stops retinal vessel growth and causes significant vaso-obliteration. On postnatal day 12, the pups were returned to room air, and by postnatal day 17, a florid compensatory retinal neovascularization occurred. This model of pathological neovascularization has been widely used as a substitute for proliferative diabetic retinopathy (DR).

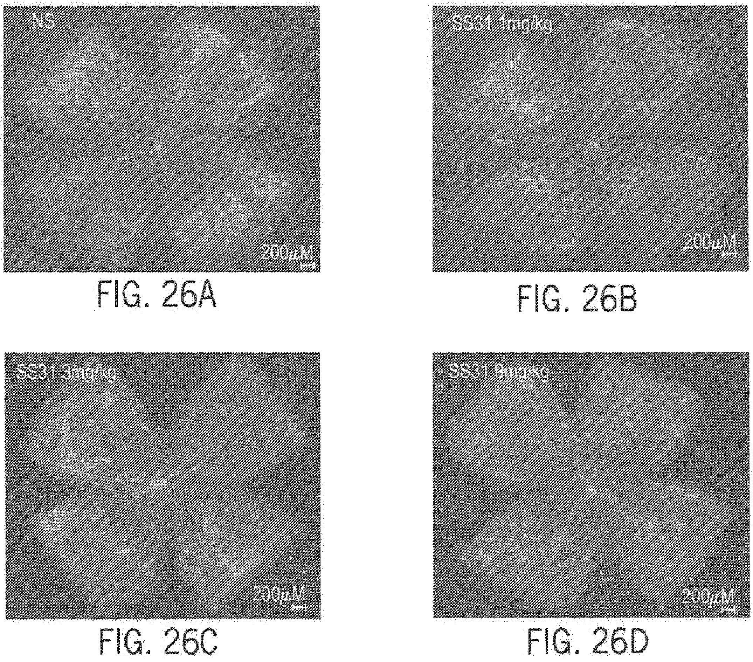

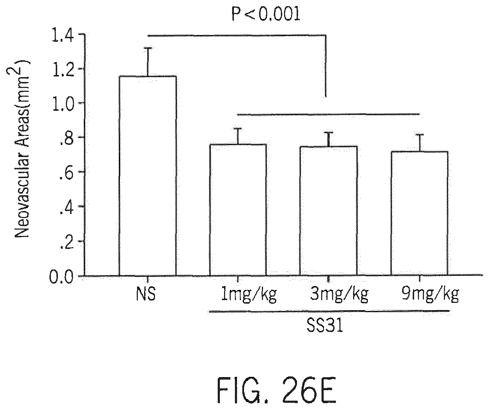

To examine the effects of the aromatic-cationic peptides of the invention on prevention of OIR, OIR was induced in mouse pups and the mice were simultaneously administered an aromatic-cationic peptide (e.g., Phe-D-Arg-Phe-Lys-NH₂ or D-Arg-2',6'-Dmt-Lys-Phe-NH₂) for approximately 6 weeks. The results are shown in FIG. 26 and indicate that treatment with D-Arg-2',6'-Dmt-Lys-Phe-NH₂ prevented the compensatory retinal neovascularization. As such, the aromatic-cationic peptides of the invention are useful in methods of preventing proliferative diabetic retinopathy in mammalian subjects.

These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of oxygen-induced retinopathy (OIR) in mammalian subjects in need thereof.

### Example 8 - Antioxidants reduce photoreceptor cell death in a model of retinitis pigmentosa.

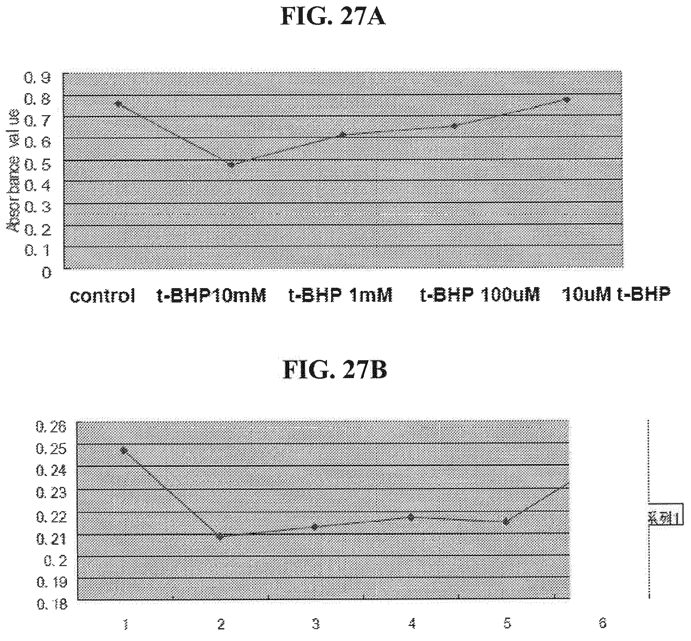

A cone cell specific line 661W was derived from a mouse retinal tumor. Methods useful in the present studies of 661 W cells have been described previously. *See generally,* Gearóid Tuohy, Sophia Millington-Ward, Paul F. Kenna, Peter Humphries and G. Jane Farrar, Sensitivity of Photoreceptor-Derived Cell Line (661W) to Baculoviral p35, Z-VAD.FMK, and Fas-Associated Death Domain, Investigative Ophthalmology and Visual Science. 2002;43:3583-3589. These cells were cultured to test the effects of the aromatic-cationic peptides of the invention in preventing or reducing oxidative damage in the cone cells (FIG. 27). First, it was shown that tBHP affected survival of 661W cells (FIG. 27A). Different doses of tBHP were administered to the cells for 3 h. Next, it was shown that different doses of D-Arg-2',6'-Dmt-Lys-Phe-NH₂ reduced tBHP-induced 661 W cell death (FIG. 27B).

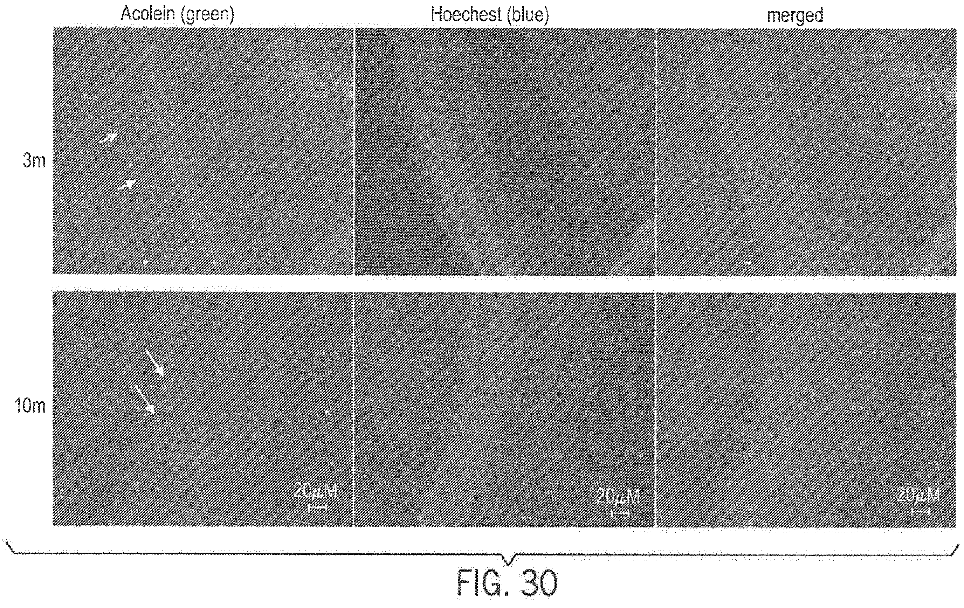

The potential of D-Arg-2',6'-Dmt-Lys-Phe-NH₂ (D-Arg-2',6'-Dmt-Lys-Phe-NH₂) to protect against loss of mitochondrial viability induced by tBHP, 100 nmol/L D-Arg-2',6'-Dmt-Lys-Phe-NH₂ was administered to the cultures of 661w cells. The results are shown in FIG. 30 and indicate that D-Arg-2',6'-Dmt-Lys-Phe-NH₂ significantly enhanced mitochondrial viability compared to cells not administered D-Arg-2',6'-Dmt-Lys-Phe-NH₂, as shown by a JC-1 assay.

These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of retinitis pigmentosa in mammalian subjects in need thereof.

### Example 9 - Effects of D-Arg-2',6'-Dmt-Lys-Phe-NH₂ in a mouse model of retina degeneration.

To further demonstrate the prevention of retinal degeneration, the aromatic-cationic peptides of the invention were tested on a mouse model of retina degeneration. CNV is induced in the eye with laser bums. (see Example 6). Mouse models of retinal degeneration have been investigated for many years in the hope of understanding the causes of photoreceptor cell death. Naturally occurring mouse mutants that manifest degeneration of photoreceptors in the retina with preservation of all other retinal cell types have been found: retinal degeneration (formerly *rd,* identical with rodless retina, *r,* now *Pde6b rd1*); Purkinje cell degeneration (*pcd*); nervous (*nr*); retinal degeneration slow (*rds,* now *Prph Rd2*); retinal degeneration 3 (*rd3*); motor neuron degeneration (*mnd*); retinal degeneration 4 (*Rd4*); retinal degeneration 5 (*rd5*); vitiligo (*vit*, now *Mitf mi-vit*); retinal degeneration 6 (*rd6*); retinal degeneration 7 (*rd7*); neuronal ceroid lipofuscinosis (*nclf*); retinal degeneration 8 (*rd8*); retinal degeneration 9 (*Rd9*); retinal degeneration 10 (*rd10*); and cone photoreceptor function loss (*cpfl1*).

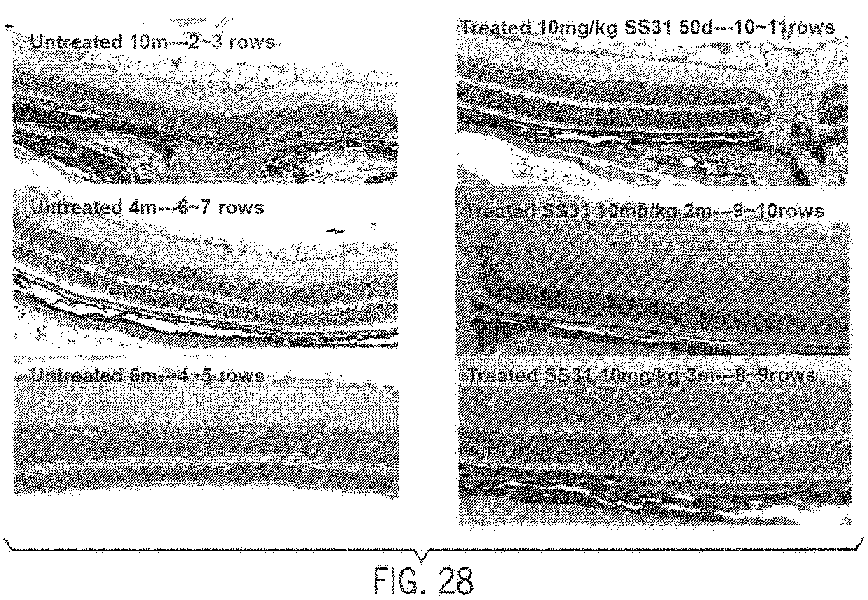

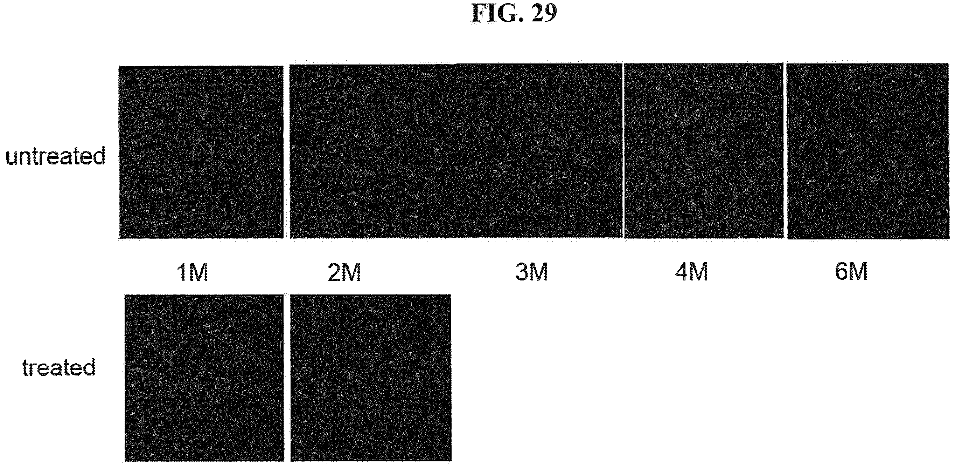

FIG. 28 is a series of micrographs showing the thickness of the retinal outer nuclear layer (ONL) in a mouse model of retina degeneration in control and D-Arg-2',6'-Dmt-Lys-Phe-NH₂-treated mice. The results indicate that D-Arg-2',6'-Dmt-Lys-Phe-NH₂ treated mice retained a greater number of rows of cells in the ONL compared to untreated mice. Retinal flat mounts stained with peanut agglutinin (PNA), which selectively stain core inner and outer segments also show that cone cell density is greater in D-Arg-2',6'-Dmt-Lys-Phe-NH₂ treated mice (FIG. 29). These results indicate that treatment with D-Arg-2',6'-Dmt-Lys-Phe-NH₂ prevented the compensatory damage to the retinal outer nuclear layer in a mouse model of retinal degeneration.

These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of retinal degeneration in mammalian subjects in need thereof.

### Example 10 -Ophthalmic delivery system: isotonic solution for unit dose ophthalmic application using an aromatic-cationic peptide of the present technology

An ophthalmic delivery system suitable for ocular delivery of aromatic-cationic peptide of the present technology in an isotonic unit dose application can be formulated. An illustrative embodiment of such a delivery system using D-Arg-2',6'-Dmt-Lys-Phe-NH₂ is described here. Either 100mg (1mg/ml) or 1g (10/mg/ml) peptide equivalent of D-Arg-2',6'-Dmt-Lys-Phe-NH₂, or a pharmaceutically acceptable salt thereof (e.g., acetate salt or tri-fluoro-acetate salt), is dissolved in approximately 50mls of water for injection NF (National Formulatory) to which is added a suitable amount of Trehalose, Mannitol, Sucrose or other sugar as a bulking agent and tonicity modifier as indicated below. To the resulting solution, disperse and dissolve the sodium chloride and L-glutathione as indicated below. Make up to 100ml with water for Injection NF and sterilize the entire solution by aseptic filtration into unit dose polyethylene containers prior to use. The term "NA" means "not applicable."

| **TABLE 17. Isotonic solution for unit dose Ophthalmic application** | | | | |
|---|---|---|---|---|
| Component | Concentration | Weight per 100ml | Concentration | Weight per 100ml |
| D-Arg-2',6'-Dmt-Lys-Phe-NH₂ or pharmaceutically acceptable salt thereof such as acetate salt or tri-fluoro-acetate salt | 1mg/ml | 100.00mg | 10mg/ml | 1g |
| Trehalose, Mannitol or Sucrose(anhydro us basis) | 4-5%(40-50mg/ml) | 4-5g | 1-2% (10-20mg/ml) | 1-2g |
| Sodium Chloride NF | 2.4-5.81mg/ml | 0.24-0.581g | 2.4-5.81mg/ml(100m M) | 0.24-0.581g |
| L-Glutathione *JP/EP | 0.1mg/ml | 0.01g | 0.1mg/ml | 0.01g |
| Water for Injection NF | NA | To 100ml | NA | To 100 ml |
| pH adj with Acetic acid/ 0.1M NaOH | Qs to pH 5.5-7.5 | Qs to pH 5.5-7.5 | Qs to pH 5.5-7.5 | Qs to pH 5.5-7.5 |
| * JP refers to the Japanese Pharmacopoeia; EP refers to the European Pharmacopoeia. | | | | |

Additionally, or alternatively, an ophthalmic delivery system suitable for ocular delivery of aromatic-cationic peptide of the present technology can be formulated to include the peptide and one or more pharmaceutically acceptable excipients. For example, a delivery system including either 100mg (1mg/ml) or 1g (10/mg/ml) peptide equivalent of D-Arg-2',6'-Dmt-Lys-Phe-NH₂, or a pharmaceutically acceptable salt thereof (e.g., acetate salt or tri-fluoro-acetate salt), is dissolved in approximately 50mls of water for injection. One or more pharmaceutical excipients, selected based on the intended use of the formulation, can then be added to provide the ophthalmic delivery system.

This example demonstrates aromatic-cationic peptide formulations useful for the ocular delivery of aromatic-cationic peptides for the prevention or treatment of ophthalmic conditions in mammalian subjects in need thereof.

### Example 11 - Ophthalmic delivery system: isotonic solution for multiple dose ophthalmic application using an aromatic-cationic peptide of the present technology

As an alternative to unit dose use, a multiple dose formulation can be made by adding the antimicrobial preservatives propylparaben (0.05-0.02%, w/v) and methylparaben (0.10-0.25%, w/v) to the same solution in Example 10. Propylene glycol (2-5%, v/v) may also be added to improve anti-microbial properties. Alternatively, 0.01-0.02% (w/v) benzalchonium chloride maybe used in combination with 0.1 % (w/v) disodium EDTA. These solutions may be aseptically filled into multiple dose, Type I Glass or polyethylene dropper bottles.

This example demonstrates aromatic-cationic peptide formulations useful for the ocular delivery of aromatic-cationic peptides for the prevention or treatment of ophthalmic conditions in mammalian subjects in need thereof.

### Example 12 - Ophthalmic delivery system: isotonic solution for multiple or single dose use ophthalmic application stabilized against heavy metals using an aromatic-cationic peptide of the present technology

As D-Arg-2',6'-Dmt-Lys-Phe-NH₂ or a pharmaceutically acceptable salt thereof (e.g., actetate salt or tri-fluoro-acetate salt) is susceptible to oxidation, in addition to the anti-oxidant L-glutathione, 0.05-0.1 % (w/v) sodium EDTA may be added to either Example 10 or 11 in order to improve protection against oxidative instability mediated by heavy metals such as iron, copper and other ions. Additionally, other antioxidants can be used such as propyl gallate, sodium metabisulfite, and other thiol-mercaptans.

This example demonstrates aromatic-cationic peptide formulations useful for the ocular delivery of aromatic-cationic peptides for the prevention or treatment of ophthalmic conditions in mammalian subjects in need thereof.

### Example 13 - Ophthalmic delivery system: isotonic solution for multiple or single dose use ophthalmic application with extended life using an aromatic-cationic peptide of the present technology

As D-Arg-2',6'-Dmt-Lys-Phe-NH₂ or a pharmaceutically acceptable salt thereof (e.g., actetate salt or tri-fluoro-acetate salt) is known to hydrolyze slowly in solution but still at a rate that could prevent obtaining a 2-3 year commercial shelf-life, solutions from Examples 10-12 can be aseptically lyophilized and provided as a two chamber, reconstitutable vial or dispenser in which the dry powder can be mixed with water for Injection to provide a dispensable dropper solution designed for limited use over the period of one day, 1 week or one month.

This example demonstrates aromatic-cationic peptide formulations useful for the ocular delivery of aromatic-cationic peptides for the prevention or treatment of ophthalmic conditions in mammalian subjects in need thereof.

### Example 14 - Ophthalmic delivery system: viscous solutions or thermosetting gel for unit or multiple dose ophthalmic application using an aromatic-cationic peptide of the present technology:

| **TABLE 18. Viscous solutions or thermosetting gel for unit or multiple dose Ophthalmic application** | | |
|---|---|---|
| Component | Concentration | Weight per 100ml |
| D-Arg-2',6'-Dmt-Lys-Phe-NH₂ or pharmaceuticall y acceptable salt thereof such as actetate salt or tri-fluoro-acetate salt) | 1-10mg/ml | 0.10-1g |
| Viscocity Inducing polymer A | 0-1% | 0-1g |
| Thermosetting Gel polymer B | 0-15% | 0-15g |
| Propyl Paraben Methyl Paraben | 0.02% 0.2% | As required As required |
| L-Glutathione JP/EP | 0.1mg/ml | 0.01g |
| Water for Injection NF | NA | To 100ml |
| pH adj with Acetic acid/ 0.1M NaOH | Qs to pH 5.5-7.5 | Qs to pH 5.5-7.5 |

Viscosity inducing polymer A includes, but is not limited to, *e.g.,* methycellulose, hydroxypropyl cellulose, hydroxy propyl methycellulose, calcium and sodium carboymethylcellulose, alginic acid and its calcium or sodium salts, polyacrylic acid and its sodium or calcium salts, methacrylate polymers and their derivatives, polyvinyl alcohol.

Thermosetting gel polymer B is typically a polyoxyethylene-polyoxypropylene block copolymer (BASF's Pluronic F-127 or F-68).

To the solution of D-Arg-2',6'-Dmt-Lys-Phe-NH₂ or a pharmaceutically acceptable salt thereof (*e.g.,* actetate salt or tri-fluoro-acetate salt), the selected amount viscosity inducing polymer A is added and then dispersed to appropriate viscosity > 100cP (centipoise). Following this, the selected amount of thermosetting polymer B is dissolved and dispersed in the mixture. The other ingredients are then added and the solution is brought to final volume, allowing the solution to form a semi-viscous gel of 50-100cP. This can be aseptically filtered and sterilized and filled into dispensers of polyethylene or other suitable and compatible polymer. Upon application to the eye, the material gels and forms a slowly eroding semi-solid beneath the lower eye-lid which slowly releases aromatic-cationic peptide (*e.g.,* D-Arg-2',6'-Dmt-Lys-Phe-NH₂) to the lacrimal fluid bathing the cornea.

This example demonstrates aromatic-cationic peptide formulations useful for the ocular delivery of aromatic-cationic peptides for the prevention or treatment of ophthalmic conditions in mammalian subjects in need thereof.

### Example 15 -Ophthalmic delivery system: liposomal emulsion to protect aromatic-cationic peptides of the present technology from proteolysis

To protect a peptide drug such as an aromatic-cationic peptide of the present technology (*e.g*., D-Arg-2',6'-Dmt-Lys-Phe-NH₂ or a pharmaceutically acceptable salt thereof such as actetate salt, tri-fluoro-acetate salt, from attack by lysosomes and proteases commonly found in lacrimal fluid, liposomal emulsion formulations, including multilammellar vesicles (MLVs) can be used to entrap and protect the drug. Liposomal formulations also offer the advantage of providing controlled release of the aromatic-cationic peptide (*e.g.,* D-Arg-2',6'-Dmt-Lys-Phe-NH₂) upon administration.

The skilled artisan will understand that a variety of lipid components can be used to formulate such therapeutic emulsions. Which components are selected will depend, in some instances, on the interaction of the components with the aromatic-cationic peptide, the intended use, and the release pattern desired. Exemplary liposomal formulations for aromatic-cationic peptide (*e.g.,* D-Arg-2',6'-Dmt-Lys-Phe-NH₂) administration are provided in Table 19, below.

| **TABLE 19: Molar ratios of ophthalmic liposomal emulsion formulations** | | | | |
|---|---|---|---|---|
| **Molar Ratios** | | | | |
| **Compositions including aromatic-cationic peptide** (*e.g.,* D-Arg-2',6'-Dmt-Lys-Phe-NH₂ or a pharmaceutically acceptable salt thereof such as actetate salt or tri-fluoro-acetate salt) | **EPC** | **EPG** | I **CH** | **a**-**T** |
| **A** | 99 | - | - | 1 |
| **B** | 94 | 5 | - | 1 |
| **C** | 49 | 5 | 40 | 1 |
| **D** | - | 99 | - | 1 |
| **E** | 59 | - | 40 | 1 |

Egg phosphatidylcholine (EPC) and egg phosphatidylglycerol (EPG) can be acquired from commercially available sources, *e.g*., Avanti Lipid (Birmingham, AL). Lipid purity can be determined by thin-layer chromatography; typically, lipids of about 99% purity are used. Cholesterol (CH) and alpha-tocopherol (a-T) can also be obtained from commercially available sources, *e.g*., NuChek Prep. Inc., (Eysian, MN) and Sigma Chemical Co., (St. Louis, MO), respectively. Again, CH and a-T having a purity of about 99% or greater are typically used. The active agent, D-Arg-2',6'-Dmt-Lys-Phe-NH₂ or a pharmaceutically acceptable salt thereof such as actetate salt or tri-fluoro-acetate salt) is provided for encapsulation. The final concentration of the active agent can vary, depending on intended use, and can be, for example, from about 0.001 mg/ml to about 1.0 mg/ml or greater.

The molar ratios of lipid components for five exemplary MLV preparations are shown in Table 19. To form the MLV formulations, the lipid components in chloroform stock solution can be mixed in a tube or round bottom flask. The chloroform can then be removed by rota-evaporation and the lipid mixture dissolved in an appropriate volume of t-butanol to completely solubilize the lipid mixture. The butanol solution can then be frozen in dry-ice/acetone and lyophilized overnight. The dry lipids can then be hydrated in phosphate buffered saline (PBS), pH 7.4.

The lipid film can then be hydrated with vortexing for about 15 minutes at room temperature to form an MLV suspension having heterogeneous sizes ranging from about 0.2 to 10 microns. The vesicle preparations can then be sized by extrusion through a polycarbonate membrane having selected pore sizes. The entire preparation can be extruded through a 1.0 micron polycarbonate membrane, producing vesicles which have an initial vesicle size (before any aggregation in case of neutral liposomes) of about 1 micron. In forming smaller-size vesicles, the sized vesicles can be further extruded successively through 0.4 and 0.2 micron pore size membranes, to produce vesicles with sizes in the 0.2 micron size range.

Typically, MLVs containing encapsulated aromatic-cationic peptide can be freed of non-liposome-associated free aromatic-cationic peptide by washing three times in PBS by centrifugation.

The protective effect of the formulation can be tested by contacting MLVs containing encapsulated aromatic-cationic peptide with a one or more proteases commonly found in lacrimal fluid and determining the rate of degradation of the aromatic-cationic peptide as a function of time compared with the rate of degradation of unencapsulated aromatic-cationic peptide as a function of time. Observation of a lower rate of degradation of the MLVs containing encapsulated aromatic-cationic peptide in the presence of a one or more proteases from lacrimal fluid compared to the rate of degradation of unencapsulated aromatic-cationic peptide alone indicates that the formulation is a suitable liposomal emulsion useful to protect aromatic-cationic peptides of the present technology from proteolysis and useful for ocular delivery of the aromatic peptide(s) to the eye of a subject.

This example demonstrates aromatic-cationic peptide formulations useful for the ocular delivery of aromatic-cationic peptides for the prevention or treatment of ophthalmic conditions in mammalian subjects in need thereof.

### Example 16 -Ophthalmic delivery system: Entrapped aromatic-cationic peptide of the present technology in albumin microspheres for slow release

For administration of an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) locally around the eye, *e.g.,* via injection, or administered via a gel or ointment into the ocular region, a formulation including peptide microspheres is provided. Microsphere formulations entrap the aromatic-cationic peptide and allow for a gradual release of the aromatic-cationic peptide and a subsequent slow build-up of the aromatic-cationic peptide over time. Like liposomal formulations, microsphere formulations can protect an aromatic-cationic peptide, such as D-Arg-2',6'-Dmt-Lys-Phe-NH₂, from proteolytic enzymes.

The protective effect of the formulation can be tested by contacting microspheres containing encapsulated aromatic-cationic peptide with a one or more proteases commonly found in lacrimal fluid and determining the rate of degradation of the aromatic-cationic peptide as a function of time compared with the rate of degradation of unencapsulated aromatic-cationic peptide as a function of time. Observation of a lower rate of degradation of the microspheres containing encapsulated aromatic-cationic peptide in the presence of a one or more proteases from lacrimal fluid compared to the rate of degradationof unencapsulated aromatic-cationic peptide alone indicates that the formulation is useful to protect aromatic-cationic peptides of the present technology from proteolysis and useful for ocular delivery of the aromatic peptide(s) to the eye of a subject.

Typically, the microspheres are prepared from a bio-compatible material that will gel in contact with a mucosal surface. Starch microspheres (cross-linked if necessary) are a typical material. Other materials that can be used to form microspheres include starch derivatives (*e.g.,* hydroxyethyl starch, hydroxypropyl starch, carboxymethyl starch, cationic starch, acetylated starch, phosphorylated starch, succinate derivatives or starch and grafted starches), modified starches such as amylodextrin, gelatin, albumin, collagen, dextran and dextran derivatives (*e.g.,* diethylaminoethyl-dextran (DEAE-dextran), dextran sulphate, dextran methyl-benzylamide sulphonates, dextran methylbenzylamide carboxylates, carboxymethyl dextran, diphosphonate dextran, dextran hydrazide, palmitoyldextran and dextran phosphate), polyvinyl alcohol, polylactide-co-glycolide, hyaluronic acid and derivatives thereof such as benzyl and ethyl esters, gellan gum and derivatives thereof such as benzyl and ethyl esters and pectin and derivatives thereof such as benzyl and ethyl esters.

Preparation of microspheres is well described in the pharmaceutical literature (see for example Davis "Microspheres and Drug Therapy," Elsevier Biomedical Press, 1984), and emulsion and phase separation methods are both suitable. For example, albumin microspheres may be made using the water-in-oil emulsification method where a dispersion of albumin may be produced in a suitable oil by homogenization techniques or stirring techniques, with the addition if necessary of small amounts of an appropriate surface active agent. The following examples demonstrate preparation of microspheres with a size range of about 0.1 µm to 10 µm. For each microsphere formulation, the drug (*e.g.,* D-Arg-2',6'-Dmt-Lys-Phe-NH₂ or a pharmaceutically acceptable salt thereof, such as actetate salt or tri-fluoro-acetate salt) can be contained within the microsphere, admixed with the microspheres or absorbed onto the microspheres.

### A. Preparation of hyaluronic acid ester microspheres by solvent extraction

An emulsion is formed by mixing a 6% w/v solution of the polymer *e.g.,* benzyl hyaluronic acid ester (Hyaff-11) in dimethylsulphoxide with white mineral oil containing 0.5% Arlacel A. The inner phase is added to the outer oil phase (their respective ratio is 1:16 v/v) with continuous stirring for 10 minutes (1000 rpm). Ethyl acetate, the extraction solvent is then added to the emulsion at a ratio of 2:1 v/v. The extraction proceeds for 15 minutes at a stirring rate of 700 rpm until the microparticles are formed. The microsphere suspension can then be filtered and extensively washed with.n-hexane and dried. A one or more aromatic-cationic peptides (*e.g.,* D-Arg-2',6'-Dmt-Lys-Phe-NH₂ or a pharmaceutically acceptable salt thereof, such as actetate salt or tri-fluoro-acetate salt)) can be incorporated into the microspheres by addition to the initial polymer solution.

### B. Preparation of small starch microspheres using emulsification

A 10% starch gel can be prepared by heating (70° C) 5 g of starch with 40 ml of water until a clear gel is formed. After cooling, water can be added to a volume of 50 ml. 20 ml of this starch gel can then be added to 100 ml of soya oil BP containing antioxidant and 1% v/v Span 80 and homogenised at 7000 rpm for 3 minutes. This emulsion can then be added to 100 ml hot (80°C) soya oil BP (containing antioxidant) and stirred at 1500 rpm with a paddle stirrer while heating to 115°C over 15 minutes. The emulsion can be stirred at 115° C and then rapidly cooled by packing in ice while stirring. 100 ml of acetone can then be added and the microspheres centrifuged at 4500 rpm for 15 minutes. The pellet can then be resuspended in acetone and separated into the desired size fraction by filtering through an appropriate sieve, for example a 0.5 µm fluoropore filter. The microspheres can then allowed to air dry.

### C. Production of small albumin microspheres

Albumin microspheres can be produced by a modification of the method described by Ratcliffe et al (1984) J. Pharm. Pharmacol. 36, 431-436. One ml of 5% human serum albumin or ovalbumin at pH 6.8 can be added to 25 ml of olive oil or light mineral oil with or without 0.25 ml of Span 85. The mixture can then be stirred in a mix-cell for 10 min under turbulent flow conditions to form a w/o emulsion, using a mechanical stirrer (Heidolph) at 775 rpm (Tachometer DOT 1, Compact Instruments). Glutaraldehyde solution 25% (w/v) can then be added to 3.6% (v/v) of aqueous phase and the emulsion stirred for a further 30 minutes to denature and crosslink the albumin. The microspheres can be collected by centrifugation at 2500 g for 20 min. The oil can then be removed and the spheres washed with diethyl ether followed by ethanol. The microspheres can be collected by decantation.

### D. Production of small starch microsphere

Five grams of potato starch can be dissolved in 95 ml of water at about 90° C. A second solution can be prepared from 3 g of polyethylene glycol (m_{w} =6000) and 47 ml of water. This solution can then be heated to about 70°C, whereafter the warm starch solution can be added while stirring, to form an emulsion. When the two-phase system has formed (with the starch solution as the inner phase) the mixture can be cooled to room temperature under continued stirring, whereupon the inner phase will be converted to gel particles. The particles can be filtered off at room temperature and stirred in 100 ml of ethanol, whereafter the particles can again be filtered off and laid to dry in air.

The microspheres as described above can be modified by chemical crosslinking or heat treatment if desired.

### E. Preparation of lyophilised microspheres formulations containing aromatic-cationic peptide of the present technology

For the preparation of lyophilised microsphere formulations containing one or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt), a solution of about 50-100 mg of aromatic-cationic peptide(s) in 50 ml of water can be prepared. The desired quantity of the microspheres (*e.g.,* as describe above, prepared without aromatic-cationic peptide(s)), can then be dispersed in 20 ml of aromatic-cationic peptide solution with 12 ml of water (to keep the ratio of microspheres to solution at about 15:1 mg:ml). The two resultant suspensions can then be stirred for one hour at room temperature and freeze-dried to obtain a powder formulation. The freeze-drying can be performed on an Edwards Modulyo freeze-dryer fitted with a bell-jar assembly and operated at a pressure of 0.08 torr (10.7 N/m²), a condenser temperature of -53° C and a product shelf temperature of approximately 20° C. The freeze-drying process is allowed to proceed for about 24 hours, after which the final product can be loaded into an appropriate administration device (if needed) and stored with dessicant at 4° C prior to administration.

This example demonstrates aromatic-cationic peptide formulations useful for the ocular delivery of aromatic-cationic peptides for the prevention or treatment of ophthalmic conditions in mammalian subjects in need thereof.

### Examples 17 -Ophthalmic delivery system: injectable PLA/PGA microspheres for depot release of the aromatic-cationic peptides of the present technology in the ophthalmic tissues

50-200 mg of an aromatic-cationic peptide such as D-Arg-2',6'-Dmt-Lys-Phe-NH₂ (or a pharmaceutically acceptable salt thereof, such as actetate salt or tri-fluoro-acetate salt) can be dissolved in a solution of 3.8 g of a lactic acid-glycolic copolymer (lactic acid/glycolic acid=75/25 (mole %), GPC weight average mol. wt.=10,000, GPC number average mol. wt.=4,400, number average mol. wt. by endgroup determination=4,300; manufacturer; Wako Pure Chemical (Lot. 880530)) in 6.7 g (5.0 ml) of dichloromethane. The resulting solution can then be cooled to 17° C and poured into 1000 ml of a 0.1% aqueous solution of polyvinyl alcohol previously adjusted to about 11° C - to about 16°C. Thereafter, the mixture can be emulsified using a turbine mixer at 7000 rpm to prepare an O/W emulsion. This O/W emulsion can be stirred at room temperature for 3 hours to evaporate the dichloromethane. The oil phase can be solidified and collected with a centrifuge (05PR-22, Hitachi) at 2000 rpm. This solid can then be redispersed in distilled water and further centrifuged to wash off the free drug. The collected microcapsules can then be redispersed in a small quantity of distilled water, followed by addition of 0.3 g of D-mannitol and freeze-dried to provide a powder.

As another example, 100-400 mg of an aromatic-cationic peptide *(e.g.,* D-Arg-2',6'-Dmt-Lys-Phe-NH₂ or a pharmaceutically acceptable salt thereof, such as actetate salt or tri-fluoro-acetate salt)) can be dissolved in a solution of the lactic acid-glycolic acid copolymer (3.6 g, in 8.0 g (6.0 ml) of dichloromethane). The resulting solution can then be cooled to 15°C and poured into 1000 ml of a 0.1% aqueous solution of polyvinyl alcohol previously adjusted to 14° C. Thereafter, the mixture can be emulsified using a turbine mixer at 7000 rpm to prepare an O/W emulsion. This O/W emulsion can be stirred at room temperature for 3 hours to evaporate the dichloromethane. The oil phase can be solidified and collected with a centrifuge (05PR-22, Hitachi) at 2000 rpm. This solid can then be redispersed in distilled water and further centrifuged to wash off the free drug *etc.* The collected microcapsules can then be redispersed in a small quantity of distilled water, followed by addition of 0.3 g of D-mannitol and freeze-dried to provide a powder.

The skilled artisan will understand that there are many other ways of making a coacervate of PLA-PGA microcapsules, and the method selected may depend, in some instances, on the release rate required, the intended use or the desired aromatic-cationic peptide concentration. For example, in some applications, the aromatic-cationic peptide (*e.g.,* D-Arg-2',6'-Dmt-Lys-Phe-NH₂ or a pharmaceutically acceptable salt thereof, such as actetate salt or tri-fluoro-acetate salt) is administered at about 1 to about 5 mg/day, locally in the eye. To achieve this dosage, formulations including 30 - 150 mg of encapsulated peptide per mole are prepared. This translates to a dose of about 0.6-3 grams, if injected locally as a depot near the ocular circulation.

In addition, solvents less toxic than dichloromethane can also be used (flurocarbons, liquid CO2, siloxanes, natural oils) and surfactants, dispersants and accelerators can be adjusted to modify release rates.

This example demonstrates aromatic-cationic peptide formulations useful for the ocular delivery of aromatic-cationic peptides for the prevention or treatment of ophthalmic conditions in mammalian subjects in need thereof.

### Example 18 - Ophthalmic delivery system: Slowly eroding, biodegradable film containing aromatic-cationic peptide of the present technology to deliver slow release of the aromatic-cationic peptide topically or via implant

Film formulations can be used to provide a targeted, sustained release of an aromatic-cationic peptide of the present technology such as D-Arg-2',6'-Dmt-Lys-Phe-NH₂ (or a pharmaceutically acceptable salt thereof, such as actetate salt or tri-fluoro-acetate salt), *e.g.,* in the ophthalmic cavity such as by implant, or when applied topically. Typically, film formulations for delivery of ophthalmic drugs include a combination of water-soluble, film-forming polymers and a fatty acid glyceride or ester.

Two different exemplary films including an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) are provided in Table 20 below. The films can be prepared by dissolving hydroxypropyl cellulose (HPC), Mvyerol 18-92 and aromatic-cationic peptide in methanol. The solution can be placed in a container and films (in the shape of discs, for example) can be obtained by evaporating the methanol. The composition and size of two exemplary films is shown in Table 20.

| **TABLE 20: Compositions and dimensions of exemplary ophthalmic films** | | |
|---|---|---|
| | **Film A** | **Film B** |
| Composition (w/w %) | 0.50- 0.84% D-Arg-2',6'-Dmt-Lys-Phe-NH₂ or a pharmaceutically acceptable salt thereof, such as actetate salt or tri-fluoro-acetate salt 99.50- 99.16% HPC | 0.55 - 0.82% D-Arg-2',6'-Dmt-Lys-Phe-NH₂ or a pharmaceutically acceptable salt thereof, such as actetate salt or tri-fluoro-acetate salt 1.94% myverol 18-92 96.69-97.24% HPC |
| Solvent used for film preparation | Methanol | Methanol |
| Average weight of film disc | 9.60 +/- 0.43 mg | 10.03 +/- 0.05 mg |
| Average thickness of film disc | 0.45-0.50 mm | 0.45-0.50 mm |

| | | |
|---|---|---|
| Diameter of film disc | 4.8 mm | 4.8 mm |

To determine if ocular administration of an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) can increase survival of a subject suffering from ALS or at risk to develop ALS, select G93A transgenic familial ALS mice are administered daily the aromatic-cationic peptide via the eye using an ophthalmic delivery system such as that described in Examples 10-19 starting at 30 days of age. The mice are assessed for the time of disease onset as judged by the appearance of tremor and hind limb clasping was well as deterioration in the rotarod performance.

The aromatic-cationic peptide will delay the onset of the disease when compared to the time of onset observed in subjects that did not receive the aromatic-cationic peptide, therefore demonstrating that ocular administration of the aromatic-cationic peptide is a useful method to prevent or treat ALS in a subject in need thereof.

This example demonstrates aromatic-cationic peptide formulations useful for the ocular delivery of aromatic-cationic peptides for the prevention or treatment of ophthalmic conditions in mammalian subjects in need thereof.

### Example 19 - Ocular Administration of a Aromatic-Cationic Peptide of the Present Technology Protects Against Parkinson's Disease

MPTP is a neurotoxin that selectively destroys striatal dopamine neurons and can be used as an animal model of Parkinson's Disease. MPP⁺, a metabolite of MPTP, targets mitochondria, inhibits complex I of the electron transport chain and increases ROS production. To determine if ocular administration of an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) can prevent loss of dopamine neurons in mice treated with MPTP, three doses of MPTP (10 mg/kg) are given to mice (n=12) 2 h apart. Aromatic-cationic peptide is administered to the subject via the eye using an ophthalmic delivery system such as that described in Examples 10-19 or 22, 30 min before each MPTP injection, and at 1 h and 12 h after the last MPTP injection. Animals are sacrificed one week later and striatal brain regions are immunostained for tyrosine hydroxylase activity and dopamine, DOPAC and HVA levels are quantified by high pressure liquid chromatography. (see generally, USP7781405).

The aromatic-cationic peptide will prevent the loss of dopamine neurons in mice treated with MPTP, therefore demonstrating that ocular administration of the aromatic-cationic peptide is a useful method to prevent or treat Parkinson's Disease in a subject in need thereof. The aromatic-anionic peptide will increase striatal dopamine, DOPAC (3,4-dihydroxyphenylacetic acid) and/or HVA (homovanillic acid) levels in mice treated with MPTP.

These results will show that ocular delivery of aromatic-cationic peptides of the present technology, such as D-Arg-2',6'-Dmt-Lys-Phe-NH₂, are useful for the prevention or treatment of neurodegenerative disorders. In particular, the results show that aromatic-cationic peptides of the present technology, such as D-Arg-2',6'-Dmt-Lys-Phe-NH₂, are useful for the prevention or treatment of Parkinson's Disease in mammalian subjects in need thereof.

### Example 20 - Ocular Administration of a Aromatic-Cationic Peptide of the Present Technology Can Increase Survival Time of Subjects with ALS

To determine if ocular administration of an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) can increase survival of a subject suffering from ALS or at risk to develop ALS, select G93A transgenic familial ALS mice are administered daily the aromatic-cationic peptide via the eye using an ophthalmic delivery system such as that described in Examples 10-19 starting at 30 days of age. The mice are assessed for the time of disease onset as judged by the appearance of tremor and hind limb clasping was well as deterioration in the rotarod performance.

The aromatic-cationic peptide will delay the onset of the disease when compared to the time of onset observed in subjects that did not receive the aromatic-cationic peptide, therefore demonstrating that ocular administration of the aromatic-cationic peptide is a useful method to prevent or treat ALS in a subject in need thereof.

These results will show that ocular delivery of aromatic-cationic peptides of the present technology, such as D-Arg-2',6'-Dmt-Lys-Phe-NH₂, are useful for the prevention or treatment of neurodegenerative disorders. In particular, the results show that aromatic-cationic peptides of the present technology, such as D-Arg-2',6'-Dmt-Lys-Phe-NH₂, are useful for the prevention or treatment of ALS in mammalian subjects in need thereof.

### Example 21 -Administration of a Aromatic-Cationic Peptide of the Present Technology Can Prevent, Treat or Ameliorate Microvascular Damge/Injury Due to Acute Ocular Ischemia

Ischemic insults to the retina and optic nerve are frequently observed in glaucoma, acute ocular hypertension, diabetic retinopathy, hypertension, and vascular occlusion, and giant cell arteritis and can lead to serious perturbation of neuronal and glial retinal elements and can ultimately lead to blindness. A rat model of acute elevation of intraocular pressure (IOP), characterized by ischemia-reperfusion injury is used as described by Grozdanic and colleagues (2003), to determine the effect of administration of an aromatic-cationic peptide of the present technology (*e.g.,* D-Arg-2',6'-Dmt-Lys-Phe-NH₂ or a pharmaceutically acceptable salt thereof, such as actetate salt or tri-fluoro-acetate salt) on microvascular damage/injury after to acute ocular ischemia. (See generally, Grozdanic, SD, et al., Functional characterization of retina and optic nerve after acute ocular ischemia in rats. Invest Ophthalmol Vis Sci. 2003 Jun;44(6):2597-605.). That is, retinal ischemia is induced in rats by acutely increasing the IOP (110 mm Hg/60 minutes). Aromatic-cationic peptide infusion is started at approximately 10 minutes before IOP elevation treatment and continued throughout approximately 60 minutes of IOP elevation treatment (dose/volume to be determined); aromatic-cationic peptide infusion time is equal to approximately 70 minutes. The retinal vessels of each eye of the subject are visualized and assessed for damage (*e.g.,* retinal capillary nonprefusion; leakage, *etc*.) using fluorescein angiography as described by Brown and Magargal. Brown GC, Magargal LE. The ocular ischemic syndrome. Clinical, fluorescein angiographic and carotid angiographic features. Int Ophthalmol. Feb 1988;11(4):239-51. The aromatic-cationic peptide will improve the degree of retinal vessel damage and/or no-perfusion as judged by less retinal capillary nonperfusion or leakage when compared with the degree of retinal capillary nonperfusion or leakage in subjects that did not receive the aromatic-cationic peptide, therefore demonstrating that administration of the aromatic-cationic peptide is a useful method to prevent, treat or ameliorate microvascular damage/injury after to acute ocular ischemia in a subject in need thereof.

These results will show that ocular delivery of aromatic-cationic peptides of the present technology, such as D-Arg-2',6'-Dmt-Lys-Phe-NH₂, are useful for the prevention or treatment of neurodegenerative disorders. In particular, the results show that aromatic-cationic peptides of the present technology, such as D-Arg-2',6'-Dmt-Lys-Phe-NH₂, are useful for the prevention or treatment of microvascular damage/injury due to acute ocular ischemia in mammalian subjects in need thereof.

### Example 22 - Topical formulations of a aromatic-cationic peptide of the present technology

The present technology provides formulations of the aromatic-cationic peptides disclosed herein useful for topical application to ocular tissue.

Illustrative embodiments of topical formulation that can be prepared and that are useful for application to ocular tissue are detailed below:

Topical Formulation 1: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising at least Polyethylene Glycol 400 - 0.4 %Lubricant, and Propylene Glycol - 0.3 %Lubricant and adjusted to a pH suitable for application to ocular tissue.

Topical Formulation 2: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising at least Boric Acid , Calcium Chloride , Hydroxypropyl Guar , Magnesium Chloride , Potassium Chloride , Purified Water , Sodium Chloride , Zinc Chloride , May Contain: Hydrochloric Acid and , or Sodium Hydroxide and adjusted to a pH suitable for application to ocular tissue

Topical Formulation 3: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising at least povidone, Boric Acid , Potassium Chloride , Sodium Borate , Sodium Chloride , Edetate Disodium, and Sorbic Acid and adjusted to a pH suitable for application to ocular tissue.

Topical Formulation 4: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising Benzalkonium Chloride , Boric Acid , Edetate Disodium , Purified Water, Sodium Borate , and Sodium Chloride and adjusted to a pH suitable for application to ocular tissue.

Topical Formulation 5: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising Boric Acid , Calcium Chloride Dihydrate , Citric Acid Monohydrate , GenAqua (Sodium Perborate), Magnesium Chloride Hexahydrate , Phosphonic Acid , Potassium Chloride , Purified Water , and Sodium Chloride and adjusted to a pH suitable for application to ocular tissue.

Topical Formulation 6: One or more aromatic-cationic peptides of the present technology (*e.g.,* D-Arg-2',6'-Dmt-Lys-Phe-NH₂ or a pharmaceutically acceptable salt thereof, such as actetate salt or tri-fluoro-acetate salt) in a composition comprising Glycerine; castor oil; polysorbate 80; carbomer copolymer type A; purified water; and sodium hydroxide to adjust pH.

Also, the present technology provides a method of preventing, treating or ameliorating dry eye disorder in a subject in need thereof wherein topical administration of a therapeutically effective amount of a one more peptides of the present technology in a topical formulation suitable for application to ocular tissue treats, prevents or ameliorates dry eye disorder in the subject in need thereof. Briefly, topical formulations 1-6 (test formulation) are individually administered in a subject at risk for, or suffering from dry eye disorder and tear production is measured. A topical formulation 1-6 that increases tear production in a subject administered such formulation when compared to tear production of a subject receiving a control formulation without aromatic-cationic peptide indicates that the test formulation is useful to prevent, treat or ameliorate dry eye disorder in the subject.

Topical formulations 1-6 may also be useful for ocular administration of the aromatic-cationic peptides of the invention in disorders of the central nervous system or other eye diseases or disorders as described herein.

This example demonstrates aromatic-cationic peptide formulations useful for the ocular delivery of aromatic-cationic peptides for the prevention or treatment of ophthalmic or neurological conditions in mammalian subjects in need thereof.

### Example 23 - Prevention and Treatment of Diabetic Macular Edema using Aromatic-Cationic Peptide

### A. Introduction

When the simplicidentata, duplicidentata or carnivorous animals that inherently do not have macula lutea are used as experimental models, edema is expressed in a retinal visual cell layer and the thickness of the retinal visual cell layer can be used in evaluation. In nonhuman primates, on the other hand, there are usually macula lutea, so edema is expressed in a macula lutea and the thickness and/or volume of the macula lutea can be used in evaluation. The thickness *etc.* of the macula lutea are evaluated preferably in the site of macular central fovea. Intraocular ischemia/reperfusion treatment can be easily carried out by stopping a retinal blood stream by increasing the intraocular pressure and then relieving the intraocular pressure to allow reperfusion. The thickness of the retinal visual cell layer or the macula lutea varies significantly depending on individuals, so in some experiments, a treated eye and untreated eye are set preferably in the same individual; only one eye is subjected to intraocular ischemia/reperfusion treatment. By so doing, the relative evaluation of "thickness of a treated eye/thickness of an untreated eye" can be carried out on the basis of the untreated eye in each animal.

A pharmacological agent to be examined (*e.g.,* D-Arg-2',6'-Dmt-Lys-Phe-NH₂) or pharmaceutically acceptable salt thereof such as acetate or trifluoroacetate salt) is administered into the model animal with diabetic maculopathy and then evaluated for the effectiveness of the pharmacological agent for edema as described above, whereby the effectiveness of the pharmacological agent for diabetic maculopathy (*e.g.,* macular edema) can be evaluated. The method of administering the pharmacological agent is not particularly limited and can be by oral, parenteral, topical (*e.g.,* eye drops or eye ointment), ocular, and administration of the pharmacological agent carried out after intraocular ischemia/reperfusion induction, thereby clarifying the therapeutic effect.

### B. Efficacy Pharmacological Test 1 - Rat Test 1

### 1. Test Method

Diabetes mellitus is induced in male Sprague Dawley rats (8-week-old) weighing about 250 g by injecting streptozotocin (STZ manufactured by Sigma) intravenously into their tail at the dose of 60 mg/kg. One week after the treatment with STZ, serum glucose is measured, and rats with at least 300 mg/dl glucose are then used in the experiment as diabetic rats. The set groups will be the following 5 groups shown below, and 2 weeks after the treatment with STZ, 5% gum arabic solution or D-Arg-2',6'-Dmt-Lys-Phe-NH₂ solution will be orally, parenterally, or topically administered once or twice per day, as noted below, for 2 days.
(1) Normal control group (5 rats): To be given 5% gum arabic solution in a ratio of 5 ml/kg.
(2) Diabetic control group (7 rats): To be given 6% gum arabic solution in a ratio of 5 ml/kg.
(3) ORAL D-Arg-2',6'-Dmt-Lys-Phe-NH₂ (or a pharmaceutically acceptable salt thereof, such as acetate salt or trifluoroacetate salt): diabetic group will be given 1 ml of an oral suspension of D-Arg-2',6'-Dmt-Lys-Phe-NH₂ from about 1 ng/ ml to about 10,000 mg/ml, including but not limited to 100 mg/ml, 500 mg/ml or 1000 mg/ml of D-Arg-2',6'-Dmt-Lys-Phe-NH₂ (4 rats at each concentration) in 5% gum arabic solution, twice per day.
(4) PARENTERAL D-Arg-2',6'-Dmt-Lys-Phe-NH₂ or a pharmaceutically acceptable salt thereof, such as acetate salt or trifluoroacetate salt: Diabetic rats will be dosed at from about 1 ng per kilogram body weight to about 10,000 mg per kilogram body weight, including but not limited to 1 mg/kg, 5 mg/kg or 10 mg/kg of D-Arg-2',6'-Dmt-Lys-Phe-NH₂ (4 rats at each concentration) locally *(e.g.,* in or near the eye) twice per day.
(5) EYE DROPS D-Arg-2',6'-Dmt-Lys-Phe-NH₂ or a pharmaceutically acceptable salt thereof, such as acetate salt or trifluoroacetate salt: Diabetic rats will be group given 2 drops (approximately 50 µl/drop) of a solution of D-Arg-2',6'-Dmt-Lys-Phe-NH₂ at a concentration of from about 0.1 ng/ml to about 1000 mg/ml (*e.g.,* from aobut 0.01 ng to about 100 mg), including but not limited to 1 mg/ml, 10 mg/ml or 100 mg/ml prepared according to Example 10 (4 rats at each concentration) directly to the eye twice per day.

After administration of SZT for 2 weeks, intraocular ischemia will be caused by the treatment described below. After the treatment is finished, the animals will be maintained as usual for 2 days, and thereafter, the eyeballs will be excised and histologically evaluated. Administration of the pharmacological agent will also be conducted for a period (2 days) of reperfusion after ischemic treatment.

### 2. Retinal Ischemia Induction by Increasing the Intraocular Pressure

A drip infusion set (Thrufusion Drip Infusion Set manufactured by Terumo) is connected to a bottle containing an intraocular perfusion solution (Opeguard MA manufactured by Senjyu Seiyaku), and an extension tube (Thrumo) to which a three-way stopcock is attached is connected thereto. A needle (30Gx1/2, manufactured by Nippon Becton Dickinton) is fitted to the end of the tube. The bottle containing an intraocular perfusion solution is fixed to a certain height with a stand. The rats are anesthetized by administering sodium pentobarbital (Somunopentyl manufactured by Schering-Plough Animal Health) intraperitoneally in a ratio of 50 mg/kg, and then a mydriatic (Mydrin P manufactured by Santen Pharmaceutical) and a local anesthetic (Benoxyl eye drop 0.4%, Santen Pharmaceutical) is dropped onto the right eye. The anesthetic is additionally administered when necessary. Thereafter, a needle is stuck into an anterior chamber of the rat right eye and the intraocular pressure load is performed by manipulating the three-way stopcock (the intraocular pressure was increased to 130 mmHg or more for 60 minutes). Because the ocular fundus in the Sprague Dawley rat turns from red to white by stopping the retinal blood stream by increasing the intraocular pressure, achievement of retinal ischemia can be easily observed. After the intraocular pressure is increased for the predetermined time, the needle is removed to relieve the intraocular pressure to allow reperfusion, and an antibacterial eye drop (tarivit eye ointment manufactured by Santen Pharmaceutical) is applied onto the right eye.

### 3. Histological Evaluation

Two days after the ischemia treatment (two days after the reperfusion), the rat left and right eyeballs are excised under anesthesia with ether. The excised eyeballs are placed in an ice-cold fixing solution (phosphate buffer solution containing 3% glutaraldehyde) and fixed therein for 2 days. Thereafter, the eyeballs are washed for 1 day with a phosphate buffer solution. The eyeballs are embedded in a usual manner into paraffin to prepare a transverse section containing a bundle of optic nerves. The section is stained with hematoxylin-eosin. The histological evaluation is conducted by each of two (2) visual fields in left and right side (4 visual fields/rat) in the vicinity of the bundle of optic nerves, from an optical microscope to an image analyzer (IPAP-WIN, Sumika Techno Service).

In each of the resulting retinal images, the thickness of the visual cell layer is measured. The degree of edema is expressed in percentage by dividing the thickness of the visual cell layer of the ischemic/re-perfused eyeball (right eye) by the thickness of the visual cell layer of the untreated eyeball (left eye) in the same individual. As an indicator of retinal cell functions, nuclei in the inner retinal layer (ganglion cell layer) are counted, and the degree of loss of nuclei are evaluated relative to the ratio of the number of nuclei occurring per unit area.

### 4. Results and Discussion

It is anticipated that the thickness of the visual cell layer after ischemia/reperfusion in the rats in the normal control group will be less than the thickness of the untreated eye. On the other hand, the rats in the diabetic control group are expected to show an increase in the visual cell layer by ischemia/reperfusion, and formation of edema will be confirmed with statistical significance. In the diabetic groups given D-Arg-2',6'-Dmt-Lys-Phe-NH₂, the thickness is anticipated to be almost the same as that of the normal control group, and that no edema or very low levels of edema will be observed.

It is anticipated that no loss of nuclei will be noted in the normal rat control group. It is anticipated that a loss of nuclei will be evident in the diabetic control group rats (*e.g.,* evident nuclei loss in 30-50% of subjects with some subjects showing a loss of 50% or more of nuclei). It is anticipated that in the diabetic group given D-Arg-2',6'-Dmt-Lys-Phe-NH₂, loss of nuclei will be absent, or much diminished as compared to the diabetic control group.

These results are expected to demonstrate that D-Arg-2',6'-Dmt-Lys-Phe-NH₂ inhibits edema formation under diabetes in a visual cell layer and also prevents disturbances in functions of retinal cells.

These results will show that oral, parenteral, or ocular delivery of aromatic-cationic peptides of the present technology, such as D-Arg-2',6'-Dmt-Lys-Phe-NH₂, is useful for the prevention or treatment of ophthalmic disorders in mammalian subjects in need therof. In particular, the results show that aromatic-cationic peptides of the present technology, such as D-Arg-2',6'-Dmt-Lys-Phe-NH₂, are useful for the prevention or treatment of diabetic macular edema in mammalian subjects in need thereof.

### C. Efficacy Pharmacological Test 2 - Rat Test 2

### 1. Test Method

The test will be carried out in accordance with Efficacy Pharmacological Test 1, described above. The set groups will be the following 5 groups of male SD rats, and from 2 weeks after the treatment with STZ, 5% gum arabic solution or D-Arg-2',6'-Dmt-Lys-Phe-NH₂ (or a pharmaceutically acceptable salt thereof, such as acetate salt or trifluoroacetate salt) solution will be orally, parenterally, or topically (eye drops) administered once per day.
(1) Normal control group (10 rats): Given 5% gum arabic solution in a ratio of 5 ml/kg.
(2) Diabetic control group. (9 rats): Given 5% gum arabic solution in a ratio of 5 rang.
(3) ORAL D-Arg-2',6'-Dmt-Lys-Phe-NH₂ (or a pharmaceutically acceptable salt thereof, such as acetate salt or trifluoroacetate salt): diabetic group will be given 1 ml of an oral suspension of D-Arg-2',6'-Dmt-Lys-Phe-NH₂ from about 1 ng/ ml to about 10,000 mg/ml,including 100 mg/ml, 500 mg/ml or 1000 mg/ml of D-Arg-2',6'-Dmt-Lys-Phe-NH₂ (4 rats at each concentration) in 5% gum arabic solution, once per day.
(4) PARENTERAL D-Arg-2',6'-Dmt-Lys-Phe-NH₂ (or a pharmaceutically acceptable salt thereof, such as acetate salt or trifluoroacetate salt): Diabetic rats will be dosed at from about 1 ng per kilogram body weight to about 10,000 mg per kilogram body weight, including but not limited to 1 mg/kg, 5 mg/kg or 10 mg/kg of D-Arg-2',6'-Dmt-Lys-Phe-NH₂ (4 rats at each concentration) locally (*e.g.,* in or near the eye) once per day.
(5) EYE DROPS D-Arg-2',6'-Dmt-Lys-Phe-NH₂ (or a pharmaceutically acceptable salt thereof, such as acetate salt or trifluoroacetate salt): Diabetic rats will be group given 2 drops (approximately 50 µl/drop) of a solution of D-Arg-2',6'-Dmt-Lys-Phe-NH₂ at a concentration of from about 0.1 ng/ml to about 1000 mg/ml, (*e.g.,* from about 0.01 ng to about 100 mg) including but not limited to 1 mg/ml, 10 mg/ml or 100 mg/ml prepared according to Example 10 (4 rats at each concentration) directly to the eye once per day.

Retinal ischemia produced by increasing the intraocular pressure will be performed in accordance with Efficacy Pharmaceutical Test 1. Histological evaluation will also be performed in accordance with Efficacy Pharmaceutical Test 1.

### 2. Results and Discussion

It is anticipated that the thickness of the visual cell layer after ischemia/reperfusion in the rats in the normal control group will be reduced as compared with that of the untreated (*i.e.,* no ischemia-reperfusion) eye. On the other hand, the rats in the diabetic control group are anticipated to show an increase in the visual cell layer by ischemia/reperfusion; it is anticipated that the formation of edema will be confirmed with statistical significance (*e.g.,* p<0.05). In the diabetic group given D-Arg-2',6'-Dmt-Lys-Phe-NH₂, it is anticipated that the thickness of the visual cell layer will measure in at about the same range/value as in the normal control group, and an evident inhibitory action on edema will be observed. These results will indicate that edema formation under diabetes in a visual cell layer is inhibited by administration of a D-Arg-2',6'-Dmt-Lys-Phe-NH₂.

These results will show that oral, parenteral, or ocular delivery of aromatic-cationic peptides of the present technology, such as D-Arg-2',6'-Dmt-Lys-Phe-NH₂, is useful for the prevention or treatment of ophthalmic disorders in mammalian subjects in need therof. In particular, the results show that aromatic-cationic peptides of the present technology, such as D-Arg-2',6'-Dmt-Lys-Phe-NH₂, are useful for the prevention or treatment of diabetic macular edema in mammalian subjects in need thereof.

### D. Efficacy Pharmacological Test 3 - Monkey (Macaca fascicularis) Test

### 1. Method

Diabetes mellitus will be induced in male monkeys (Macaca fascicularis) (3-year-old) weighing about 2.1 to 2.4 kg by intravenously injecting STZ into their foreleg vein at the dose of 80 mg/kg. Two days after the treatment with STZ, blood glucose level will be measured, and monkeys with at least 200 mg/dl glucose will then be used in the experiment as diabetic monkeys. Insulin will be administered subcutaneously once or twice per day into monkeys showing a blood glucose level of 300 mg/dl. The set groups will be the following 5 groups, and from 2 weeks after the treatment with STZ, 5% gum arabic solution or D-Arg-2',6'-Dmt-Lys-Phe-NH₂ (or a pharmaceutically acceptable salt thereof, such as acetate salt or trifluoroacetate salt) solution will be orally, parenterally or topically (eye drops) administered once a day.
(1) Normal control group (4 monkeys): Given 5% gum arabic solution in a ratio of 5 ml/kg.
(2) Diabetic control group (6 monkeys): Given 5% gum arabic solution in a ratio of 5 ml/kg.
(3) ORAL D-Arg-2',6'-Dmt-Lys-Phe-NH₂ (or a pharmaceutically acceptable salt thereof, such as acetate salt or trifluoroacetate salt): diabetic monkeys will be given 1 ml of an oral suspension of D-Arg-2',6'-Dmt-Lys-Phe-NH₂ from about 1 ng / ml to about 10,000 mg/ml, including but not limited to including 100 mg/ml, 500 mg/ml or 1000 mg/ml of D-Arg-2',6'-Dmt-Lys-Phe-NH₂ (4 monkeys at each concentration) in 5% gum arabic solution, once per day.
(4) PARENTERAL D-Arg-2',6'-Dmt-Lys-Phe-NH₂ (or a pharmaceutically acceptable salt thereof, such as acetate salt or trifluoroacetate salt): Diabetic monkeys will be dosed at about 0.1 ng mg per kilogram body weight to about 10,000 mg per kilogram body weight, including but not limited to 1 mg/kg, 5 mg/kg or 10 mg/kg of D-Arg-2',6'-Dmt-Lys-Phe-NH₂ (4 monkeys at each concentration) locally *(e.g.,* in or near the eye) once per day.
(5) EYE DROPS D-Arg-2',6'-Dmt-Lys-Phe-NH₂ (or a pharmaceutically acceptable salt thereof, such as acetate salt or trifluoroacetate salt): Diabetic monkeys will be group given 2 drops (approximately 50 µl/drop) of a solution of D-Arg-2',6'-Dmt-Lys-Phe-NH₂ at a concentration of from about 0.1 ng/ml to about 1000 mg/ml (*e.g.,* from about 0.01 ng to about 100 mg), including but not limited to 1 mg/ml, 10 mg/ml or 100 mg/ml prepared according to Example 10 (4 monkeys at each concentration) directly to the eye once per day.

After administration for 2 weeks, intraocular ischemic treatment will be carried out as described below, and after the treatment is finished, the animals will be maintained as usual for 7 days. Before the ischemic treatment and 7 days after treatment, the thickness and volume of the macular central fovea (in the diameter range of 1 mm from the center of the macula lutea) will be measured by an OCT scanner (Stratus OCT, Carl Zeiss). Administration of the pharmacological agent will also be conducted for the period (7 days) of reperfusion after the ischemic treatment.

Retinal ischemia produced by increasing intraocular pressure will be in accordance with Efficacy Pharmaceutical Test 1. However, the size of the needle used will be 25G x 1/2 (Terumo). After a mydriatic (Mydrin P manufactured by Santen Pharmaceutical) is dropped onto the right eye, the monkey will be anesthetized by intramuscularly administering ketaral (Sankyo Life Tech). Subsequently, a local anesthetic (Benoxyl eye drop 0.4%) will be dropped onto the eye, and the monkey will be prevented from blinking with an eyelid speculum. Anesthesia with ketaral will be additionally carried out when necessary.

The thickness and volume of the macular central fovea will be measured in the following manner. After a mydriatic (Mydrin P) is dropped onto the right eye of the monkey to dilate the pupil of the eye sufficiently, the monkey will be anesthetized by intramuscularly administering Ketaral. Thereafter, the monkey will be allowed to sit on a monkey chair and the head will be fixed. The inside of the eye will be observed with an OCT scanner to identify the macula lutea, followed by scanning. On the basis of the resulting cross-sectional macular image, the thickness and volume of the macular central fovea will be analyzed.

### 2. Results

It is anticipated that in the normal control group, formation of edema will not be observed, and the thickness and volume (average) of the macular central fovea after ischemia and reperfusion are anticipated to be the same before the treatment (ischemia-reperfusion) and 7 days after the treatment. In the diabetic control group, on the other hand, an increase in the thickness and volume of the macular central fovea is anticipated when measured 7 days after the ischemia-reperfusion treatment, and formation of edema is anticipated to be confirmed with statistical significance. This change (increase in thickness and volume of the macular central fovea) in the diabetic group is anticipated to be significantly increased as compared with that of the normal control group. In the diabetic group given D-Arg-2',6'-Dmt-Lys-Phe-NH₂, formation of edema is not anticipated to be observed (*e.g.,* these samples are anticipated to be similar to the normal control group), or to be less than that observed in the diabetic controls to a statistically significant degree. These results are anticipated to show that D-Arg-2',6'-Dmt-Lys-Phe-NH₂ inhibits edema formation under diabetes in the macular central fovea.

These results will show that oral, parenteral, or ocular delivery of aromatic-cationic peptides of the present technology, such as D-Arg-2',6'-Dmt-Lys-Phe-NH₂, is useful for the prevention or treatment of ophthalmic disorders in mammalian subjects in need therof. In particular, the results show that aromatic-cationic peptides of the present technology, such as D-Arg-2',6'-Dmt-Lys-Phe-NH₂, are useful for the prevention or treatment of diabetic macular edema in mammalian subjects in need thereof.

### E. Efficacy Pharmacological Test 4 - Clinical Results

### 1. Method

Among patients with diabetic maculopathy 10 patients with diabetic macular edema having a retinal thickening or a hard exudates in a posterior pole of the retina will be the subjects. D-Arg-2',6'-Dmt-Lys-Phe-NH₂ (or a pharmaceutically acceptable salt thereof, such as acetate salt or trifluoroacetate salt) will be orally, parenterally, or topically (eye drops) administered in a dose corresponding to the most effective does as determined in the animal models, above, once a day before breakfast for 8 weeks. During this test period, simultaneous use of epalrestat, intravitreal injection and sub-Tenon injection of an adrenal cortical hormone, and photocoagulation and vitrectomy will be prohibited. Basic therapy of diabetes mellitus will be carried out so as to give good blood glucose control throughout the test period.

Evaluation will be carried out in terms of the thickness of the macular central fovea (in the diameter range of 1 mm from the center of macula lutea) and the thickness at the center of the central fovea measured by optical coherence tomography (OCT, Carl Zeiss), as well as corrected visual acuity (Log MAR).

Log MAR (Log Minimum Angle of Resolution) is one kind of logarithmic visual acuity, which is visual acuity expressed in logarithmic minimum angle of resolution. Decimal visual acuity 1.0 is 0.0 in terms of Log MAR, and decimal visual acuity 0.1 is 1.0 in Log MAR. A log MAR visual acuity of 0.1 to 0.5 corresponds to a decimal visual acuity of 0.8 to 0.32.

### 2. Results

All evaluable eyes from the 10 cases will be evaluated. When the tests are initiated, the thickness of the macular central fovea (in the diameter range of 1 mm) is anticipated to be about 325 µm on average, and the thickness at the center of the central fovea is anticipated to be similar on average. After 8 weeks, these dimensions are expected to be reduced by an average of about 3-10% or more *(e.g.,* to about 300 µm respectively).

Out of all the examined eyes, it is anticipated that corrected visual acuity will be recognized in most if not all subjects, and deterioration will be seen in few or no subjects. The corrected visual acuity (Log MAR) on average is anticipated to improve for subjects in the treatment group. It is anticipated that D-Arg-2',6'-Dmt-Lys-Phe-NH₂ will be shown to have a visual acuity-improving action important in therapy of maculopathy, and in particular macular edema.

Diabetic maculopathy according to conventional findings is a gradually worsening disease that is considered difficult to treat. The results of the present examples will indicate that D-Arg-2',6'-Dmt-Lys-Phe-NH₂ is effective for treatment of diabetic macular edema. With respect to safety, no particularly problematic side effects are anticipated.

These results will show that oral, parenteral, or ocular delivery of aromatic-cationic peptides of the present technology, such as D-Arg-2',6'-Dmt-Lys-Phe-NH₂, is useful for the prevention or treatment of ophthalmic disorders in mammalian subjects in need therof. In particular, the results show that aromatic-cationic peptides of the present technology, such as D-Arg-2',6'-Dmt-Lys-Phe-NH₂, are useful for the prevention or treatment of diabetic macular edema in mammalian subjects in need thereof.

### Example 24 - treatment and Prevention of Diabetic Macular Edema using- Aromatic-Cationic Peptides

### A. Overview

In diabetic control rats, diabetic rats receiving D-Arg-2',6'-Dmt-Lys-Phe-NH₂ (or a pharmaceutically acceptable salt thereof, such as acetate salt or trifluoroacetate salt), age-matched control rats, and rats experiencing dilutional hyponatremia (as a positive edema control), whole central retinal thickness, intraretinal water content and apparent diffusion coefficients (ADC, 'water mobility') will be measured in vivo using quantitative MRI methods as described in Berkowitz et al., (Jan. 2012) PLoS One, vol. 7, issue 1. Glycated hemoglobin and retinal thickness ex vivo (histology) will also be measured in control, diabetic groups and D-Arg-2',6'-Dmt-Lys-Phe-NH₂ treatment groups.

### B. Methods:

### 1. Induction of diabetes

Diabetes is induced with intraperitoneal injection of streptozocin (60 mg/kg) within 5 minutes of its preparation in 0.01 M citrate buffer (pH 4.5) in male SD rats with body weights of approximately 200 g after overnight fast. Diabetes is verified 3 days later by the presence of plasma hyperglycemia (>300 mg/dl) and elevated urine volume (more than 60 ml/d) in nonfasted rats. Rat body weight and blood glucose levels are monitored weekly. Subtherapeutic levels of insulin (0-2 U neutral protamine Hagedorn insulin administered subcutaneously daily) is administered to maintain blood glucose levels between 450 and 550 mg/dl without urine ketones. Glycated hemoglobin is measured after 2 months of diabetes (Glyco-Tek affinity columns, kit 5351; Helena Laboratories, Beaumont, TX).

### 2. Induction and scanning of dilutional hyponatremia subjects

Rats experiencing dilutional hyponatremia (DH): After being anesthetized with urethane (in preparation for MRI, see below), non-diabetic rats (n =5) are scanned ("preDH"), then injected intraperitoneally with distilled water (three injections, 50 ml per kg body weight, spaced five minutes apart) before a second MRI scan ("postDH"; with 3.860.2 hrs (mean 6 SEM) between the starts of scanning periods), (ii) non-diabetic control rats used to test the reproducibility of ADC measurements throughout the thickness of the retina (n =4), especially regarding the prolonged anesthesia used in the DH group, are scanned ("earlyC"), then maintained for several hours before a second scan ("later C", 4.660.3 hrs between starts of scanning periods), (iii) diabetic male SD rats ("D") are scanned once (n= 7), and (iv) a corresponding set of age-matched controls ("C") are scanned once (n= 8).

### 3. Treatment with D-Arg-2',6'-Dmt-Lys-Phe-NH₂

Nine month old diabetic rats are treated with D-Arg-2',6'-Dmt-Lys-Phe-NH₂ by injection, either intraperitoneal (IP) or subcutaneous (SC), with 1, 5, or 10 mg/kg D-Arg-2',6'-Dmt-Lys-Phe-NH₂. Injections are once/day. After 7 days of treatment, the retinas of the rats in the treatment and control groups are evaluated.

### 4. Evaluation

Male SD rats are examined by MRI by the methods of Berkowitz et al. Central retinal thickness at 2, 3, 4, 6, and 9 months after induction of diabetes is measured, and in age-matched non-diabetic controls. At day 7 after D-Arg-2',6'-Dmt-Lys-Phe-NH₂ treatment, rats are again evaluated via MRI according to the methods of Berkowitz et al.

### 5. Results

In the dilutional hyponatremia model, central retinal thickness and water content are anticipated to be supernormal by quantitative MRI, and intraretinal water mobility profiles are anticipated to change in a manner consistent with intracellular edema. Diabetic control rats are anticipated to show supernormal whole central retinal thickness. Water content metrics are anticipated to be significantly greater than normal, and apparent diffusion coefficient is anticipated to be subnormal in the outer retina. Diabetic rats treated with D-Arg-2',6'-Dmt-Lys-Phe-NH₂ are anticipated to show normal or only a slight deviation from normal in retinal thickness, intraretinal water content and apparent diffusion coefficients. Exemplary data is shown in Table 21, below. "N" indicates a normal range; "+" indicates a deviation from normal, either high or low, depending on the context of the measurement.

These results will show that intraperitoneal (IP) or subcutaneous (SC) delivery of aromatic-cationic peptides of the present technology, such as D-Arg-2',6'-Dmt-Lys-Phe-NH₂, is useful for the prevention or treatment of ophthalmic disorders in mammalian subjects in need therof. In particular, the results show that aromatic-cationic peptides of the present technology, such as D-Arg-2',6'-Dmt-Lys-Phe-NH₂, are useful for the prevention or treatment of diabetic macular edema in mammalian subjects in need thereof.

| **TABLE 21: Subjects breakout and results of MRI studies** | | | | | | |
|---|---|---|---|---|---|---|
| | **Non diabetic control** | **Non-diabetic dilutional hyponatremia control** | **Diabeti c control** | **Diabetic D-Arg-2',6'-Dmt-Lys-Phe-NH₂ treatment (IP or SC injection)** | | |
| | | | | 1 mg/kg | 5 mg/kg | 10 mg/kg |
| Retinal thickness | N | N+++ | N+++ | N to N+ | N to N+ | N to N+ |
| Intraretina 1 water content | N | N+++ | N++ | N to N+ | N to N+ | N to N+ |
| Apparent diffusion coefficient | N | N+++ | N+++ | N to N+ | N to N+ | N to N+ |

### Example 25 - Spatial Frequency (SPF) Response of Streptozotocin (STZ)-Treated Mice Administered D-Arg-2',6'-Dmt-Lys-Phhe-NNH₂

This example shows the spatial frequency (SPF) response of streptozotocin (STZ)-treated mice administered D-Arg-2',6'-Dmt-Lys-Phe-NH₂.

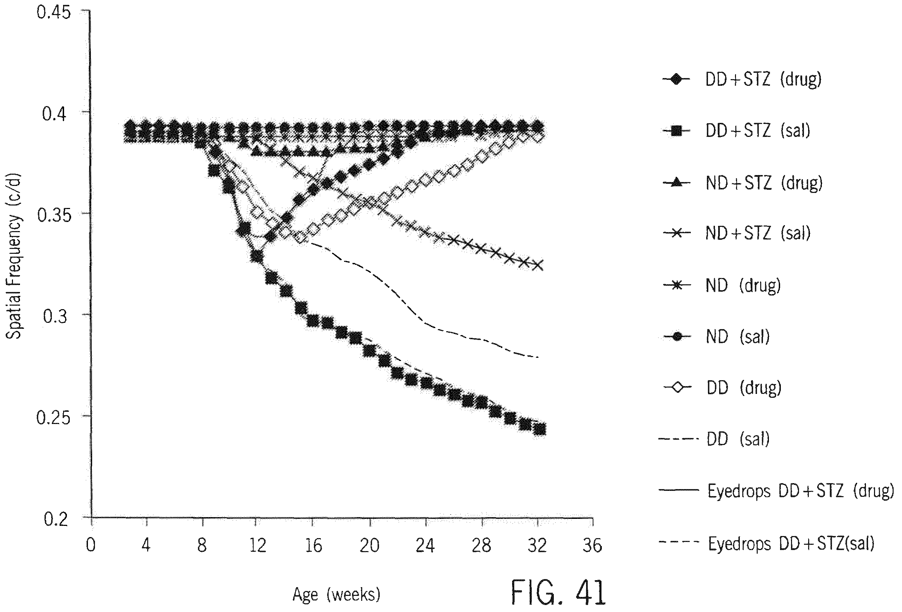

Subjects were maintained on a normal diet (ND) or diabetic diet (DD) for up to 32 weeks, and were administered D-Arg-2',6'-Dmt-Lys-Phe-NH₂ or control vehicle with or withour streptozotocin. Results are shown in FIG. 41, with response values are averaged for each eye.

The results show that D-Arg-2',6'-Dmt-Lys-Phe-NH₂ rescued visual impairment in animals with metabolic dysfunction. STZ-treated animals had the least effect on visual function, with the deficit was fully rescued by D-Arg-2',6'-Dmt-Lys-Phe-NH₂ by 24 weeks after systemic treatment was initiated at 12 weeks. Animals on a diabetic diet alone showed a greater decline by 12 weeks, and also showed rescue by 30 weeks.

Animals maintained on a diabetic diet and administered STZ showed the greatest degree of visual decline, with the deficit fully restored by 24 weeks. Notably, D-Arg-2',6'-Dmt-Lys-Phe-NH₂ administered by eyedrop rescued the visual impairment in STZ-treated animals maintained on a diabetic diet one month sooner than systemic administration.

These results show that aromatic-cationic peptides of the present technology, such as D-Arg-2',6'-Dmt-Lys-Phe-NH₂, are useful for the prevention or treatment of ophthalmic conditions in mammalian subjects in need thereof. In particular, the results show that aromatic-cationic peptides of the present technology, such as D-Arg-2',6'-Dmt-Lys-Phe-NH₂, are useful for the prevention or treatment of visual impairment induced by diabetes in mammalian subjects in need thereof.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

## Claims

1. A composition for preventing, treating, or ameliorating the symptoms of diabetic macular edema in a mammalian subject in need thereof, comprising: a therapeutically effective amount of a peptide D-Arg-2'6'-Dmt-Lys-Phe-NH₂ or a pharmaceutically acceptable salt thereof, wherein the composition is administered ocularly, orally, or parenterally.

2. The composition of claim 1, wherein the composition is administered ocularly to one or more regions of the eye.

3. The composition of claim 2, wherein the one or more regions of the eye is selected from the group consisting of the posterior chamber, ora serrata, ciliary muscle, ciliary zonules, canal of Schlemm, pupil, anterior chamber, cornea, iris, lens cortex, lens nucleus, ciliary process, conjunctiva, inferior oblique muscle, inferior rectus muscle, medial rectus muscle, retinal arteries and veins, optic disc, dura mater, central retinal artery, central retinal vein, optic nerve, vorticose vein, bulbar sheath, macula, fovea, sclera, choroid, superior rectus muscle, and retina.

4. The composition of claim 3, wherein the region of the eye is the macula.

5. The composition of claim 1, wherein the mammal is a human.

6. The composition of claim 1, wherein the subject is at risk of having, suspected of having, or diagnosed as having one or more of macular degeneration, central retinal thickness, intraretinal water content, macular central fovea thickness, contrast sensitivity, or loss of visual acuity.

7. The composition of claim 1, wherein the composition is administered in combination with at least one additional therapeutic agent.

8. The composition of claim 7, wherein the additional therapeutic agent is administered before, after, or simultaneous to the composition, or a combination thereof.

9. A composition for use in treating, preventing, or ameliorating microvascular damage caused by acute ocular ischemia in a mammalian subject in need thereof, the composition comprising a therapeutically effective amount of a peptide D-Arg-2'6'-Dmt-Lys-Phe-NH₂ or a pharmaceutically acceptable salt thereof, wherein the composition is for administration ocularly, orally, or parenterally.

10. The composition of claim 9, wherein the treatment further comprises the step of performing a revascularization procedure on the subject.

11. The composition of claim 9, wherein the composition is formulated for administration prior to or following the formation of ocular ischemia.

12. The composition of claim 9, wherein the composition is formulated for administration before, during, or after the revascularization procedure, or continuously before, during, and after the revascularization procedure.
